(19) Europäisches Patentamt
European Patent Office
Office européen des brevets

(11) Publication number: **0 027 948**
**B1**

(12) # EUROPEAN PATENT SPECIFICATION

(45) Date of publication of patent specification: **21.03.84**

(21) Application number: **80106232.4**

(22) Date of filing: **14.10.80**

(51) Int. Cl.³: **C 07 C 59/90,**
C 07 C 69/738,
C 07 C 62/38,
C 07 D 295/08,
C 07 D 307/12,
C 07 D 213/30,
C 07 C 103/175,
C 07 C 67/30,
C 07 C 67/03,
A 61 K 31/215,
A 61 K 31/19 //C07C51/43,
C07C67/52

(54) **Tetrahydro-fluorene compounds, processes for their preparation and pharmaceutical compositions thereof.**

(30) Priority: **19.10.79 US 86602**
**19.10.79 US 86605**
**19.10.79 US 86625**
**19.10.79 US 86626**
**19.10.79 US 86627**
**30.06.80 US 164704**
**30.06.80 US 164706**
**30.06.80 US 164708**
**30.06.80 US 164753**
**30.06.80 US 164754**

(43) Date of publication of application:
**06.05.81 Bulletin 81/18**

(45) Publication of the grant of the patent:
**21.03.84 Bulletin 84/12**

(84) Designated Contracting States:
**AT BE CH DE FR GB IT LI LU NL SE**

(56) References cited:
None

(73) Proprietor: **MERCK & CO. INC.**
**126, East Lincoln Avenue P.O. Box 2000**
**Rahway New Jersey 07065 (US)**

(72) Inventor: **Cragoe, Edward J., Jr.**
**2211 Oak Terrace Drive**
**Lansdale, PA, 19446 (US)**
Inventor: **Stokker, Gerald E.**
**Plymouth Road**
**Gwynedd Valley, PA 19437 (US)**
Inventor: **Gould, Norman P.**
**413 Williamsburg Road**
**Lansdale, PA 19446 (US)**

(74) Representative: **Blum, Rudolf Emil Ernst et al,**
**c/o E. Blum & Co Patentanwälte Vorderberg 11**
**CH-8044 Zürich (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 027 948 B1

Courier Press, Leamington Spa, England.

# Tetrahydro-fluorene compounds, processes for their preparation and pharmaceutical compositions thereof

## Background of the Invention

Trauma to the brain or spinal chord caused by physical forces acting on the skull or spinal column, by ischemic stroke or by hydrocephalus results in edema and swelling of the affected tissues. This is followed by ischemia, temporary or permanent brain and/or sprinal chord injury and may result in death. The tissues mainly affected are classified as grey matter.

The specific therapy currently used for the treatment of the medical problems described is limited to steroids, such as, the soldium salt of 6-$\alpha$-methylprednisolone succinate. Although these agents are effective in situations involving white matter edema, they comprise relative ineffective therapy for grey matter edema. Thus, the compounds of this invention comprise a novel and specific treatment for a medical problem where no specific therapy is available.

The compounds of the invention have the added advantage of being devoid of the toxic side effects of the steroids and of having little or no renal effects.

## Description of the Invention

The compounds of the instant invention are best characterized by reference to the following structural formula I

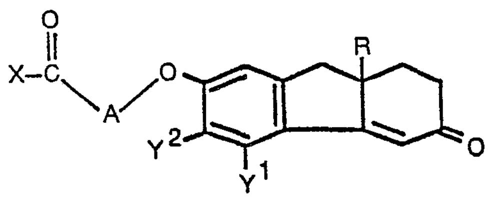

I

wherein

R is H, lower alkyl, branched or unbranched; aryl, aryl-$C_1$ to $C_4$ alkyl where the alkyl is branched or unbranched; $C_2$ to $C_4$ alkynyl, branched or unbranched;
$C_3$ to $C_6$ cycloalkyl and $C_3$ to $C_6$ cycloalkyl-$C_1$ to $C_4$ alkyl, branched or unbranched or $C_2$ to $C_4$ alkenyl, branched or unbranched;

X is hydroxy, a group forming an acid anhydride, amide, hydrazide, or guanidide or a group having the formula —O—$R^1$, wherein
$R^1$ is lower alkyl, lower alkenyl, lower alkynyl, lower cycloalkyl, heteroaryl, aryl, carboxy-substituted cycloalkyl, substituted lower alkyl, where the substituent is carboxy, lower alkoxycarbonyl, oxo, hydroxy, lower alkoxy, halo, lower acyloxy, lower dialkylamino, sulfamoyl, pyridyl, furyl, tetrahydrofuryl, 1-methylpiperidyl, morpholinyl, pyrrolidinyl, 1-methylpiperazinyl, thienyl or aryl,

$Y^1$ and $Y^2$ are independently Cl and $CH_3$;

A is the group

$-CH_2-CH_2-$ or 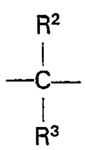

wherein
$R^2$ is H, methyl or ethyl,
$R^3$ is H, F or methyl or
$R^2$ and $R^3$ may be joined together to form the ring

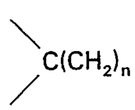

where n is the integer 2, 3 or 4

and pharmaceutically acceptable salts of the compounds of formula I.

Typical examples for R in compounds of formula I are hydrogen, methyl, ethyl, propyl, isopropyl, butyl, sec. butyl or isobutyl, vinyl or allyl, propargyl, phenyl, benzyl, cyclopropyl, and cyclobutyl or cyclopropylmethyl.

Typical examples for X in the compounds of formula I are hydroxy and the group of formula —O$R^1$, wherein

$R^1$ is an alkyl group having 1—4 carbon atoms, such as methyl, ethyl, propyl, isopropyl, butyl, isobutyl and tertbutyl, an alkenyl group having 2—4 carbon atoms, such as allyl, 2-butenyl, an alkynyl group having 3 or 4 carbon atoms, such as propargyl or butynyl, cycloalkyl having 4 or 5 carbon atoms, such as cyclobutyl or cyclopentyl, a carboxy-cycloalkyl, a heteroaryl, such as imidazolyl, pyridyl, thiazolyl, pyrazinyl, or furyl, phenyl, carboxyphenyl or hydroxymethylphenyl, or substituted

lower alkyl, where the substituent is carboxy, lower alkoxycarbonyl, oxo, hydroxy, lower alkoxy, halo, lower acyloxy, lower dialkylamino, sulfamoyl, pyridyl, furyl, tetrahydrofuryl, 1-methylpiperidyl, morpholinyl, pyrrolidinyl, 1-methylpiperazinyl, thienyl or aryl.

Specially preferred are those compounds of formula I wherein X is the group of formula —O—$R^1$ wherein $R^1$ is lower alkyl or carboxyloweralkyl.

In the compounds of formula I A is preferably the group

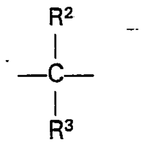

Since the 9a carbon atom in the compounds of formula I is asymmetric, the compounds of the invention are racemic; however, these compounds can be resolved, so that the invention includes the pure enantiomers. In addition, in some instances, the group represented by A includes an asymmetric carbon atom. Thus, these molecules may contain two asymmetric carbon atoms and now can consist of two diastereometers, each of which consists of two enantiomers. The invention includes the two diastereomeric pairs and their enantiomers whenever they exist.

The present invention accordingly involves novel esters of [(5,6,9a-substituted-3-oxo-1,2,9,9a-tetrahydro-3H-fluoren-7-yl)oxy]alkanoic and cycloalkanoic acids, the corresponding acids, and their salts, anhydrides, amides, hydrazides and guanidides.

Of the compounds of formula I, those are preferred which have the following formula Ia

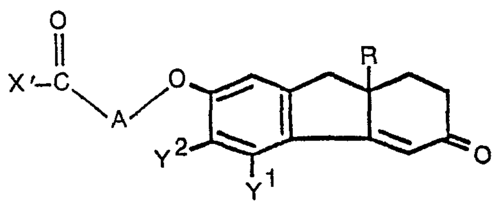

Ia

wherein
X' is hydroxy or a group having the formula —O—$R^1$ and wherein
R, $R^1$, $Y^1$, $Y^2$ and A have the same meaning as in formula I above
or are a pharmaceutically acceptable salt thereof.

Specially preferred are the compounds of formula Ib

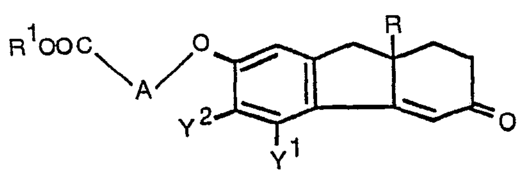

Ib

wherein
R, $R^1$, $Y^1$, $Y^2$ and A have the same meaning as in formula I.

The present invention, furthermore, concerns a composition in unit dosage form for the treatment of grey matter edema comprising a compound of the formula I

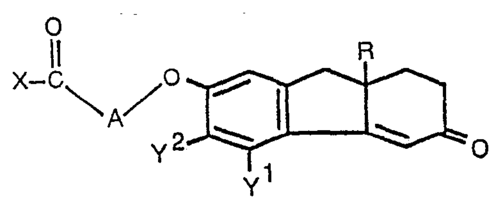

I

wherein
X, R, $R^1$, $Y^1$, $Y^2$ and A have the same meaning as outlined above
or a pharmaceutically acceptable salt thereof.

The present invention also involves processes for making the compounds of formula I, and preferably the preferred compounds of formula Ib.

Preferred embodiments of the Invention

The preferred embodiments of the instant invention are realized in structural formula Ib

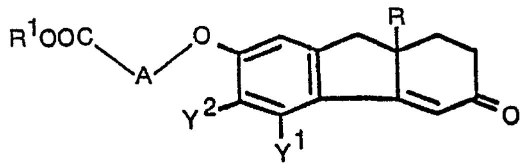

Ib′

wherein

R is lower alkyl, $C_{2-4}$ alkenyl, $C_{2-4}$ alkynyl, cyclopropyl, cyclopropylmethyl, aryl- $C_{1-4}$alkyl or phenyl,

$R^1$ is lower alkyl, lower alkenyl, lower alkynyl, lower cycloalkyl, aryl or substituted alkyl, such as carboxyalkyl, hydroxyalkyl, dihydroxyalkyl, trihydroxyalkyl, oxoalkyl, dilower alkylaminoalkyl, heteroaryl-alkyl, and aralkyl, wherein heteroaryl is pyridyl, furyl, tetrahydrofuryl, 1-methylpiperidyl, morpholinyl, pyrrolidinyl, 1-methyl piperazinyl or thienyl.

$Y^1$ and $Y^2$ are chloro

A is $-CH_2-$.

Also included are the enantiomers of each racemic compound.

A preferred compound is ethyl [(5,6-dichloro-9a-ethyl-3-oxo-1,2,9,9a-tetrahydro-3H-fluoren-7-yl)oxy]-acetate and its enantiomers.

Another preferred compound is ethyl [(5,6-dichloro-3-oxo-9a-propyl-1,2,9,9a-tetrahydro-3H-fluoren7-yl)oxy]acetate and its enantiomers.

Another preferred compound is ethyl [(9a-butyl-5,6-dichloro-3-oxo-1,2,9,9a-tetrahydro-3H-fluoren-7-yl)oxy]acetate and its enantiomers.

Another preferred compound is ethyl [(9a-allyl-5,6-dichloro-3-oxo-1,2,9,9a-tetrahydro-3H-fluoren-7-yl)oxy]acetate and its enantiomers.

Another preferred compound is ethyl [(5,6-dichloro-3-oxo-9a-propargyl-1,2,9,9a-tetrahydro-3H-fluoren7-yl)oxy]acetate and its enantiomers.

Another preferred compound is ethyl [(5,6-dichloro-9a-cyclopropylmethyl-3-oxo-1,2,9,9a-tetrahydro-3H-fluoren7-yl)oxy]acetate and its enantiomers.

Another preferred compound is ethyl [(9a-benzyl-5,6-dichloro-3-oxo-1,2,9,9a-tetrahydro-3H-fluoren7-yl)oxy]acetate and its enantiomers.

Another preferred compound is carboxymethyl [(5,6-dichloro-9a-ethyl-3-oxo-1,2,9,9a-tetrahydro-3H-fluoren-7-yl)oxy]acetate and its enantiomers.

Another preferred compound is the carboxymethyl [(5,6-dichloro-3-oxo-9a-propyl-1,2,9,9a-tetrahydro-3H-fluoren-7-yl)oxy]acetate and its enantiomers.

Another preferred compound is carboxymethyl[(9a-butyl-5,6-dichloro-3-oxo-1,2,9,9a-tetrahydro-3H-fluoren-7-yl)oxy]acetate and its enantiomers.

Another preferred compound is carboxymethyl [(9a-allyl-5,6-dichloro-3-oxo-1,2,9,9a-tetrahydro-3H-fluoren7-yl)oxy]acetate and its enantiomers.

Another preferred compound is carboxymethyl [5,6-dichloro-3-oxo-9a-propargyl-1,2,9,9a-tetrahydro-3H-fluoren-7-yl)oxy]acetate and its enantiomers.

Another preferred compound is carboxymethyl [(5,6-dichloro-9a-cyclopropylmethyl-3-oxo-1,2,9,9a-tetrahydro-3H-fluoren-7-yl)oxy]acetate and its enantiomers.

Another preferred compound is carboxymethyl [(9a-benzyl-5,6-dichloro-3-oxo-1,2,9,9a-tetrahydro-3H-fluoren-7-yl)oxy]acetate and its enantiomers.

Another preferred compound is 2-(4-morpholinyl)-ethyl [(5,6-dichloro-9a-ethyl-3-oxo-1,2,9,9a-tetrahydro-3H-fluoren-7-yl)oxy]acetate and its enantiomers.

Another preferred compound is 1-(hydroxymethyl)-2-hydroxyethyl [(5,6-dichloro-9a-ethyl-3-oxo-1,2,9,9a-tetrahydro-3H-fluoren-7-yl)oxy]acetate and its enantiomers.

Another preferred compound is 2,3-dihydroxypropyl [5,6-dichloro-9a-ethyl-3-oxo-1,2,9,9a-tetrahydro-3H-fluoren-7-yl)oxy]acetate and its enantiomers.

Another preferred compound is 2-(dimethylamino)-ethyl [(5,6-dichloro-9a-ethyl-3-oxo-1,2,9,9a-tetrahydro-3H-fluoren-7-yl)oxy]acetate and its enantiomers.

Another preferred compound is 3-carboxypropyl [(5,6-dichloro-9a-ethyl-3-oxo-1,2,9,9a-tetrahydro-3H-fluoren-7-yl)oxy]acetate and its enantiomers.

Included within the scope of this invention are the carboxylic acids of formula I, wherein X is hydroxy and the salts thereof and the corresponding acid anhydrides, amides, hydrazides or guanidides. Said derivatives may be prepared by conventional methods well known to those skilled in the art.

Thus, the acid addition salts can be prepared by the reaction of the carboxylic acids of the invention with an appropriate amine, guanidine, ammonium hydroxide, alkali metal hydroxide, alkali metal bicarbonate or alkali metal carbonate and the like. The salts are selected from among the nontoxic, pharmaceutically acceptable bases.

The compounds of formula I which are esters, i.e. like compounds of formula Ib,

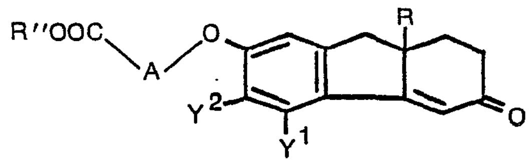

Ib

in which R'' has the same meaning as $R^1$ in formula I, can be prepared by a variety of methods including one or more of the following seven methods.

The first method involves the etherification of compounds of Formula II with a haloalkanoic acid ester or halocycloalkanoic acid ester of the formula X—A—COOR'', where X is halo and A and R'' are as defined previously to produce compounds of Formula Ib. The reaction is illustrated graphically below:

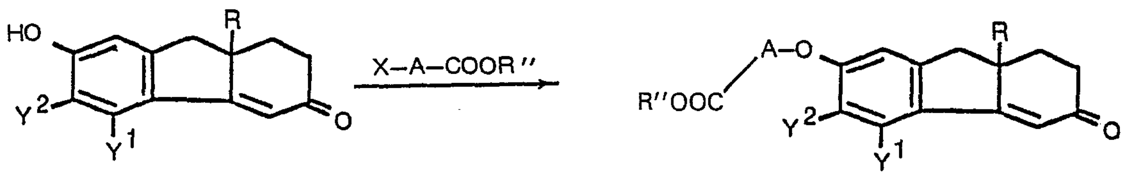


II Ib

In general, the reaction is conducted in the presence of a base, such as an alkali metal carbonate, hydroxide or alkoxide such as potassium carbonate, sodium carbonate, potassium hydroxide, sodium ethoxide and the like. Solvents which are essentially inert to the reactants and product and in which the reactants and product are reasonably soluble are usually employed. Dimethylformamide, ethanol and acetone, for example, have been found to be especially advantageous to use as solvents. The reaction may be conducted at a temperature in the range from about 25°C. to the boiling point temperature of the particular solvent employed. The reaction is generally complete in about 15 to 60 minutes; but, if lower temperatures are employed or if the particular halo ester is not very reactive, the reaction time may be much longer.

The second method for the synthesis of compounds of Formula Ib involves the cyclization of compounds of Formula IV to produce those of Formula Ib. This method will be discussed later.

The third method for the preparation of compounds of Formula Ib involves the esterification of acids of Formula I wherein X is hydroxy. This may be accomplished by well known esterification methods such as those described for the conversion of compounds of Formula VIII to those of Formula IX (vide infra).

A fourth method involves a transesterification process as shown below.

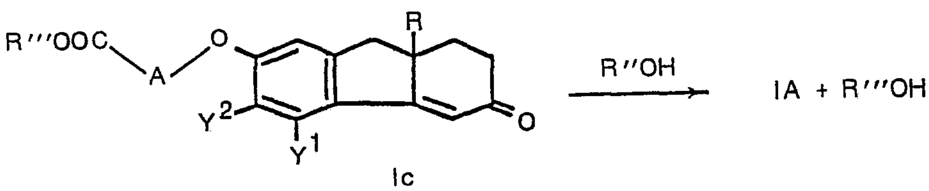


Ic

This involves heating an ester of Formula Ic (where R''' is selected from the same group as R'' except it cannot be identical to R'' in this reaction). The reaction proceeds successfully with a variety of alcohols (R''OH) and is generally carried out in the presence of a catalytic amount of R''OM where M is a sodium or potassium ion. The reaction is carried out in the presence of an ion-exchange resin, such as, "Amberlite" (registered Trade Mark) and the like. Solvents, such as, methylene chloride, chloroform, carbon tetrachloride, are employed and the reaction is conducted at temperatures ranging from 30°C to the boiling point of the solvent employed. The reaction time varies from 2 hours to one day.

A fifth method consists of the esterification of a salt of the corresponding carboxylic acids (V) with the appropriate halide of the formula R''X where X represents halogen and R'' is as defined above.

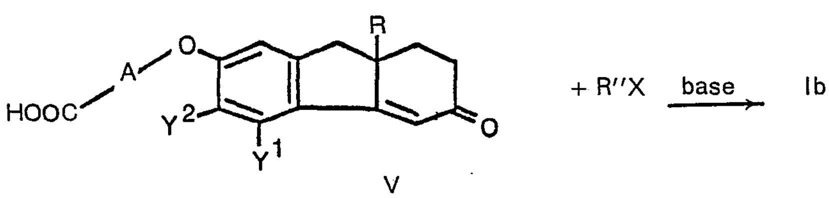


V

The reaction is carried out in the presence of a base, such as, potassium carbonate, sodium

carbonate and the like which generates the corresponding salt of the compound of Formula V. The reaction is generally carried out in the presence of a solvent such as, dimethylformamide, dimethylacetamide, N-methylpyrrolidinone and the like at temperatures usually in the range of 50° to 120°C. The reaction time varies from one to 24 hours depending on the temperature and the nature of the reactants.

A sixth method consists of the reaction of a mixed anhydride of Formula Id with an appropriate alcohol, R″OH.

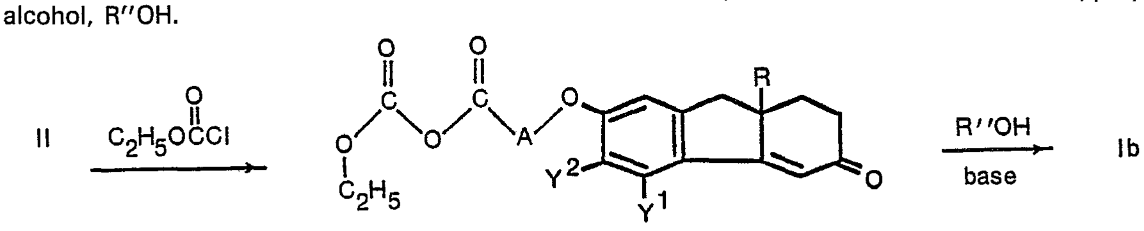

II
Ib

Id

The mixed anhydride is generally generated *in situ* from the corresponding carboxylic acid (Formula V) and ethyl chloroformate in tetrahydrofuran (or similar solvent) at a low temperature (such as, −10°C to +10°C) in the presence of an appropriate base (such as triethylamine and the like). The alcohol, R″OH, is added and the reaction mixture gradually increased to ambient temperature over a period of one to 5 hours and finally heated at 50°C to the boiling point of the solvent for one to 6 hours.

The seventh method involves the reaction of a 1-acylimidazole derivative of Formula Ie with an alcohol of Formula R″OH.

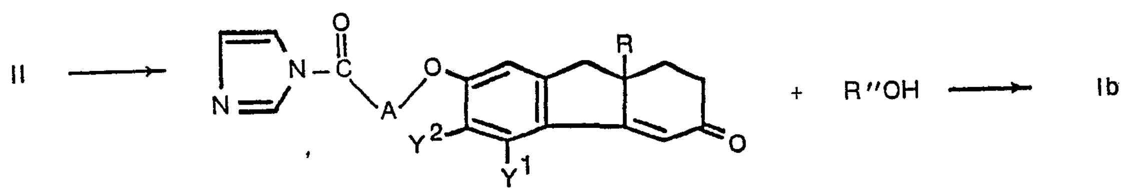
II
+ R″OH
Ib

Ie

The acylimidazole Ie is generally prepared *in situ* by the reaction of a Compound V with 1,1′-carbonyldiimidazole in a solvent (such as tetrahydrofuran, 1,4-dioxane and the like) at a temperature of −10° to +10°C. Reaction of the alcohol (R″OH) with Ie is generally conducted in the presence of a catalytic amount of a base (such as sodium hydride, $R''OC(CH_3)_3$ and the like). The reaction is generally carried out at temperatures between 0°C and ambient temperature and requires from one to 24 hours for completion.

It should be pointed out that the R″ moiety of the esters of Formula Ib may possess a chemically reactive function which requires the presence of a chemical protective group during one or another of the seven synthetic methods described. These protective groups may be removed by one of several processes, such as selective hydrolysis, hydrogenolysis and the like.

The synthesis of the carboxylic acid compounds of Formula V is generally carried out by the hydrolysis of the corresponding alkyl ester (If):

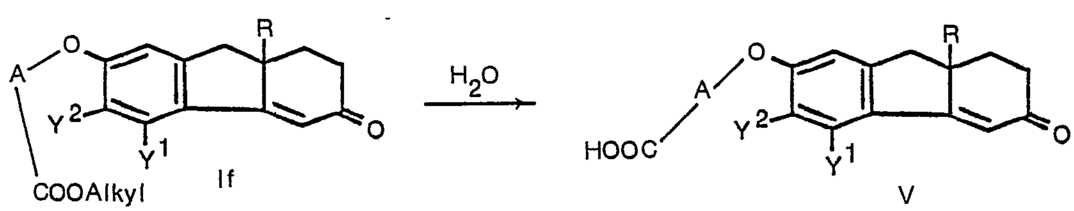

If
V

The process can be effected by heating the ester in a solution of acetic and aqueous inorganic acid, such as hydrochloric acid, sulfuric acid and the like. The reaction can be carried out at temperatures of 30°C to 100°C for periods of about 20 minutes to 6 hours, depending on the specific ester used and the other reaction conditions. In the instances, such as where A is —CHF—, it is advantageous to carry out the hydrolysis using a weak base, such as aqueous sodium bicarbonate. A solvent, such as aqueous ethanol, methanol or isopropyl alcohol is used and the mixture heated at 45°C to 100°C for periods of 15 minutes to 4 hours. Acidification of the reaction mixture with strong aqueous acids, such as hydrochloric acid, hydrobromic acid or sulfuric acid produces the desired compound of Formula V.

A second method for the preparation of compounds of the type illustrated by Formula V involve the reaction of a haloalkanoic or halocycloalkanoic acid (X—A—COOH) with the appropriate phenol (II).

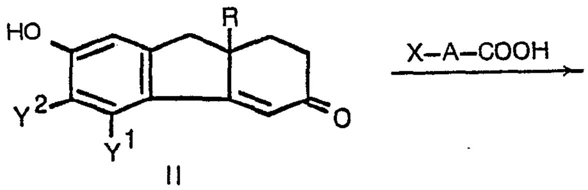


V

II

Using a haloalkanoic or halocycloalkanoic acid X—A—COOH, where X= iodo, bromo or chloro and A is as defined above, for example, iodoacetic acid or bromofluoroacetic acid, as the etherification agent, the reaction is conducted in the presence of a base. The base is selected from among the alkaline earth or alkali metal bases such as sodium or potassium carbonate, calcium hydroxide and the like. The reaction is carried out in a liquid reaction milieu, the choice being based on the nature of the reactants; however, solvents which are reasonably inert to the reactants and are fairly good solvents for the compounds of Formula II and the X—A—COOH reagent, can be used. Highly preferable are dimethylformamide, ethanol, acetone, and N-methylpyrrolidine-2-one, and the like.

A third method for preparing compounds of Formula V involves the pyrolysis of the corresponding tert.-butyl ester (Ig):

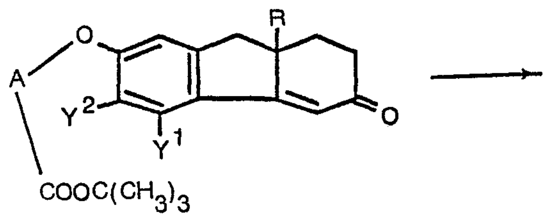

V + isobutylene

Ig

This method involves heating a tert.-butyl ester of the type illustrated by formula ID at from about 80°C to 120°C in a suitable non aqueous solvent in the presence of catalytic amount of a strong acid. The solvents are generally selected from among the type benzene, toluene, xylene, etc. and the acid catalyst may be a strong organic or inorganic acid, such as p-toluenesulfonic acid, benzenesulfonic acid, methanesulfonic acid, sulfuric etc. The acid, being a catalyst, is generally used in relatively small quantities as compared to the tert.-butyl ester (Ig). It is to be noted that this reaction is a pyrolysis and not a hydrolysis, since water is excluded from the reaction and the products are a carboxylic acid (Formula V) and isobutylene and no alcohol is produced.

A fourth method is limited to the instances wherein compounds of Formula VA are produced, i.e., where A is ethylene:

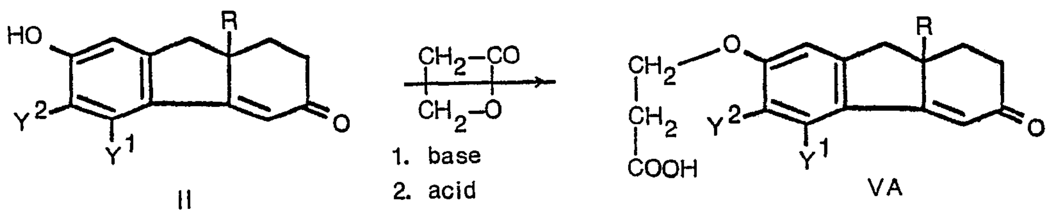


II

VA

This synthesis involves the reaction of the appropriate phenol (II) with propiolactone in the presence of a base and a solvent, preferably while heating and stirring the reaction mixture. Solvents, such as a mixture of water and an alcohol, such as methanol, ethanol or propanol are used. The base is selected from among the alkali metal hydroxides or carbonates, such as sodium hydroxide, potassium hydroxide, potassium carbonate and the like. The temperature is generally at the boiling point of the solvent mixtures but it may be at 50°C—110°C depending on the reactants involved. The product is isolated by acidification of the reaction mixture with a strong acid such as hydrochloric or sulfuric acid.

The compounds of formula If and Ig may be prepared by one or another of the seven methods described for the preparation of compounds of Formula Ib.

The preparation of compound II can be carried out by one or the other of two methods. The first method involves the following three-step process:

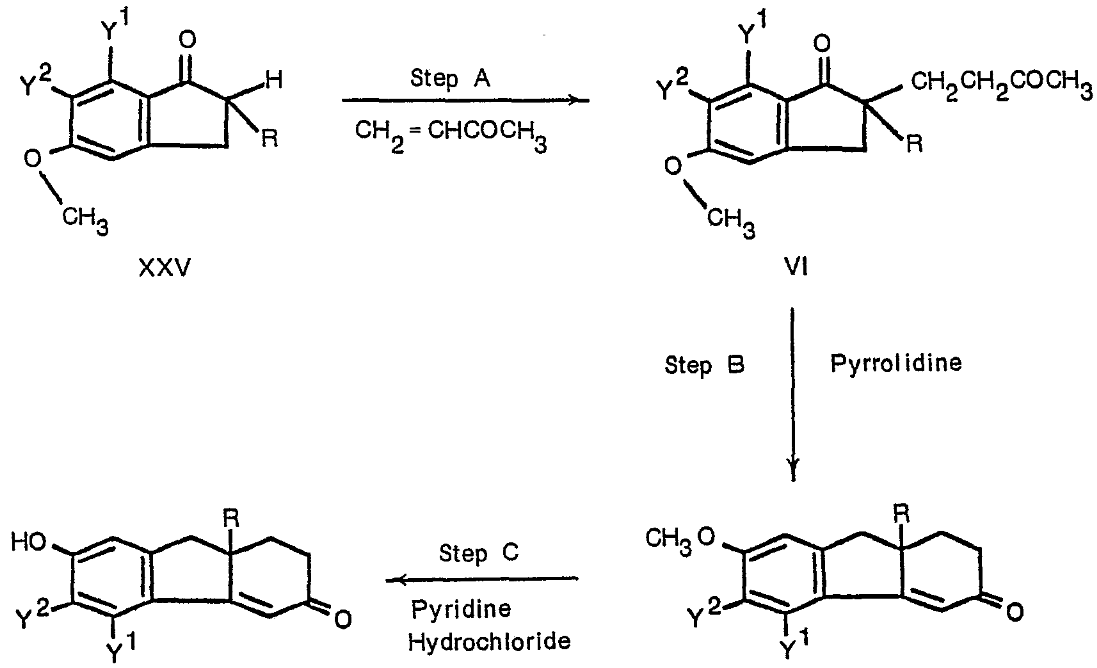


XXV

VI

VII

II

Compounds of the type represented by Formula XXV which are used to produce the compounds of this invention have generally been described in the scientific and/or patent literature. For those that have not been previously disclosed or must be prepared by new methods, a description of their synthesis will be described later.

The first step (Step A) in the synthesis involves the reaction of a compound of Formula XXV with methyl vinyl ketone to produce a compound of Formula VI. The reaction is catalyzed by a base, such as benzyltrimethylammonium hydroxide in methanol but other bases such as tetramethylammonium hydroxide, tetraethylammonium hydroxide or sodium methoxide are effective catalysts. Solvents which are inert to the reaction and are effective in dissolving the reactants and product are normally used. Tetrahydrofuran is an especially preferred solvent but others, like p-dioxane or 1,2-dimethoxyethane can be used. The reaction is generally conducted at ambient temperatures but temperatures in the range of 10°C to 45°C or somewhat above or below these values can be used.

The second step (Step B) in the synthesis is the cyclization of a compound of Formula VI to give a compound of Formula VII. The reaction is generally conducted in the presence of a solvent such as benzene, toluene, xylene, anisole and the like but many other solvents which are inert to the reactants and to the catalyst are useful. A catalyst is necessary for increasing the rate of reaction. Salts of weak organic acids and weak inorganic or organic bases are preferred; for example, pyrrolidine acetate, piperidine acetate, ammonium acetate, ammonium propionate, pyrrolidine propionate and the like. The reaction is preferably conducted at the temperature of the boiling solvent but temperature of 30°C—120°C can be used. It is advantageous to carry out the reaction under conditions in which the water produced in the reaction is constantly removed. This is effected by various means, such as using a Dean-Stark constant water separator or co-distilling the solvent and the water. Another device for removing the water is to add molecular sieves designed for removing water, for example, Davison Molecular Sieves of 3Å size M564 (Cation: potassium, base: alumina-silicate).

The last step (Step C) involves the ether cleavage of a compound of Formula VII to produce a compound of Formula II. This can be accomplished by many of the agents known to cleave ethers, especially useful are hydrohalide salts of weak bases, such as pyridine hydrochloride or pyridine hydrobromide, but other agents, such as aqueous hydrobromic acid or aluminum bromide can be used. When pyridine hydrohalides are used, the temperatures above that which these substances melt are generally employed. This usually involves temperatures in the range of 150°C to 215°C, but temperatures somewhat lower or higher can be used. The period of heating varies depending on the specific compound but periods of from 15 minutes to 2 hours may be used.

The second method for preparing compounds of Formula II is shown by the five-step reaction illustrated below. It should be mentioned that this is not simply a cyclical method whereby a compound of the invention (Formula V) is converted to one of its precursors (Formula II) simply to be reconverted to the product again (Formula V).

In this instance, a readily accessible compound of Formula VIII, for example, where the A moiety is $CH_2$, is converted to a compound of Formula V which, in turn, is converted to a compound of Formula II

which now can be used to synthesize a compound of Formula V which is difficultly accessible, i.e., wherein A is

$-CHF-$, $>C(CH_3)_2$, or $>C(CH_2)_3$.

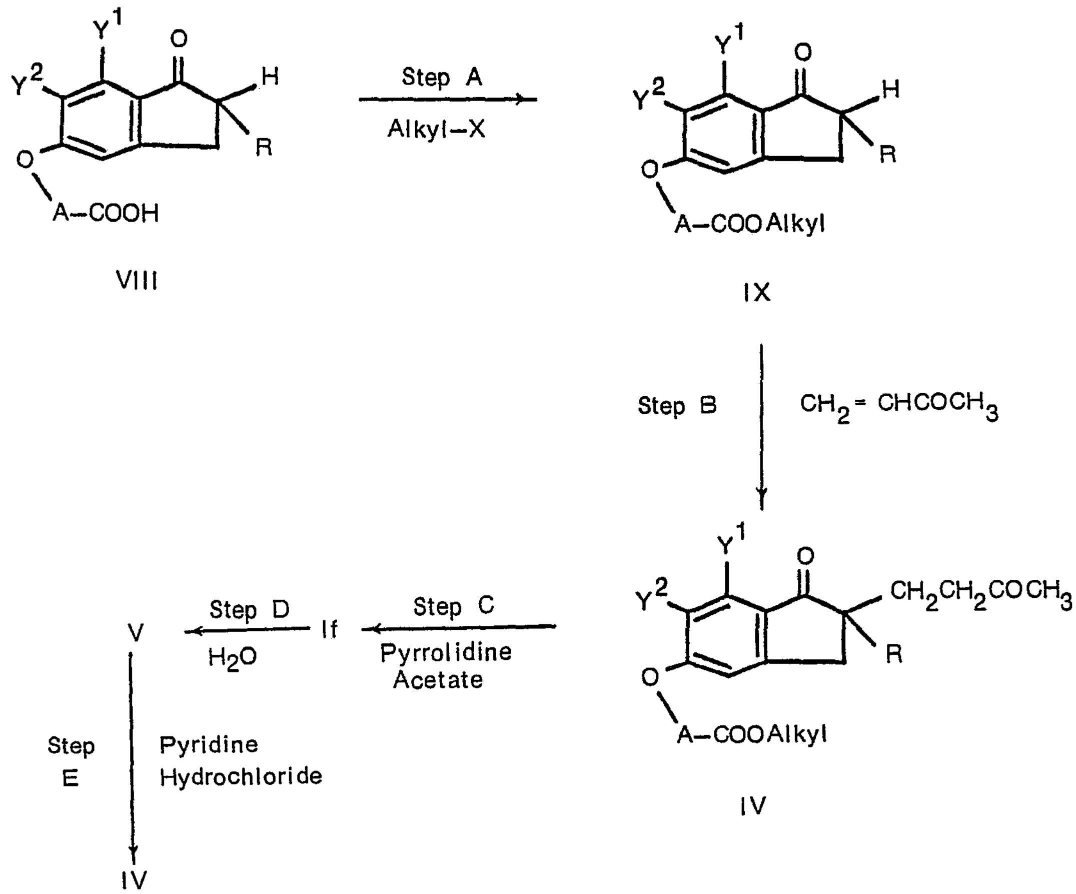


The first step (Step A) involves the esterification of compound of Formula VIII to produce the ester of Formula IX. The starting material, Formula VIII, is generally known; however, the synthesis of those which are not known or are accessible only by new methods will be discussed later. The esterification can be effected by many of the known methods. Especially useful is the procedure whereby compound of Formula VIII is reacted with an alkyl halide, such as methyl iodide, in the presence of an alkaline earth or alkali metal carbonate, such as potassium carbonate or sodium carbonate in the presence of a solvent such as dimethylformamide, 1-methylpyrrolidin-2-one, and the like to produce a compound of Formula IX. The reaction is generally conducted at a temperature of 30°C—60°C for from 3 to 20 hours. Alternatively, the esterification may be effected by the reaction of a compound of Formula VIII with an alkanol, such as methanol, ethanol or iospropyl alcohol in the presence of an acid catalyst. The acid catalyst may be a strong organic or inorganic acid, such as sulfuric acid, hydrochloric acid, p-toluenesulfonic acid and the like.

The second step (Step B) involves the reaction of a compound of Formula IX with methyl vinyl ketone to form a compound of Formula IV. The conditions for this reaction are similar to those described above for the conversion of a compound of Formula XXV to one of Formula VI.

The third step (Step C) is the cyclization of compounds of Formula IV to form those of Formula If. The conditions for this reaction are similar to those described for the conversion of compounds of Formula VI to those of Formula VII.

The fourth step (Step D) is the hydrolysis of compounds of Formula If to those of Formula V. The conditions of this reaction have been described earlier.

The final step (Step E) is the ether cleavage of a compound of Formula V to produce a compound of Formula II. The conditions for this reaction are similar to those described for the conversion of a compound of Formula VII to those of Formula II.

As indicated earlier, those compounds of Formula XXV and Formula VIII which are not known or are accessible only by new synthetic routes, may be prepared by methods disclosed in this invention which are illustrated below.

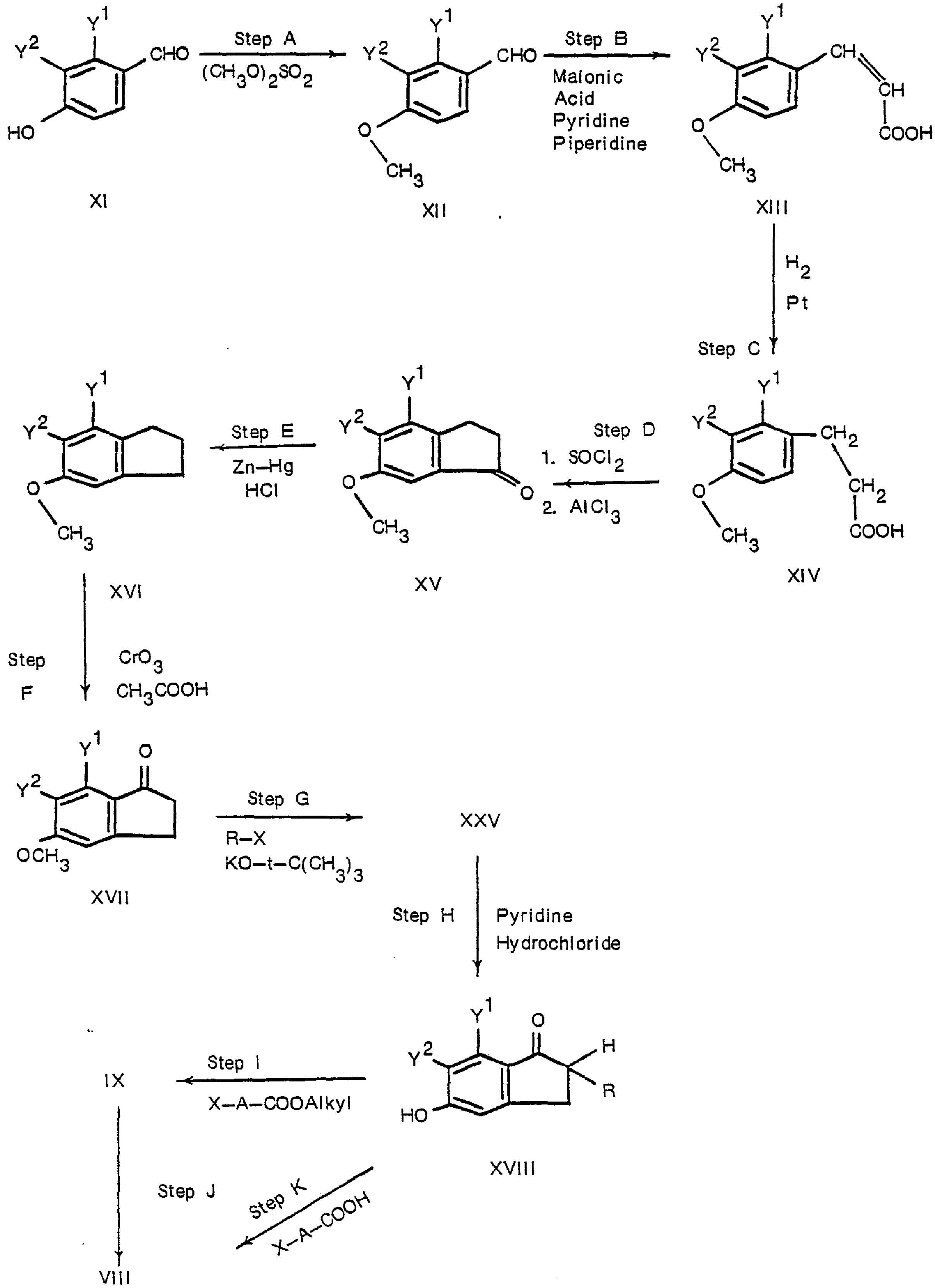


The first step is the etherification of a compound of Formula XI to produce a compound of Formula XII. This is accomplished by a reaction involving a compound of Formula XI in any one of a number of chemical processes; especially useful is dimethyl sulfate in the presence of potassium carbonate using a solvent, such as dimethylformamide.

The second step (Step B) is the conversion of a compound of Formula XII to one of Formula XIII. This can be accomplished by one or another of many methods; especially useful is the reaction of a compound of Formula XII with malonic acid in the presence of a solvent such as pyridine and a weak organic base, such as piperidine or pyrrolidine. The reaction is effected by heating for 1 to 6 hours at a temperature of 75°C to 120°C, preferably at about 95°C to 100°C. Quenching the reaction mixture in ice and acidification with a strong acid, such as hydrochloric acid yields the desired compound of Formula XIII.

The third step (Step C) involves the catalytic hydrogenation of a compound of Formula XIII to produce a compound of Formula XIV. The reduction is conducted in an atmosphere of hydrogen at an initial pressure of 0.7—7.0 kg/$cm^2$ and at temperatures of from 15°C to 45°C. A solvent in which the starting material and product are reasonably soluble and which is inert to the reactants, product and catalyst is employed. Solvents such as tetrahydrofuran, dioxane, and the like are especially useful. The catalyst is usually a finely divided noble metal (which may be on an inert support) i.e., palladium on charcoal or it may be a noble metal derivative which upon exposure to hydrogen is reduced to the free metal, i.e., platinum oxide.

The fourth step (Step D) consists of the cyclization of a compound of Formula XIV to produce a compound of Formula XV. This is accomplished by first converting a compound of Formula XIV to the corresponding acid chloride using a reagent like thionyl chloride or phosphorus oxychloride. A solvent, such as benzene is advantageous. The acid chloride is subjected to the conditions of the Friedel-Crafts reaction which results in the cyclization of the acid chloride to form a compound of Formula XV. A reagent such as aluminum chloride, stannic chloride and the like are used to effect the cyclization. The reaction is conducted in a typical Friedel-Crafts solvent, such as methylene chloride or carbon disulfide. The reaction time is generally 1 to 6 hours.

The fifth step (Step E) involves the reduction of a compound of Formula XV to one of Formula XVI. This reaction is accomplished by using zinc amalgam and hydrochloric acid. The reaction is generally conducted in a solvent such as a mixture of water and an alkanol, such as ethanol or 1-propanol. The reaction time is generally between 3 and 10 hours and is generally conducted at temperatures of 60°C to 110°C. The time and temperature are interdependent so that when the temperature is lower, the time is longer.

The sixth step (Step F) involves the oxidation of a compound of formula XVI to produce one of Formula XVII. The oxidation is best accomplished using a reagent such as chromium trioxide in a mixture of acetic acid and water. The reaction is advantageously conducted at ambient temperatures but temperatures somewhat higher or lower may be used.

The seventh step (Step G) consists of the alkylation of a compound of Formula XVII to produce one of Formula XXV. This first involves formation of the anion of a compound of Formula XVII by treatment with a base, such as an alkali metal alkoxide, for example, potassium tert.-butoxide in tert.-butyl alcohol. An alkali metal hydride, such as sodium hydride and the like, or an alkali metal amide, such as sodium amide, lithium amide and the like, also may be used. The anion is then treated with a compound of the formula RX, wherein R is as defined above and X is halo. Solvents which are inert to the reactants may be employed. Suitable solvents are 1,2-dimethoxyethane, tert.-butyl alcohol, dimethylformamide, benzene and the like. The reaction is conducted at a temperature in the range from about 25°C to about 150°C.

The eighth step (Step H) consists of the ether cleavage of a compound of Formula XXV to produce one of Formula XVIII. This reaction is carried out under conditions similar to those described earlier for the conversion of compounds of Formula VII to those of Formula II.

The ninth step (Step I) consists of the etherification of a compound of Formula XVIII to one of Formula IX. This reaction is carried out under conditions described for the conversion of compounds of Formula II to those of Formula Ib (vide supra).

Compound VIII may be prepared by one of two methods. The first method (Step J) involves the hydrolysis or pyrolysis of a compound of Formula IX to produce one of Formula VIII. This reaction is carried out by the methods described for the conversion of compounds of Formula If or Ig to those of Formula V.

The second method for the preparation of compounds of Formula VIII is the etherification of compounds of Formula XVIII. This reaction is carried out by the methods described for the conversion of compounds of Formula II to those of Formula V.

As mentioned earlier, the compounds of this invention possess one and sometimes two asymmetric carbon atoms. In the instances where they possess two asymmetric carbon atoms, the reaction whereby these chiral centers are established can produce two diastereomers. These may be separated to obtain each pure diastereomer by methods well known to those skilled in the art, such as by fractional crystallization, column chromatography, high pressure liquid chromatography and the like.

Those compounds possessing only one asymmetric carbon atom, as well as each pure diastereomer from compounds possessing two asymmetric carbon atoms, consist of a racemate composed of two enantiomers. The resolution of the two enantiomers may be accomplished by forming a salt of the racemic mixture with an optically active base such as (+) or (—) amphetamine, (—) cinchonidine, dehydroabietylamine, (+) or (—)-α-methylbenzylamine, (+) or (—)-α-(1-naphthyl)ethylamine, (+)

cinchonine, brucine, or strychnine and the like in a suitable solvent such as methanol, ethanol, 2-propanol, benzene, acetonitrile, nitromethane, acetone and the like. There is formed in the solution two diastereomeric salts one of which is usually less soluble in the solvent than the other. Repetitive recrystallization of the crystalline salt generally affords a pure diasteriomeric salt from which is obtained the desired pure enantiomer. The optically pure enantiomer of the [(5,6,9a-substituted-3-oxo-1,2,9,9a-tetrahydro-3H-fluoren-7-yl)oxy]alkanoic or cycloalkanoic acid is obtained by acidification of the salt with a mineral acid, isolation by filtration and recrystallization of the optically pure antipode.

The other optically pure antipode may generally be obtained by using a different base to form the diastereomeric salt. It is of advantage to isolate the partially resolved acid from the filtrates of the purification of the first diastereomeric salt and to further purify this substance through the use of another optically active base. It is especially advantageous to use an optically active base for the isolation of the second enantiomer which is the antipode of the base used for the isolation of the first enantiomer. For example, if (+)-α-methylbenzylamine was used first, then (—)-α-methylbenzylamine is used for the isolation of the second (remaining) enantiomer.

The preparation of derivatives of the carboxylic acids (Formula V) of the invention are accomplished by methods well known by those skilled in the art. For example, an anhydride of Formula I (see also Formula XIX) is prepared from a compound of Formula V by reaction with a carbodiimide in an organic solvent. For example,

A

V + Carbodiimide

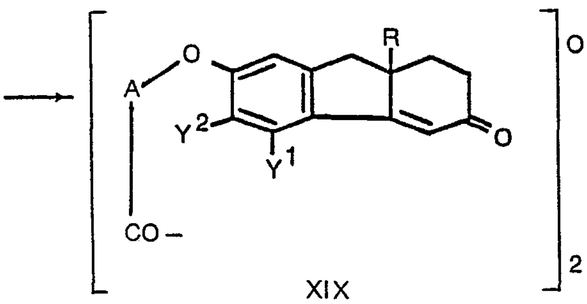

XIX

a compound of Formula V can be reacted with a carbodiimide, such as N,N'-dicyclohexylcarbodiimide or N,N'-di-p-tolylcarbodiimide in a solvent such as benzene, chlorobenzene, methylene chloride or dimethylformamide to produce a compound of Formula XIX.

The amide, hydrazide, guanidide and the like derivatives of Formula I (see also Formula XX) which are compounds of the invention are prepared by methods well known to those skilled in the art. For example, a compound of Formula V is converted to the acylimidazole (Ie) (described above) which, in turn, is treated

Ie

$R^4R^5NH$

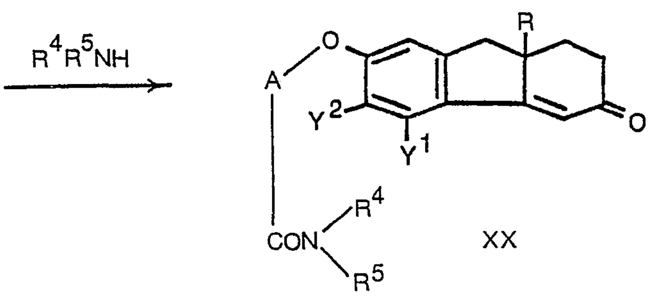

XX

with the appropriate base ($R^4R^5NH$) to obtain the desired derivative (Formula XX). Thus, if $R^4R^5NH$ represents ammonia or an amine, i.e., where $R^4$ and $R^5$ are hydrogen or lower alkyl, and the like, the product is an amide. If $R^4R^5NH$ represents a hydrazide, i.e., $R^4$ is amino or dilower-alkylamino and $R^5$ is hydrogen or lower alkyl, the product is a hydrazide. If $R^4$ is

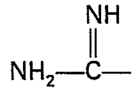

and $R^5$ is hydrogen the product is guanidide.

The reactions described may be conducted in an excess of the reactant base as a solvent or one may use a conventional solvent, such as dimethylformamide, 1-methylpyrrolidin-2-one and the like.

The acid addition salts of Formula XXI (where $B^+$ represents a cation from a pharmaceutically acceptable base) are prepared by reacting a carboxylic acid of Formula V with an appropriate alkali

metal or alkaline earth bicarbonate, carbonate or alkoxide, an amine, ammonia, an organic quaternary ammonium hydroxide, guanidine and the like. The reaction is illustrated below:

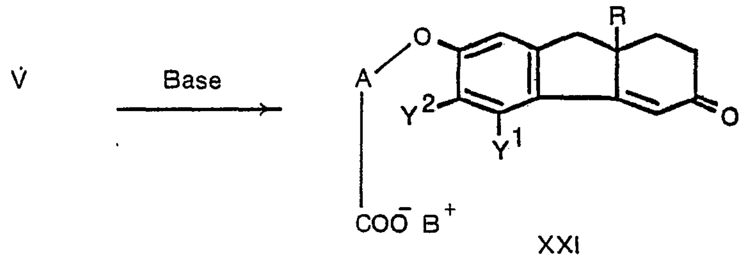


XXI

The reaction is generally conducted in water but when alkali metal hydroxides and alkoxides and the organic bases are used, the reaction can be conducted in an organic solvent, such as ethanol, dimethylformamide and the like.

The preferred salts are the sodium, ammonium, diethanolamine, 1-methylpiperazine, piperazine and the like salts.

Inasmuch as there is a wide variety of symptoms and severity associated with grey matter edema, particularly when it is caused by blows to the head or spinal chord, the precise treatment protocol is left to the practitioner. It is up to the practitioner to determine the patient's rèsponse to treatment and to vary the dosages accordingly. A recommended dosage range is from 1 μg. to 10 mg./kg of body weight as a primary dose and a sustaining dose of half to equal the primary dose, every 4 to 24 hours.

The compounds of this invention can be administered by a variety of established methods, including intravenously, intramuscularly, subcutaneously, and orally. As with dosage, the precise mode of administration is left to the discretion of the practitioner.

Recent studies in experimental head injury by R. S. Bourke et. al. (R. S. Bourke, M. A. Dazé and H. K. Kimelberg, Monograph of the International Glial Cell Symposium, Leige, Belgium, August 29—31, 1977 and references cited therein) and experimental stroke by J.H. Garcia et al. (J.H. Garcia, H. Kalimo, Y. Kamijyo and B.F. Trump, Virchows Archiv. [Zellopath.], *25*, 191 (1977), indicate that astroglial swelling, a secondary and potentially inhibitable process, is a fundamental pathophysiological response to ischemic/traumatic brain insult in both pathological disorders. Furthermore, astroglial swelling is believed to reduce oxygen available to neurons by prolongation of the oxygen diffusion pathaway. Thus, the damage to cerebral grey matter may be far more extensive as a result of pathological events secondary to astroglial swelling than as a result of damage inflicted by the initial ischemic/traumatic insult. Consequently, it is of prime importance that the treatment commence as soon as possible after the initial trauma in order to minimize the brain cell damage and the possibility of death or permanent paralysis.

One aspect of the present invention is, as outlined before, a composition in unit dosage form for the treatment of grey matter edema which comprises as active ingredient a compound of formula I or a pharmaceutically acceptable salt thereof. Accordingly, the active ingredient of this pharmaceutical composition can be an ester of formula Ib, or the free acid of formula V, i.e. a compound of formula I wherein X is hydroxy, the corresponding acid derivatives of formula I wherein X is an acid anhydride, amide, hydrazide or guanidide, i.e. the corresponding compounds having e.g. the structures XIX or XX given before as well as also acid addition salts having the formula XXI stated before. Preferably the pharmaceutical compositions comprise in addition to the inventive compound of formula I also an antiinflammatory steroid. These steroids are of some, albeit limited, use in control of white matter edema associated with ischemic stroke and head injury. Therefore, it is sometimes advantageous to administer the inventive compounds of formula I together with such a steroid.

The inventive pharmaceutical composition can be made by formulating the compounds of formula I, or pharmaceutically acceptable salts thereof in a composition such as tablet, capsule or elixir for oral administration. Sterile solutions or suspensions can be used for parenteral administration. About 70 μg to 750 mg. of a compound or mixture of compounds of formula I or a physiologically acceptable salt thereof, is compounded with a physiologically acceptable vehicle, carrier, excipient, binder, preservative, stabilizer, flavor, etc. in a unit dosage form as called for by accepted pharmaceutical practice. The amount of active substance in the composition is such that dosage in the range indicated is obtained.

Illustrative of the adjuvants which may be incorporated in tablets, capsules and the like are the following: a binder such as gum tragacanth, acacia, corn starch or gelatin; an excipient such as dicalcium phosphate; a disintegrating agent such as corn starch, potato starch, alginic acid and the like; a lubricant such as magnesium stearate; a sweetening agent such as sucrose, lactose, or saccharin; a flavoring agent such as peppermint, oil of wintergreen or cherry. When the dosage unit form is a capsule, it may contain in addition to materials of the above type a liquid carrier such as a fatty oil. Various other materials may be present as coatings or to otherwise enhance the pharmaceutical elegance of the preparation. For instance, tablets may be coated with shellac, sugar or the like. A syrup

or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a conventional vehicle such as water for injection, a naturally occurring vegetable oil like sesame oil, coconut oil, peanut oil, cottonseed oil, etc., or a synthetic fatty vehicle like ethyl oleate or the like. Buffers, preservatives, antioxidants and the like can be incorporated as required.

The following examples are included to illustrate the preparation of representative compounds of formula I.

Example 1

[(5,6-Dichloro-9a-ethyl-3-oxo-1,2,9,9a-tetrahydro-3H-fluoren-7-yl)oxy]acetic Acid

*Steps A and B.* 5,6-Dichloro-9a-ethyl-7-methoxy-1,2,9,9a-tetrahydro-3H-fluoren-3-one

6,7-Dichloro-2-ethyl-5-methoxy-2,3-dihydro-1H-inden-1-one (32.4 gm., 0.125 mole) is dissolved in tetrahydrofuran (300 ml.) and 40% "Triton-B" (registered Trade Mark) in methanol (3 ml.) is added. Methyl vinyl ketone (12.3 gm., 0.175 moles) is added dropwise to the stirring reaction mixture over a period of 10 minutes. The temperature rises from 26°C. to 35°C. The mixture is stirred for an additional hour and most of the volatile material is removed by evaporation *in vacuo* using a rotary evaporator.

Water (200 ml.) is added and the mixture extracted with ether (three 100 ml. portions). The combined ether extracts are dried over $Na_2SO_4$ and then evaporated *in vacuo* to give a residual oil which is 6,7-dichloro-2-ethyl-5-methoxy-2-(3-oxobutyl)-2,3-dihydro-1H-inden-1-one. This material is dissolved in dry benzene (200 ml.), placed in a constant water separator (Dean-Stark), treated with acetic acid (1.5 ml.) and pyrrolidine (1.5 ml.). The mixture is refluxed for two hours after which time water ceases to separate. Then the solvent is distilled until only 50 ml. remains and then methanol (100 ml.) is added. Upon chilling in an ice bath, 5,6-dichloro-9a-ethyl-7-methoxy-1,2,9,9a-tetrahydro-3H-fluoren-3-one (16.2 gm.) separates, m.p. 159°—165°C. This material is satisfactory for use in the next step.

*Step C.* 5,6-Dichloro-9a-ethyl-7-hydroxy-1,2,9,9a-tetrahydro-3H-fluoren-3-one

5,6-Dichloro-9a-ethyl-7-methoxy-1,2,9,9a-tetrahydro-3H-fluoren-3-one (15 gm., 0.048 mole) is mixed with pyridine hydrochloride (120 gm.) in a reaction vessel and heated in a metal bath at 195°—200°C. for 30 minutes. The mixture is cooled somewhat and poured with stirring into cold water (800 ml.). The solid that separates is removed by filtration and washed with water. After drying, the product is triturated with acetonitrile (80 ml.) at 60°, cooled, filtered and dried. The yield of crude 5,6 - dichloro - 9a - ethyl - 7 - hydroxy - 1,2,9,9a - tetrahydro - 3H - fluoren - 3 - one is 12.2 gm., m.p. 260°—265°C. This material is pure enough for use in the next step.

*Step D.* Ethyl [(5,6-Dichloro-9a-ethyl-3-oxo-1,2,9,9a-tetrahydro-3H-fluoren-7-yl)oxy]acetate

A mixture of 5,6-dichloro-9a-ethyl-7-hydroxy-1,2,9,9a-tetrahydro-3H-fluoren-3-one (6.0 gm., 0.02 mole), ethyl bromoacetate (4.0 gm., 0.025 mole) and potassium carbonate (8.3 gm., 0.06 mole) in dimethylformamide (35 ml.) is heated at 50°C. and stirred for one hour. The mixture is cooled and poured into water (300 ml.). The solid product that separates is removed by filtration, then washed with water followed by benzene (10 ml.).

The yield of ethyl [(5,6-dichloro-9a-ethyl-3-oxo-1,2,9,9a-tetrahydro-3H-fluoren-7-yl)oxy]acetate after drying, is 6.9 gm. After two recrystallizations from acetonitrile 4.8 gm. remains, m.p. 164°—166°C.

*Step E.* [(5,6-Dichloro-9a-ethyl-3-oxo-1,2,9,9a-tetrahydro-3H-fluoren-7-yl)oxy]acetic Acid

Ethyl [(5,6-dichloro-9a-ethyl-3-oxo-1,2,9,9a-tetrahydro-3H-fluoren-7-yl)oxy]acetate (4.8 gm., 0.012 mole) is dissolved in acetic acid (30 ml.) containing 5% aqueous hydrochloric acid (10 ml.). The mixture is heated and stirred on a steam bath for an hour. The solution is diluted with water (8 ml.) and cooled. The product that separates is recrystallized twice from a 1:6 (v/v) mixture of water and acetic acid. The yield of [(5,6-dichloro-9a-ethyl-3-oxo-1,2,9,9a-tetrahydro-3H-fluoren-7-yl)-oxy]acetic acid is 2.6 gm., m.p. 237°—238°C.

Anal. Calcd. for $C_{17}H_{16}Cl_2O_4$: C, 57.48; H, 4.54.
Found: C, 57.97; H, 4.43.
C, 57.21; H, 4.29.

Using a procedure similar to that described in Example 1, except in Step A there is substituted for the 6,7-dichloro-2-ethyl-5-methoxy-2,3-dihydro-1H-inden-1-one an equimolar amount of:

Example 2

*Step A.* 6,7-Dichloro-5-methoxy-2,3-dihydro-1H-inden-1-one.

Example 3

*Step A.* 6,7-Dichloro-5-methoxy-2-methyl-2,3-dihydro-1H-inden-1-one.

Example 4

*Step A.* 6,7-Dichloro-5-methoxy-2-propyl-2,3-dihydro-1H-inden-1-one.

Example 5

*Step A.* 6,7-Dichloro-2-isopropyl-5-methoxy-2,3-dihydro-1H-inden-1-one.

Example 6

*Step A.* 2-Butyl-6,7-dichloro-5-methoxy-2,3-dihydro-1H-inden-1-one.

Example 7

*Step A.* 6,7-Dichloro-2-isobutyl-5-methoxy-2,3-dihydro-1H-inden-1-one.

Example 8

*Step A.* 6,7-Dichloro-2-cyclopentyl-5-methoxy-2,3-dihydro-1H-inden-1-one.

Example 9

*Step A.* 2-Benzyl-6,7-dichloro-5-methoxy-2,3-dihydro-1H-inden-1-one.

Example 10

*Step A.* 6,7-Dichloro-2-phenyl-5-methoxy-2,3-dihydro-1H-inden-1-one.

In Examples 2, Step A through Example 10, Step A, the reaction is carried out as described in Example 1, Step A, then by using each product and conducting the reaction as in Example 1, Step B, there is obtained:

Example 2

*Steps A and B:* 6,7-Dichloro-5-methoxy-2-(3-oxobutyl-2,3-dihydro-1H-inden-1-one and then 5,6-dichloro-7-methoxy-1,2,9,9a-tetrahydro-3H-fluoren-3-one.

Example 3

*Steps A and B.* 6,7-Dichloro-5-methoxy-2-methyl-2-(3-oxobutyl)-2,3-dihydro-1H-inden-1-one and then 5,6-dichloro-7-methoxy-9a-methyl-1,2,9,9a-tetrahydro-3H-fluoren-3-one.

Example 4

*Steps A and B.* 6,7-Dichloro-5-methoxy-2-(3-oxobutyl)-2-propyl-2,3-dihydro-1H-inden-1-one and then 5,6-dichloro-7methoxy-9a-propyl-1,2,9,9a-tetrahydro-3H-fluoren-3-one.

Example 5

*Steps A and B.* 6,7-Dichloro-2-isopropyl-5-methoxy-2-(3-oxobutyl)-2,3-dihydro-1H-inden-1-one and then 5,6-dichloro-9a-isopropyl-7-methoxy-1,2,9,9a-tetrahydro-3H-fluoren-3-one.

Example 6

*Steps A and B.* 2-Butyl-6,7-dichloro-5-methoxy-2-(3-oxobutyl)-2,3-dihydro-1H-inden-1-one and then 5,6-dichloro-9a-butyl-7-methoxy-1,2,9,9a-tetrahydro-3H-fluoren-one.

Example 7

*Steps A and B.* 6,7-Dichloro-2-isobutyl-5-methoxy-2-(3-oxobutyl)-2,3-dihydro-1H-inden-1-one and then 5,6-dichloro-9a-isobutyl-7-methoxy-1,2,9,9a-tetrahydro-3H-fluoren-3-one.

Example 8

*Steps A and B.* 6,7-Dichloro-2-cyclopentyl-5-methoxy-2(3-oxobutyl)-2,3-dihydro-1H-inden-1-one and then 5,6-dichloro-9a-cyclopentyl-7-methoxy-1,2,9,9a-tetrahydro-3H-fluoren-3-one.

Example 9

*Steps A and B.* 2-Benzyl-6,7-dichloro-5-methoxy-2-(3-oxobutyl)-2,3-dihydro-1H-inden-1-one and then 5,6-dichloro-9a-benzyl-7-methoxy-1,2,9,9a-tetrahydro-3H-fluoren-3-one.

Example 10

*Steps A and B.* 6,7-Dichloro-5-methoxy-2-(3-oxobutyl)-2-phenyl-2,3-dihydro-1H-inden-1-one and then 5,6-dichloro-7-methoxy-9a-phenyl-1,2,9,9a-tetrahydro-3H-fluoren-3-one.

Carrying out the reaction as described in Example 1, Step C, except that the 5,6-dichloro-9a-ethyl-7-methoxy-1,2,9,9a-tetrahydro-3H-fluoren-3-one of Example 1, Step C is substituted by an equimolar quantity of the product of Example 2, Step B, Example 3, Step B, Example 4, Step B, Example 5,

Step B, Example 6, Step B, Example 7, Step B, Example 8, Step B, Example 9, Step B, and Example 10, Step B to obtain:

Example 2

*Step. C.* 5,6-Dichloro-7-hydroxy-1,2,9,9a-tetrahydro-3H-fluoren-3-one.

Example 3

*Step C.* 5,6-Dichloro-7-hydroxy-9a-methyl-1,2,9,9a-tetrahydro-3H-fluoren-3-one.

Example 4

*Step C.* 5,6-Dichloro-7-hydroxy-9a-propyl-1,2,9,9a-tetrahydro-3H-fluoren-3-one.

Example 5

*Step C.* 5,6-Dichloro-7-hydroxy-9a-isopropyl-1,2,9,9a-tetrahydro-3H-fluoren-3-one.

Example 6

*Step C.* 9a-Butyl-5,6-dichloro-7-hydroxy-1,2,9,9a-tetrahydro-3H-fluoren-3-one.

Example 7

*Step C.* 5,6-Dichloro-9a-isobutyl-7-hydroxy-1,2,9,9a-tetrahydro-3H-fluoren-3-one.

Example 8

*Step C.* 5,6-Dichloro-9a-cyclopentyl-7-hydroxy-1,2,9,9a-tetrahydro-3H-fluoren-3-one.

Example 9

*Step C.* 9a-Benzyl-5,6-dichloro-7-hydroxy-1,2,9,9a-tetrahydro-3H-fluoren-3-one.

Example 10

*Step C.* 5,6-Dichloro-7-hydroxy-9a-phenyl-1,2,9,9a-tetrahydro-3H-fluoren-3-one.

Carrying out the reaction as described in Example 1, Step D, except that the 5,6-dichloro-9a-ethyl-7-hydroxy-1,2,9,9a-tetrahydro-3H-fluoren-3-one of Example 1, Step D, is substituted by an equimolar quantity of the product of Example 2, Step C, Example 3, Step C, Example 4, Step C, Example 5, Step C, Example 6, Step C, Example 7, Step C, Example 8, Step C, Example 9, Step C or Example 10, Step C to obtain:

Example 2

*Step D.* Ethyl [(5,6-dichloro-3-oxo-1,2,9,9a-tetrahydro-3H-fluoren-7-yl)oxy]acetate.

Example 3

*Step D.* Ethyl [(5,6-dichloro-9a-methyl-3-oxo-1,2,9,9a-tetrahydro-3H-fluoren-7-yl)oxy]acetate.

Example 4

*Step D.* Ethyl [(5,6-dichloro-3-oxo-9a-propyl-1,2,9,9a-tetrahydro-3H-fluoren-7-yl)oxy]acetate.

Example 5

*Step D.* Ethyl [(5,6-dichloro-9a-isopropyl-3-oxo-1,2,9,9a-tetrahydro-3H-fluoren-7-yl)oxy]acetate.

Example 6

*Step D.* Ethyl [(9a-Butyl-5,6-dichloro-3-oxo-1,2,9,9a-tetrahydro-3H-fluoren-7-yl)oxy]acetate.

Example 7

*Step D.* Ethyl [(5,6-dichloro-9a-isobutyl-3-oxo-1,2,9,9a-tetrahydro-3H-fluoren-7-yl)oxy]acetate.

Example 8

*Step D.* Ethyl [(5,6-dichloro-9a-cyclopentyl-3-oxo-1,2,9,9a-tetrahydro-3H-fluoren-7-yl)oxy]acetate.

Example 9

*Step D.* Ethyl [(9a-Benzyl-5,6-dichloro-3-oxo-1,2,9,9a-tetrahydro-3H-fluoren-7-yl)oxy]acetate.

Example 10

*Step D.* Ethyl [(5,6-dichloro-9a-phenyl-3-oxo-1,2,9,9a-tetrahydro-3H-fluoren-7-yl)oxy]acetate.

Carrying out the reaction as described in Example 1, Step E, except that the ethyl [(5,6-dichloro-9a-ethyl-3-oxo-1,2,9,9a-tetrahydro-3H-fluoren-7-yl)oxy]acetate of Example 1, Step E is substituted by an equimolar quantity of the product of Example 2, Step D, Example 3, Step D, Example 4, Step D, Example 5, Step D, Example 6, Step D, Example 7, Step D, Example 8, Step D, Example 9, Step D or Example 10, Step D, there is obtained:

Example 2

*Step E.* [(5,6-Dichloro-3-oxo-1,2,9,9a-tetrahydro-3H-fluoren-7-yl)oxy]acetate acid.

Example 3

*Step E.* [(5,6-Dichloro-9a-methyl-3-oxo-1,2,9,9a-tetrahydro-3H-fluoren-7-yl)oxy]acetate acid.

Example 4

*Step E.* [(5,6-Dichloro-9a-propyl-3-oxo-1,2,9,9a-tetrahydro-3H-fluoren-7-yl)oxy]acetate acid.

Example 5

*Step E.* [(5,6-Dichloro-3-oxo-9a-isopropyl-1,2,9,9a-tetrahydro-3H-fluoren-7-yl)oxy]acetate acid.

Example 6

*Step E.* [(9a-Butyl-5,6-dichloro-3-oxo-1,2,9,9a-tetrahydro-3H-fluoren-7-yl)oxy]acetate acid.

Example 7

*Step E.* [(5,6-Dichloro-9a-isobutyl-3-oxo-1,2,9,9a-tetrahydro-3H-fluoren-7-yl)oxy]acetate acid.

Example 8

*Step E.* [(5,6-Dichloro-9a-cyclopentyl-3-oxo-1,2,9,9a-tetrahydro-3H-fluoren-7-yl)oxy]acetate acid.

Example 9

*Step E.* [(9a-Benzyl-5,6-dichloro-3-oxo-1,2,9,9a-tetrahydro-3H-fluoren-7-yl)oxy]acetate acid.

Example 10

*Step E.* [(5,6-Dichloro-3-oxo-9a-phenyl-1,2,9,9a-tetrahydro-3H-fluoren-7-yl)oxy]acetate acid.

By carrying out the reaction as described in Example 1, Step D, except that the ethyl bromoacetate is substituted by an equimolar quantity of:

Example 11

*Step A.* Ethyl 2-bromopropanoate.

Example 12

*Step A.* Ethyl 2-bromobutyrate.

Example 13

*Step A.* Ethyl 2-bromo-2-methylpropanoate.

Example 14

*Step A.* Ethyl 1-bromocyclobutane-1-carboxylate.
There is obtained:

Example 11

*Step A.* Ethyl [(5,6-dichloro-9a-ethyl-3-oxo-1,2,9,9a-tetrahydro-3H-fluoren-7-yl)oxy]propanoate.

Example 12

*Step A.* Ethyl 2-[(5,6-dichloro-9a-ethyl-3-oxo-1,2,9,9a-tetrahydro-3H-fluoren-7-yl)oxy]butyrate.

Example 13

*Step A.* Ethyl 2-methyl-2-[(5,6-dichloro-9a-ethyl-3-oxo-1,2,9,9a-tetrahydro-3H-fluoren-7-yl)oxy]-propanoate.

Example 14

*Step A.* Ethyl 1-[(5,6-dichloro-9a-methyl-3-oxo-1,2,9,9a-tetrahydro-3H-fluoren-7-yl)oxy]cyclobutane-1-carboxylate.

By carrying out the reaction as described in Example 1, Step D, except that the 5,6-dichloro-9a-ethyl-7-hydroxy-1,2,9,9a-tetrahydro-3H-fluoren-3-one is substituted by 5,6-dichloro-7-hydroxy-9a-methyl-1,2,9,9a-tetrahydro-3H-fluoren-3-one (Example 3, Step C) and the ethyl bromoacetate substituted in Example 15, Step A: ethyl 2-bromo-2-methylpropanoate Example 16, Step A: ethyl 1-bromocyclobutane-1-carboxylate. There is obtained:

Example 15

*Step A.* Ethyl 2-methyl-2-[(5,6-dichloro-9a-methyl-3-oxo-1,2,9,9a-tetrahydro-3H-fluoren-7-yl)oxy]-propanoate.

Example 16

*Step A.* Ethyl 1-[(5,6-dichloro-9a-methyl-3-oxo-1,2,9,9a-tetrahydro-3H-fluoren-7-yl)oxy]cyclobutane-1-carboxylate.

By carrying out the reaction as described in Example 1, Step E, except that the ethyl [(5,6-dichloro-9a-ethyl-3-oxo-1,2,9,9a-tetrahydro-3H-fluoren-7-yl)oxy]acetate is substituted by the product of Example 11, Step A, Example 12, Step A, Example 13, Step A, Example 14, Step A, Example 15, Step A, or Example 16, Step A, whereby there is obtained:

Example 11

*Step B.* 2-[(5,6-Dichloro-9a-ethyl-3-oxo-1,2,9,9a-tetrahydro-3H-fluoren-7-yl)oxy]propanoic acid.

Example 12

*Step B.* 2-[(5,6-Dichloro-9a-ethyl-3-oxo-1,2,9,9a-tetrahydro-3H-fluoren-7-yl)oxy]butyric acid.

Example 13

*Step B.* 2-Methyl-2-[(5,6-dichloro-9a-ethyl-3-oxo-1,2,9,9a-tetrahydro-3H-fluoren-7-yl)oxy]propanoic acid.

Example 14

*Step B.* 1-[(5,6-Dichloro-9a-ethyl-3-oxo-1,2,9,9a-tetrahydro-3H-fluoren-7-yl)oxy]cyclobutane-1-carboxylic acid.

Example 15

*Step B.* 2-Methyl-2-[(5,6-dichloro-9a-methyl-3-oxo-1,2,9,9a-tetrahydro-3H-fluoren-7-yl)oxy]-propanoic acid.

Example 16

*Step B.* 1-[(5,6-Dichloro-9a-methyl-3-oxo-1,2,9,9a-tetrahydro-3H-fluoren-7-yl)oxy]cyclobutane-1-carboxylic acid.

Example 17

3-[(5,6-Dichloro-9a-methyl-3-oxo-1,2,9,9a-tetrahydro-3H-fluoren-7-yl)oxy]propanoic acid

5,6-Dichloro-9a-methyl-7-hydroxy-1,2,9,9a-tetrahydro-3H-fluoren-3-one (9.0 gm., 0.03 mole) is dissolved in 10% aqueous sodium hyroxide solution (35 ml.). The solution is heated to boiling with stirring, the heat source is removed and $\beta$-propiolactone (23.8 gm., 0.33 mole) is added at such a rate to keep the solution boiling. During the reaction, 10% aqueous sodium hydroxide is added as necessary to keep the reaction mixture alkaline to litmus paper.

When the reaction is complete, the solution is cooled and made acid to Congo red paper with 6 normal hydrochloric acid. The product that separates is removed by filtration and dissolved by treatment with a 5% solution of sodium hydroxide (three 50 ml. portions). The combined aqueous extracts are acidified to congo red paper with 6 normal hydrochloric acid. The product is removed by filtration, washed with water and dried. Recrystallization from a mixture of acetic acid and water giving pure 3-[(5,6-dichloro-9a-methyl-3-oxo-1,2,9,9a-tetrahydro-3H-fluoren-7-yl)oxy]propanoic acid.

Carrying out the reaction as described in Example 17 except that the 5,6-dichloro-9a-methyl-7-hydroxy-1,2,9,9a-tetrahydro-3H-fluoren-3-one is substituted by an equimolar quantity of:

Example 18

5,6-Dichloro-9a-ethyl-7-hydroxy-1,2,9,9a-tetrahydro-3H-fluoren-3-one.

Example 19

5,6-Dichloro-9a-isopropyl-7-hydroxy-1,2,9,9a-tetrahydro-3H-fluoren-3-one.

There is obtained:

Example 18

3-[(5,6-Dichloro-9a-ethyl-3-oxo-1,2,9,9a-tetrahydro-3H-fluoren-7-yl)oxy]propanoic acid.

Example 19

3-[(5,6-Dichloro-9a-isopropyl-3-oxo-1,2,9,9a-tetrahydro-3H-fluoren-7-yl)oxy]propanoic acid.

Example 20

[(5,6-Dichloro-9a-ethyl-3-oxo-1,2,9,9a-tetrahydro-3H-fluoren-7-yl)oxy]acetate acid

*Step A.* Methyl [(6,7-dichloro-2-ethyl-1-oxo-2,3-dihydro-1H-inden-5-yl)oxy]acetate

[(6,7-Dichloro-2-ethyl-1-oxo-2,3-dihydro-1H-inden-5-yl)oxy]acetic acid (31.7 gm. 0.1 mole) is dissolved in dimethylformamide (300 ml.) and potassium carbonate (20.7 gm., 0.15 mole) is added. The mixture is stirred and heated at 60°C. for 10 minutes in a vessel protected from atmospheric moisture. Methyl iodide (12.6 ml., 28.4 gm., 0.2 mole) is added and heating and stirring continued for 3 hours. The mixture is cooled, added, with stirring to water (3 liters) and the solid that separates

removed by filtration and dried. Recrystallization from methanol gives 27 gm. of methyl [(6,7-dichloro-2-ethyl-1-oxo-2,3-dihydro-1H-inden-5-yl)oxy]acetate.

*Step B.* Methyl [(6,7-dichloro-2-ethyl-1-oxo-2-(3-oxobutyl)-2,3-dihydro-1H-inden-5-yl)oxy]acetate

Methyl [(6,7-dichloro-2-ethyl-1-oxo-2,3-dihydro-1H-inden-5-yl)oxy]acetate (15.8 gm., 0.05 mole) is suspended in dry tetrahydrofuran (100 ml.) containing triton-B (1 ml.). The suspension is stirred at ambient temperature and methyl vinyl ketone (5.02 gm., 0.072 mole) is added. The solution which becomes warm initially, is stirred at ambient temperature for 4 hours. Then, the stirring solution is treated with methyl vinyl ketone (0.3 ml.) and triton-B (0.6 ml.) every 4 hours for the next twenty hours.

The reaction mixture is evaporated *in vacuo* at 50°C and the residual material dissolved in ether (100 ml.). The ether solution is washed with water (two 20 ml. portions), dried over anhydrous magnesium sulfate and filtered. The filtrate is evaporated *in vacuo* and the residue triturated with ether (10 ml.), filtered and dried. The yield of methyl {[6,7-dichloro-2-ethyl-1-oxo-2-(3-oxobutyl)-2,3-dihydro-1H-inden-5-yl]oxy}acetate is 12.3 gm., m.p. 78°—79°C. This material is adequate for use in the next step.

*Step C.* Methyl [(5,6-dichloro-9a-ethyl-3-oxo-1,2,9,9a-tetrahydro-3H-fluoren-7-yl)oxy]acetate

A mixture of methyl [(6,7-dichloro-2-ethyl-1-oxo-2-(3-oxobutyl)-2,3-dihydro-1H-inden-5-yl)-oxy]acetate (9.0 gm., 0.024 mole), pyrrolidine (1.70 gm., 0.024 mole) and acetic acid (1.42 gm., 0.024 mole) in toluene (100 ml.) is heated at 85°C. for one hour. The solution is concentrated *in vacuo* at 50°C. to obtain the crude product.

The product is subjected to column chromatography on silica gel (300 gm.) using a mixture of dichloromethane and tetrahydrofuran (100/4 v./v.) as the eluent. The yield of pure methyl [(5,6-dichloro-9a-ethyl-3-oxo-1,2,9,9a-tetrahydro-3H-fluoren-7-yl)oxy]acetate is 5.05 gm., m.p. 182°—183°C.

*Step D.* [(5,6-Dichloro-9a-ethyl-3-oxo-1,2,9,9a-tetrahydro-3H-fluoren-7-yl)oxy]acetate acid

Methyl [(5,6-dichloro-9a-ethyl-3-oxo-1,2,9,9a-tetrahydro-3H-fluoren-7-yl)oxy]acetate (4.0 gm., 0.011 mole) is added to a solution composed of 20% aqueous sodium hydroxide solution (4.32 ml. 0.022 mole) and methanol (45 ml.). The mixture is stirred and heated at reflux for two hours and then concentrated *in vacuo* at 50°C. The residue is dissolved in water (50 ml.) and made acid to Congo red paper with 6 normal hydrochloric acid. The solid that separates is removed by filtration, washed with water, then with a little methanol and finally with a little ether. After drying, the product weights 3.14 gm., m.p. 235°C. After recrystallization from acetic acid, the yield of pure [(5,6-dicyhloro 9a-ethyl-3-oxo-1,2,9,9a-tetrahydro-3H-fluoren-7-yl)oxy]acetate acid is 2.61 gm., m.p. 237°—239°C.

Anal. Calcd. for $C_{17}H_{16}Cl_2O_4$: C, 57.48; H, 4.54.
Found: C, 57.35; H, 4.62.

Carrying out the reaction as described in Example 20, Step A, except that the [(6,7-dichloro-2-ethyl-1-oxo-2,3-dihydro-1H-inden-5-yl)oxy]acetic acid is substituted by an equimolar quantity of:

Example 21

*Step A.* [(6,7-Dichloro-2-propyl-1-oxo-2,3-dihydro-1H-inden-5-yl)oxy]acetic acid.

Example 22

*Step A.* [(6,7-Dichloro-2-butyl-1-oxo-2,3-dihydro-1H-inden-5-yl)oxy]acetic acid.

Example 23

*Step A.* [(2-Ethyl-6,7-dimethyl-1-oxo-2,3-dihydro-1H-inden-5-yl)oxy]acetic acid.
There is obtained:

Example 21

*Step A.* Methyl [(6,7-dichloro-2-propyl-1-oxo-2,3-dihydro-1H-inden-5-yl)oxy]acetate.

Example 22

*Step A.* Methyl [(6,7-dichloro-2-butyl-1-oxo-2,3-dihydro-1H-inden-5-yl)oxy]acetate.

Example 23

*Step A.* Methyl [(2-ethyl-6,7-dimethyl-1-oxo-2,3-dihydro-1H-inden-5-yl)oxy]acetate.

Carrying out the reaction as described in Example 20, Step B, except that the methyl [(6,7-dichloro-2-ethyl-1-oxo-2,3-dihydro-1H-inden-5-yl)oxy]acetate is substituted by an equimolar quantity of the product of Example 21, Step A, Example 22, Step A or Example 23, Step A.
There is obtained:

Example 21

*Step B.* Methyl {[6,7-dichloro-2-propyl-1-oxo-2-(3-oxobutyl)-2,3-dihydro-1H-inden-5-yl]oxy}acetate, m.p. 91—93°C.

Example 22

*Step B.* Methyl {[6,7,dichloro-2-butyl-1-oxo-2-(3-oxobutyl)-2,3-dihydro-1H-inden-5-yl]oxy}acetate, m.p. 92—93°C.

Example 23

*Step B.* Methyl {[2-ethyl-6,7-dimethyl-1-oxo-2-(3-oxobutyl)-2,3-dihydro-1H-inden-5-yl]oxy}acetate.

Carrying out the reaction as described in Example 20, Step C, except that the methyl {[(6,7-dichloro-2-ethyl-1-oxo-2-(3-oxobutyl)-2,3-dihydro-1H-inden-5-yl)oxy}acetate is substituted by an equimolar quantity of the product of Example 21, Step B, Example 22, Step B or Example 23, Step B, there is obtained:

Example 21

*Step C.* Methyl [(5,6-dichloro-9a-propyl-3-oxo-1,2,9,9a-tetrahydro-3H-fluoren-7-yl)oxy]acetate, m.p. 158—159°C.

Example 22

*Step C.* Methyl [(5,6-dichloro-9a-butyl-3-oxo-1,2,9,9a-tetrahydro-3H-fluoren-7-yl)oxy]acetate, 153.5—154°C.

Example 23

*Step C.* Methyl [(9a-ethyl-5,6-dimethyl-3-oxo-1,2,9,9a-tetrahydro-3H-fluoren-7-yl)oxy]acetate.

Carrying out the reaction as described in Example 20, Step D, except that the methyl [(5,6-dichloro-9a-ethyl-3-oxo-1,2,9,9a-tetrahydro-3H-fluoren-7-yl)oxy]acetate is substituted by an equimolar quantity of the product of Example 21, Step C, Example 22, Step C or Example 23, Step C. There is obtained:

Example 21

*Step D.* [(5,6-Dichloro-9a-propyl-3-oxo-1,2,9,9a-tetrahydro-3H-fluoren-7-yl)oxy]acetic acid.

Example 22

*Step D.* [(5,6-Dichloro-9a-butyl-3-oxo-1,2,9,9a-tetrahydro-3H-fluoren-7-yl)oxy]acetic acid.

Example 23

*Step D.* [(9a-Ethyl-5,6-dimethyl-3-oxo-1,2,9,9a-tetrahydro-3H-fluoren-7-yl)oxy]acetic acid.

Examples 24 and 25

Resolution of [(5,6-dichloro-9a-ethyl-3-oxo-1,2,9,9a-tetrahydro-3H-fluoren-7-yl)oxy]acetic acid

This resolution yielded the corresponding pure enantiomers, i.e. (+) enantiomer according to example 24 and the (—) enantiomer according to example 25.

*Step A.* A mixture of racemic [(5,6-dichloro-9a-ethyl-3-oxo-1,2,9,9a-tetrahydro-3H-fluoren-7-yl)oxy]-acetic acid (7.1 gm., 0.02 mole) and dextro(+)cinchonine (5.89 gm., 0.02 mole) is dissolved in the minimum volume of boiling acetonitrile, is cooled to 5°C and aged for 48 hours. The crystalline product is removed by filtration and recrystallized twice from the minimum volume of acetonitrile. (The mother liquors from each recrystallization are combined and saved).

The pure salt is suspended in water (40 ml.) treated with 6 normal hydrochloric acid (5 ml.) and the precipitate that forms is removed by filtration and dried. This pure (+) enantiomer of [(5,6-dichloro-9a-ethyl-3-oxo-1,2,9,9a-tetrahydro-3H-fluoren-7-yl)oxy]acetic acid is then recrystallized from a mixture of tetrahydrofuran and ethyl ether, m.p. 239—241°C; $\alpha_{23}^{589}$ (C=1.1) = +151.2 (solvent: chloroform).

*Step B.* The combined acetonitrile mother liquors from Step A are evaporated at reduced pressure and he residue treated with water (50 ml.) and 6 normal hydrochloric acid (7.5 ml.). The resulting precipitate is removed by filtration and dried.

This residual partially resolved [(5,6-dichloro-9a-ethyl-3-oxo-1,2,9,9a-tetrahydro-3H-fluoren-7-yl)oxy]acetic acid (2.86 gm., 0.0118 mole) is treated with 1(—)cinchonidine (3.47 gm., 0.0118 mole) and the mixture dissolved in the minimum quantity of acetonitrile. After cooling for 48 hours, the salt that separates is removed by filtration and recrystallized twice from the minimum volume of acetonitrile. The pure salt is suspended in water (50 ml), treated with 6 normal hydrochloric acid (5 ml) and the precipitate that separates is removed by filtration and dried. This solid is recrystallized from a mixture of tetrahydrofuran and ethyl ether to give the (—) enantiomer of [(5,6-dichloro-9a-ethyl-3-oxo-1,2,9,9a-tetrahydro-3H-fluoren-7-yl)oxy]acetic acid, m.p. 239—241°C, $\alpha_{23}^{589}$ (C=1.1) = +151.2 (solvent: chloroform).

Example 26

*Steps A and B.* The Two Enantiomers of [(9a-butyl-5,6-dichloro-3-oxo-1,2,9,9a-tetrahydro-3H-fluoren-7-yl)oxy]acetic acid

Carrying out the resolution as described in Examples 24 and 25 except that the racemic [(5,6-dichloro-9a-ethyl-3-oxo-1,2,9,9a-tetrahydro-3H-fluoren-7-yl)oxy]acetic acid (Example 1, Step E) is substituted by an equimolar quantity of racemic [(9a-butyl-5,6-dichloro-3-oxo-1,2,9,9a-tetrahydro-3H-fluoren-7-yl)oxy]acetic acid (Example 6, Step E), there is obtained the two enantiomers of [(9a-butyl-5,6-dichloro-3-oxo-1,2,9,9a-tetrahydro-3H-fluoren-7-yl)oxy]acetic acid.

Example 27

*Steps A and B.* The Two Enantiomers of [(9a-allyl-5,6-dichloro-3-oxo-1,2,9,9a-tetrahydro-3H-fluoren-7-yl)oxy]acetic acid

Carrying out the resolution as described in Examples 24 and 25, except that the racemic [(5,6-dichloro-9a-ethyl-3-oxo-1,2,9,9a-tetrahydro-3H-fluoren-7-yl)oxy]acetic acid is replaced by an equimolar quantity of recemic [(9a-allyl-5,6-dichloro-3-oxo-1,2,9,9a-tetrahydro-3H-fluoren-7-yl)oxy]-acetic acid (Example 31, Step L) there is obtained the two enantiomers of [(9a-allyl-5,6-dichloro-3-oxo-1,2,9,9a-tetrahydro-3H-fluoren-7-yl)oxy]acetic acid.

Example 28

*Steps A and B.* The Two Enantiomers of [(5,6-dichloro-3-oxo-9a-propargyl-1,2,9,9a-tetrahydro-3H-fluoren-7-yl)oxy]acetic acid

Carrying out the resolution as described in Examples 24 and 25, except that the racemic [(5,6-dichloro-9a-ethyl-3-oxo-1,2,9,9a-tetrahydro-3H-fluoren-7-yl)oxy]acetic acid is replaced by an equimolar quantity of racemic [(5,6-dichloro-3-oxo-9a-propargyl-1,2,9,9a-tetrahydro-3H-fluoren-7-yl)-oxy]acetic acid (Example 32, Step L), there is obtained the two enantiomers of [(5,6-dichloro-3-oxo-9a-propargyl-1,2,9,9a-tetrahydro-3H-fluoren-7-yl)oxy]acetic acid.

Example 29

1-[(5,6-Dichloro-9a-methyl-3-oxo-1,2,9,9a-tetrahydro-3H-fluoren-7-yl)oxy]cyclopentane-1-carboxylic acid

*Step A.* 5,6-Dichloro-7-hydroxy-9a-methyl-1,2,9,9a-tetrahydro-3H-fluoren-3-one.

Carrying out a reaction as described in Example 1, Step C, except that the 5,6-dichloro-9a-ethyl-7-methoxy-1,2,9,9a-tetrahydro-3H-fluoren-3-one is replaced by an equimolar quantity of [(5,6-dichloro-9a-methyl-3-oxo-1,2,9,9a-tetrahydro-3H-fluoren-7-yl)oxy]acetic acid (Example 3, Step E), there is obtained 5,6-dichloro-7-hydroxy-9a-methyl-1,2,9,9a-tetrahydro-3H-fluoren-3-one.

*Step B.* Ethyl 1-[(5,6-Dichloro-9a-methyl-3-oxo-1,2,9,9a-tetrahydro-3H-fluoren-7-yl)oxy]cyclopentane-1-carboxylate.

Carrying out the reaction as described in Example 1, Step D, except that the 5,6-dichloro-9a-ethyl-7-hydroxy-1,2,9,9a-tetrahydro-3H-fluoren-3-one and ethyl bromoacetate are replaced by equimolar quantities of 5,6-dichloro-7-hydroxy-9a-methyl-1,2,9,9a-tetrahydro-3H-fluoren-3-one and ethyl 1-bromocyclopentane-1-carboxylate, there is obtained eithyl 1-[(5,6-dichloro-9a-methyl-3-oxo-1,2,9,9a-tetrahydro-3H-fluoren-7-yl)oxy]cyclopentane-1-carboxylate.

*Step C.* 1-[(5,6-Dichloro-9a-methyl-3-oxo-1,2,9,9a-tetrahydro-3H-fluoren-7-yl)oxy]cyclopentane-1-carboxylic Acid.

Carrying out the reaction as described in Example 1, Step E, except that the ethyl [(5,6-dichloro-9a-ethyl-1,2,9,9a-tetrahydro-3H-fluoren-7-yl)oxy]acetate is replaced by an equimolar quantity of ethyl 1[(5,6-dichloro-9a-methyl-3-oxo-1,2,9,9a-tetrahydro-3H-fluoren-7-yl)oxy]cyclopentane-1-carboxylate, to give 1-[(5,6-dichloro-9a-methyl-3-oxo-1,2,9,9a-tetrahydro-3H-fluoren-7-yl)oxy]cyclopentane-1-carboxylic acid.

Example 30

Fluoro[(5,6-dichloro-9a-ethyl-3-oxo-1,2,9,9a-tetrahydro-3H-fluoren-7-yl)oxy]acetic Acid

*Step A.* 5,6-Dichloro-9a-ethyl-7-hydroxy-1,2,9,9a-tetrahydro-3H-fluoren-3-one.

Carrying out the reaction as described in Example 1, Step C, except that the 5,6-dichloro-9a-ethyl-7-methoxy-1,2,9,9a-tetrahydro-3H-fluoren-3-one is replaced by an equimolar quantity of [(5,6-dichloro-9a-ethyl-3-oxo-1,2,9,9a-tetrahydro-3H-fluoren-7-yl)oxy]acetic acid (Example 20, Step D), there is obtained 5,6-dichloro-9a-ethyl-7-hydroxy-1,2,9,9a-tetrahydro-3H-fluoren-3-one.

*Step B.* Ethyl fluoro[(5,6-dichloro-9a-ethyl-3-oxo-1,2,9,9a-tetrahydro-3H-fluoren-7-yl)oxy]acetate.

Carrying out the reaction as described in Example1, Step D, except that the ethyl bromoacetate is replaced by an equimolar quantity of ethyl bromofluoroacetate, there is obtained ethyl fluoro[(5,6-dichloro-9a-ethyl-3-oxo-1,2,9,9a-tetrahydro-3H-fluoren-7-yl)oxy]acetate.

*Step C.* Fluoro[(5,6-dichloro-9a-ethyl-3-oxo-1,2,9,9a-tetrahydro-3H-fluoren-7-yl)oxy]acetic Acid.

Ethyl fluoro[(5,6-dichloro-9a-ethyl-3-oxo-1,2,9,9a-tetrahydro-3H-fluoren-7-yl]acetate (4.01 gm., 0.02 mole) is dissolved in ethanol (75 ml.) and treated with a solution of sodium bicarbonate (3.36 gm., 0.04 mole) in water (150 ml.). The mixture is heated and stirred on a steam bath for 15 minutes and then concentrated *in vacuo* to a volume of 50 ml. The residual solution is diluted with water (50 ml.) and acidified to Congo red test paper with 6 normal hydrochloric acid. The precipitate that forms is removed by filtration, washed with water and dried. The product is sufficiently pure for user but it may be further purified by recrystallization from a mixture of acetic acid and water to produce pure fluoro [(5,6-dichloro-9a-ethyl-3-oxo-1,2,9,9a-tetrahydro-3H-fluoren-7-yl)oxy]acetic acid.

Example 31

[(9a-Allyl-5,6-dichloro-3-oxo-1,2,9,9a-tetrahydro-3H-fluoren-7-yl)oxy]acetic Acid.

*Step A.* 2,3-Dichloro-4-methoxybenzaldehyde.

A mixture of 2,3-dichloro-4-hydroxybenzaldehyde (19.1 gm., 0.1 mole) and potassium carbonate (34.4 gm., 0.25 mole) in dimethylformamide (50 ml.) is stirred and treated dropwise over 15 minutes with dimethyl sulfate (14.1 gm., 0.11 mole). The reaction is exothermic; after 45 minutes the mixture is poured with stirring into water (300 ml.). The solid product that separates is removed by filtration, washed with water and then washed with a little methanol. After drying the yield of 2,3-dichloro-4-methoxybenzaldehyde is 16 gm., m.p., 117°—118°C.

*Step B.* 2,3-Dichloro-4-methoxycinnamic Acid

A mixture if 2,3-dichloro-4-methoxybenzaldehyde (158 gm., 0.77 mole), malonic acid (146 gm., 1.4 mole), pyridine (450 ml.) and piperidine (15 ml.) is heated and stirred on a steam bath for 2 3/4 hours. The hot reaction mixture is poured, with stirring into a mixture of concentrated hydrochloric acid (770 ml.) and crushed ice (3.1 kg.).

The solid that separates is removed by filtration, then washed, first with water and then with a little methanol. After drying, the solid is dissolved in a solution containing sodium hydroxide (61.6 gm.) and water (7.7 liters). The insoluble material is removed by filtration and the filtrate acidified with concentrated hydrochloric acid.

The solid 2,3-dichloro-4-methoxycinnamic acid that separates is removed by filtration, washed with water and dried. The yield is 179 gm. (96%), m.p. 246°—249°C. This crude material is adequate for use in the next step.

*Step C.* 3-(2,3-Dichloro-4-methoxyphenyl)-propanoic Acid.

2,3-Dichloro-4-methoxycinnamic acid (200.8 g., 0.813 mole) is dissolved in tetrahydrofuran (1600 ml.) and 5% platinum on charcoal (16.3 gm.) is added. The mixture is divided into 8 separate units and each is placed in a Parr hydrogenation apparatus in an atmosphere of hydrogen at an initial pressure of 50 p.s.i. Upon shaking, the time required for the reduction of each batch is 35 to 60 minutes.

The combined reaction mixtures are filtered and the solvent removed by distillation at reduced pressure to give crude 3 - (2,3 - dichloro - 4 - methoxyphenyl)propanoic acid, 176.3 gm., m.p. 141°—143°C. The material is adequate for use in the next step.

*Step D.* 4,5-Dichloro-6-methoxy-2,3-dihydro-1H-inden-1-one.

3-(2,3-Dichloro-4-methoxyphenyl)propanoic acid (176.3 gm., 0.708 mole) is placed in benzene (500 ml.) and thionyl chloride (101.8 ml., 1.42 mole) is added. The mixture is refluxed for 1 1/2 hours and the volatile materials removed by distillation at reduced pressure. The residual oil is dissolved in benzene (50 ml.) and the solvent again removed at reduced pressure. This process is repeated and the residue is dissolved in dry methylene chloride (500 ml.).

The solution is placed in a flask protected from atmospheric moisture by a calcium chloride drying tube and cooled via an ice bath. Then aluminum chloride (94.4 gm., 0.708 mole) is added portionwise via a vessel attached to the reaction flask by Gooch rubber tubing. After the addition is complete, the cooling bath is removed and stirring at ambient temperature is continued for 3 hours.

The reaction mixture is poured, with stirring into ice water (1635 ml.) containing concentrated hydrochloric acid (218 ml.). The solid that separates is collected by filtration, washed with water and dried. The yield is 160 gm. This material is recrystallized (while treating with decolorizing charcoal) from acetonitrile to give 4,5-dichloro-6-methoxy-2,3-dihydro-1H-inden-1-one, 129.1 gm., m.p. 163°—165°.

*Step E.* 4,5-Dichloro-6-methoxy-2,3-dihydro-1H-indene.

An amalgam of zinc, prepared from zinc (65 gm.) and mercuric chloride (3.5 gm.), is stirred with a mixture water (20 ml.), ethanol (20 ml.) and concentrated hydrochloric acid (50 ml.). The mixture is heated to boiling and a slurry of 4,5-dichloro-6-methoxy-2,3-dihydro-1H-inden-1-one (23.1 gm., 0.1 mole) in a mixture of hot ethanol (150 ml.), water (20 ml.) and concentrated hydrochloric acid (50 ml.) is added over 15 minutes. Then the boiling mixture is treated with concentrated hydrochloric

acid (50 ml.) over an hour. Boiling and stirring is continued for 5 hours longer and then the stirring is terminated and the mixture is cooled. The liquid portion is separated by decantation and concentrated to half its volume *in vacuo.* The solid portion of the reaction mixture is extracted with ether (four 50 ml. portions). The combined ether extracts and liquid portion of the reaction mixture are united in a separatory funnel and water (250 ml.) is added. The mixture is thoroughly shaken and the ether layer separated, washed with water, then with brine and finally dried over anhydrous magnesium sulfate. Evaporation of the ether gives 15.2 g. of crude 4,5-dichloro-6-methoxy-2,3-dihydro-1H-indene, m.p. 78°—82°C. This material is adequate for use in the next step.

*Step F.* 6,7-Dichloro-5-methoxy-2,3-dihydro-1H-inden-1-one.

4,5-Dichloro-6-methoxy-2,3-dihydro-1H-indene (21 gm., 0.097 mole) is dissolved in acetic acid (280 ml.) and chromium trioxide (14 gm., 0.14 mole) in a mixture of water (15 ml.) and acetic acid (40 ml.) is added, dropwise with stirring over a period of one hour. The mixture is poured, with stirring into cold water (1200 ml.) and the solid that separates is removed by filtration, washed with water and dried. The product, which consists of a mixture of the desired material and the isomer described in Step D, is subjected to column chromatographic separation using 225 gm. of silica gel and chloroform is used for elution. Evaporation of the solvent gives 6,7-dichloro-5-methoxy-2,3-dihydro-1H-inden-1-one, 5.2 gm., m.p. 154°—156°C.

*Step G.* 2-Allyl-6,7-dichloro-5-methoxy-2,3-dihydro-1H-inden-1-one.

6.7-Dichloro-5-methoxy-2,3-dihydro-1H-inden-1-one (63.5 gm., 0.274 mole) is dissolved in a mixture of dry tert.-butyl alcohol (500 ml.) and dry benzene (1.5 liters). The solution is refluxed and potassium tert.butoxide (46 gm., 0.301 mole) in dry tert.-butyl alcohol (1 liter) is added as rapidly as possible. The solution is refluxed for another 1/2 hour, cooled to 20°C., stirred and treated with allyl bromide (36 gm., 0.301 mole). The mixture is stirred and refluxed for 4 hours, cooled and treated with water (250 ml.). The crude 2-allyl-6,7-dichloro-5-methoxy-2,3-dihydro-1H-inden-1-one is separated by filtration, washed with water, dried and recrystallized from a mixture of benzene and hexane.

*Step H.* 2-Allyl-6,7-dichloro-5-hydroxy-2,3-dihydro-1H-inden-1-one.

Dry pyridine hydrochloride (500 gm.) is melted and heated to 195°C. and 2-allyl-6,7-dichloro-5-methoxy-2,3-dihydro-1H-inden-1-one (45.4 gm., 0.167 mole) is added with stirring. The reaction mixture is kept at 195°C. for 45 minutes and then poured with vigorous stirring into crushed ice (2 kg.). The crude product is collected by filtration, washed with water and dried. The 2-allyl-6,7-dichloro-5-hydrox-2,3-dihydro-1H-inden-1-one is recrystallized from a mixture of ethanol and water.

*Step I.* Ethyl [(2-allyl-6,7-dichloro-1-oxo-2,3-dihydro-1H-inden-5-yl)oxy]acetate.

Carrying out a reaction as described in Example 1, Step D, except that the 5,6-dichloro-9a-ethyl-7-hydroxy-1,2,9,9a-tetrahydro-3H-fluoren-3-one is replaced by an equimolar quantity of 2-allyl-6,7-dichloro-5-hydroxy-2,3-dihydro-1H-inden-1-one, there is obtained ethyl [(2-allyl-6,7-dichloro-1-oxo-2,3-dihydro-1H-inden-5-yl)oxy]acetate.

*Step J.* Ethyl{[2-allyl-6,7-dichloro-1-oxo-2-(3-oxobutyl)-2,3-dihydro-1H-inden-5-yl]oxy}-acetate.

Carrying out a reaction as described in Example 20, Step B, except that the methyl [(6,7-dichloro-2-ethyl-1-oxo-2,3-dihydro-1H-inden-5-yl)-oxy]acetate is replaced by an equimolar quantity of ethyl [(2-allyl-6,7-dichloro-1-oxo-2,3-dihydro-1H-inden-5-yl)oxy]acetate, there is obtained ethyl {[2-allyl-6,7-dichloro-1-oxo-2-(3-oxobutyl)-2,3-dihydro-1H-inden-5-yl]oxy}acetate.

*Step K.* Ethyl [(9a-allyl-5,6-dichloro-3-oxo-1,2,9,9a-tetrahydro-3H-fluoren-7-yl)oxy]acetate.

Carrying out a reaction as described in Example 20, Step C, except that the methyl {[6,7-dichloro-2-ethyl-1-oxo-2-(3-oxobutyl)-2,3-dihydro-1H-inden-5-yl]oxy]acetate is replaced by an equimolar quantity of ethyl (2-allyl-6,7-dichloro-1-oxo-2-(3-oxobutyl)-2,3-dihydro-1H-inden-5-yl)oxy]acetate, there is obtained ethyl [(9a-allyl-5,6-dichloro-3-oxo-1,2,9,9a-tetrahydro-3H-fluoren-7-yl)oxy]acetate.

*Step L.* [(9a-Allyl-5,6-dichloro-3-oxo-1,2,9,9a-tetrahydro-3H-fluoren-7-yl)oxy]acetic Acid.

Carrying out a reaction as described in Example 20, Step D, except that the methyl [(5,6-dichloro-9a-ethyl-3-oxo-1,2,9,9a-tetrahydro-3H-fluoren-7-yl)oxy]acetate is replaced by an equimolar quantity of ethyl [(9a-allyl-5,6-dichloro-3-oxo-1,2,9,9a-tetrahydro-3H-fluoren-7-yl)oxy]acetate, there is obtained [(9a-allyl-5,6-dichloro-3-oxo-1,2,9,9a-tetrahydro-3H-fluoren-7-yl)oxy]acetic acid.

Example 32

[(5,6-Dichloro-3-oxo-9a-propargyl-1,2,9,9a-tetrahydro-3H-fluoren-7-yl)oxy]acetic Acid.

By carrying out a series of reactions as described in Example 31, except that in Step G, the allyl bromide is replaced by an equimolar quantity of propargyl bromide and using the product from each step in the subseqeunt step, there is obtained:

*Step G.* 6,7-Dichloro-5-methoxy-2-propargyl-2,3-dihydro-1H-iden-1-one.

*Step H.* 6,7-Dichloro-5-hydroxy-2-propargyl-2,3-dihydro-1H-inden-1-one.

*Step I.* Ethyl [6,7-dichloro-1-oxo-2-propargyl-2,3-dihydro-1H-inden-5-yl)oxy]acetate.

*Step J.* Ethyl {[6,7-dichloro-1-oxo-2-(3-oxobutyl)-2-propargyl-2,3-dihydro-1H-inden-5-yl]oxy}acetate.

*Step K.* Ethyl [5,6-dichloro-3-oxo-9a-propargyl-1,2,9,9a-tetrahydro-3H-fluoren-7-yl)oxy]acetate.

*Step L.* [(5,6-Dichloro-3-oxo-9a-propargyl-1,2,9,9a-tetrahydro-3H-fluoren-7-yl)oxy]acetic Acid.

Example 33
[(5,6-Dichloro-9a-(2-methylpropyl)-3-oxo-1,2,9,9a-tetrahydro-3H-fluoren-7-yl)oxy]acetic Acid.

Carrying out a series of reactions as described in Example 31, except that in Step G, the allyl bromide is replaced by an equimolar quantity of 2-methyl-propyl bromide, and using the product from each step in the subsequent one, there is obtained:

*Step G.* 6,7-Dichloro-2-(2-methylpropyl)-5-methoxy-2,3-dihydro-1H-inden-1-one.

*Step H.* 6,7-Dichloro-2-(2-methylpropyl)-5-hydroxy-2,3-dihydro-1H-inden-1-one.

*Step I.* Ethyl [6,7-dichloro-2-(2-methylpropyl)-1-oxo-2,3-dihydro-1H-inden-5-yl)oxy]acetate.

*Step J.* Ethyl{[6,7-dichloro-2-(2-methylpropyl)-1-oxo-2-(3-oxobutyl)-2,3-dihydro-1H-inden-5-yl]oxy}acetate.

*Step K.* Ethyl [(5,6-dichloro-9a-(2-methylpropyl)-3-oxo-1,2,9,9a-tetrahydro-3H-fluoren-7-yl)oxy]-acetate.

*Step L.* [(5,6-Dichloro-9a-(2-methylpropyl)-3-oxo-1,2,9,9a-tetrahydro-3H-fluoren-7-yl)oxy]acetic acid.

Example 34
[(5,6-Dichloro-9a-cyclopropylmethyl-3-oxo-1,2,9,9a-tetrahydro-3H-fluoren-7-yl)oxy]acetic Acid

Carrying out a series of reactions as described in Example 31, except that in Step G, the allyl bromide is replaced by an equimolar quantity of (bromomethyl)cyclopropane, and using the product from each step in the subsequent step, there is obtained:

*Step G.* 6,7-Dichloro-2-cyclopropylmethyl-5-methoxy-2,3-dihydro-1H-inden-1-one.

*Step H.* 6,7-Dichloro-2-chloropropylmethyl-5-hydroxy-2,3-dihydro-1H-inden-1-one.

*Step I.* Ethyl [(6,7-dichloro-2-cyclopropylmethyl-1-oxo-2,3-dihydro-1H-inden-5-yl)oxy]acetate.

*Step J.* Ethyl {[6,7-dichloro-2-cyclopropylmethyl-1-oxo-2-(3-oxobutyl)-2,3-dihydro-1H-inden-5-yl]oxy}acetate.

*Step K.* Ethyl (5,6-dichloro-9a-cyclopropylmethyl-3-oxo-1,2,9,9a-tetrahydro-3H-fluoren-7-yl)oxy]-acetate.

*Step L.* [(5,6-Dichloro-9a-cyclopropylmethyl-3-oxo-1,2,9,9a-tetrahydro-3H-fluoren-7-yl)oxy]acetic acid.

Example 35
[(5,6-Dichloro-9a-ethyl-3-oxo-1,2,9,9a-tetrahydro-3H-fluoren-7-yl)oxy]acetic Acid

5,6-Dichloro-9a-ethyl-7-hydroxy-1,2,9,9a-tetrahydro-3H-fluoren-3-one (Example 1, Step C) (15 gm., 0.048 mole), potassium carbonate (13.3 gm., 0.096 mole), dimethylformamide (100 ml.) and iodoacetic acid (10.6 gm., 0.057 mole) is stirred at room temperature for 24 hours. The reaction mixture is poured into water (150 ml.), stirred, warmed to 50°C., filtered and the filtrate acidified with 6N hydrochloric acid to obtain [(5,6-dichloro-9a-ethyl-3-oxo-1,2,9,9a-tetrahydro-3H-fluoren-7-yl)-oxy]acetic acid, which after recrystallization from acetic acid, melts at 237°C—238°C.

Example 36
[(5,6-Dichloro-9a-methyl-3-oxo-1,2,9,9a-tetrahydro-3H-fluoren-7-yl)oxy]acetic Acid

Carrying out a reaction as described in Example 35, except that the 5,6-dichloro-9a-ethyl-7-

hydroxy-1,2,9,9a-tetrahydro-3H-fluoren-3-one is replaced by an equivalent quantity of 5,6-dichloro-9a-methyl-7-hydroxy-1,2,9,9a-tetrahydro-3H-fluoren-3-one (Example 3,, Step C), and there is obtained [(5,6-dichloro-9a-methyl-3-oxo-1,2,9,9a-tetrahydro-3H-fluoren-7-yl)oxy]acetic acid.

Example 37

[(5,6-Dichloro-9a-ethyl-3-oxo-1,2,9,9a-tetrahydro-3H-fluoren-7-yl)oxy]acetic Acid

*Step A.* tert.-Butyl [(5,6-dichloro-9a-ethyl-3-oxo-1,2,9,9a-tetrahydro-3H-fluoren-7-yl)oxy]acetate.

A mixture of 5,6-dichloro-9a-ethyl-7-hydroxy-1,2,9,9a-tetrahydro-3H-fluoren-3-one (Example 1, Step C) (15 gm., 0.048 mole), tert.-butyl bromoacetate (10.3 gm., 0.0528 mole), potassium carbonate (13.3 gm., 0.096 mole) and dimethylformamide (100 ml.) is stirred at 35°C. for an hour and the cooled and poured into water (100 ml.). The solid that separtes is tert.-butyl [(5,6-dichloro-9a-ethyl-3-oxo-1,2,9,9a-tetrahydro-3H-fluoren-7-yl)oxy]acetate, which is separated by filtration, washed with water and dried. This material is adequate for use in the next step.

*Step B.* [(5,6-Dichloro-9a-ethyl-3-oxo-1,2,9,9a-tetrahydro-3H-fluoren-7-yl)oxy]acetic Acid.

Tert.-butyl [(5,6-dichloro-9a-ethyl-3-oxo-1,2,9,9a-tetrahydro-3H-fluoren-7-yl)oxy]acetate (8.22 gm., 0.02 mole) in benzene (100 ml.) containing p-toluenesulfonic acid (0.6 gm.) is refluxed for 20 minutes. The solid that separates upon cooling is [(5,6-dichloro-9a-ethyl-3-oxo-1,2,9,9a-tetrahydro-3H-fluoren-7-yl)oxy]acetic acid, m.p. 237°C—238°C.

Example 38

[(5,6-Dichloro-3-oxo-9a-propyl-1,2,9,9a-tetrahydro-3H-fluoren-7-yl)oxy]acetic Acid

*Step A* Tert.-butyl [(5,6-dichloro-3-oxo-9a-propyl-1,2,9,9a-tetrahydro-3H-fluoren-7-yl)oxy]acetate.

Carrying out a reaction as described in Example 37, Step A, except that the 5,6-dichloro-9a-ethyl-7-hydroxy-1,2,9,9a-tetrahydro-3H-fluoren-3-one is replaced by an equimolar quantity of 5,6-dichloro-7-hydroxy-9a-propyl-1,2,9,9a-tetrahydro-3H-fluoren-3-one (Example 4, Step C), thereby is obtained tert.-butyl [(5,6-dichloro-3-oxo-9a-propyl-1,2,9,9a-tetrahydro-3H-fluoren-7-yl)oxy]acetate.

*Step B.* [(5,6-Dichloro-3-oxo-9a-propyl-1,2,9,9a-tetrahydro-3H-fluoren-7-yl)oxy]acetic Acid.

Carrying out a reaction as described in Example 37, Step B, except that the tert.-butyl [(5,6-dichloro-9a-ethyl-3-oxo-1,2,9,9a-tetrahydro-3H-fluoren-7-yl)oxy]acetate is replaced by an equimolar amount of tert.-butyl [(5,6-dichloro-3-oxo-9a-propyl-1,2,9,9a-tetrahydro-3H-fluoren-7-yl)oxy]acetate, thereby is obtained [(5,6-dichloro-3-oxo-9a-propyl-1,2,9,9a-tetrahydro-3H-fluoren-7-yl)oxy]acetic acid.

Example 39

Ethyl [(5,6-Dichloro-3-oxo-9a-ethyl-1,2,9,9a-tetrahydro-3H-fluoren-7-yl)oxy]acetate

Methyl [(5,6-dichloro-9a-ethyl-3-oxo-1,2,9,9a-tetrahydro-3H-fluoren-7-yl)oxy]acetate (Example 20, Step C) (5.8 g, 0.016 mole) is dissolved in a mixture of dry ethanol (50 ml) and methylene chloride (40 ml) containing Amerblite® IRA 400 (ethoxide) (5—6 g). The mixture is stirred magnetically at reflux for four hours and then filtered. The filtrate is concentrated *in vacuo* to give a yellow solid (5.3 g) which upon trituration with ethanol and washing with ether yields the product, 4.88 g, m.p. 164—166°C.

Using a procedure similar to that described in Example 39, except that there is substituted for the methyl [(5,6-dichloro-9a-ethyl-3-oxo-1,2,9,9a-tetrahydro-3H-fluoren-7-yl)oxy]acetate an equimolar amount of benzyl alcohol there is obtained:

Example 40

Benzyl [(5,6-dichloro-3-oxo-9a-ethyl-1,2,9,9a-tetrahydro-3H-fluoren-7-yl)oxy]acetate.

Example 41

Ethyl [(5,6-dichloro-9a-ethyl-3-oxo-1,2,9,9a-tetrahydro-3H-fluoren-7-yl)oxy]acetate

A solution of [(5,6-dichloro-9a-ethyl-3-oxo-1,2,9,9a-tetrahydro-3H-fluoren-7-yl)oxy]acetic acid (Example 20, Step D) (1.5 g, 0.004 mole) absolute ethanol (60 ml) and 12 N HCl (5 ml) in ethanol (20 ml) is heated under reflux on the stream bath with stirring for an hour. The excess solvent is removed under reduced pressure. Ice and water are added to the oily residue and the product extracted into ether. After drying over $MgSO_4$, the solution is concentrated to yield 1.6 g of product, m.p. 160—162°C.

Recrystallization from tetrahydrofuran-ether-petroleum ether (1:1:2) gives 1.15 g of ethyl [(5,6-dichloro-9a-ethyl-3-oxo-1,2,9,9a-tetrahydro-3H-fluoren-7-yl)oxy]acetate, m.p. 164—166°C.

By carrying out the reaction as described in Example 41, except that the ethanol is replaced by an equal amount of:

Example 42

1-Propanol

Example 43
Isopropyl alcohol

Example 44
1-Butanol

Example 45
Cyclobutanol

Example 46
Allyl alcohol

Example 47
Propargyl alcohol

There is obtained:

Example 42
Propyl [(5,6-dichloro-9a-ethyl-3-oxo-1,2,9,9a-tetrahydro-3H-fluoren-7-yl)oxy]acetate.

Example 43
Isopropyl [(5,6-dichloro-9a-ethyl-3-oxo-1,2,9,9a-tetrahydro-3H-fluoren-7-yl)oxy]acetate.

Example 44
Butyl [(5,6-dichloro-9a-ethyl-3-oxo-1,2,9,9a-tetrahydro-3H-fluoren-7-yl)oxy]acetate.

Example 45
Cyclobutyl [(5,6-dichloro-9a-ethyl-3-oxo-1,2,9,9a-tetrahydro-3H-fluoren-7-yl)oxy]acetate.

Example 46
Allyl [(5,6-dichloro-9a-ethyl-3-oxo-1,2,9,9a-tetrahydro-3H-fluoren-7-yl)oxy]acetate.

Example 47
Propargyl [(5,6-dichloro-9a-ethyl-3-oxo-1,2,9,9a-tetrahydro-3H-fluoren-7-yl)oxy]acetate.

By carrying out the reaction as described in Example 41 except that the [(5,6-dichloro-9a-ethyl-3-oxo-1,2,9,9a-tetrahydro-3H-fluoren-7-yl)oxy]acetic acid is replaced by an equimolar amount of:

Example 48
(+) [(5,6-Dichloro-9a-ethyl-3-oxo-1,2,9,9a-tetrahydro-3H-fluoren-7-yl)oxy]acetic acid (Example 25).

Example 49
(—) [(5,6-Dichloro-9a-ethyl-3-oxo-1,2,9,9a-tetrahydro-3H-fluoren-7-yl)oxy]acetic acid (Example 25).

Example 50
(+) [(9a-Allyl-5,6-dichloro-3-oxo-1,2,9,9a-tetrahydro-3H-fluoren-7-yl)oxy]acetic acid (Example 27).

Example 51
(—) [(9a-Allyl-5,6-dichloro-3-oxo-1,2,9,9a-tetrahydro-3H-fluoren-7-yl)oxy]acetic acid (Example 27).

Example 52
(+) [(5,6-Dichloro-3-oxo-9a-propargyl-1,2,9,9a-tetrahydro-3H-fluoren-7-yl)oxy]acetic acid (Example 28).

Example 53
(—) [(5,6-Dichloro-3-oxo-9a-propargyl-1,2,9,9a-tetrahydro-3H-fluoren-7-yl)oxy]acetic acid (Example 28).

There is obtained:

Example 48
Ethyl (+) [(5,6-dichloro-9a-ethyl-3-oxo-1,2,9,9a-tetrahydro-3H-fluoren-7-yl)oxy]acetate, m.p. 94—97°C.

Example 49
Ethyl (—) [(5,6-dichloro-9a-ethyl-3-oxo-1,2,9,9a-tetrahydro-3H-fluoren-7-yl)oxy]acetate, m.p. 94—97°C.

Example 50
Ethyl (+) [(9a-allyl-5,6-dichloro-3-oxo-1,2,9,9a-tetrahydro-3H-fluoren-7-yl)oxy]acetate.

Example 51
Ethyl (—) [(9a-allyl-5,6-dichloro-3-oxo-1,2,9,9a-tetrahydro-3H-fluoren-7-yl)oxy]acetate.

Example 52

Ethyl (+) [(5,6-dichloro-3-oxo-9a-propargyl-1,2,9,9a-tetrahydro-3H-fluoren-7-yl)oxy]acetate.

Example 53

Ethyl (—) [(5,6-dichloro-3-oxo-9a-propargyl-1,2,9,9a-tetrahydro-3H-fluoren-7-yl)oxy]acetate.

Example 54

Carboxymethyl [(5,6-dichloro-9a-ethyl-3-oxo-1,2,9,9a-tetrahydro-3H-fluoren-7-yl)oxy]acetate

*Step A.* Benzyloxycarbonylmethyl [(5,6-dichloro-9a-ethyl-3-oxo-1,2,9,9a-tetrahydro-3H-fluoren-7-yl)-oxy]acetate

A mixture containing [(5,6-dichloro-9a-ethyl-3-oxo-1,2,9,9a-tetrahydro-3H-fluoren-7-yl)oxy]-acetic acid (Example 20, Step D) (1.07 g, 0.003 mole), potassium carbonate (0.41 g, 0.003 mole) and benzyl bromoacetate (0.69 g, 0.003 mole) in dimethylformamide (35 ml) is heated at 80°C for two hours. Then this mixture is filtered and the filtrate is concentrated *in vacuo.* The residue is dissolved in methylene chloride, washed with aqueous sodium bicarbonate and then water, dried over anhydrous magnesium sulfate and concentrated *in vacuo* to give a viscous oil which is triturated with ether to give 1.06 g. of product.

*Step B.* Carboxymethyl [(5,6-dichloro-9a-ethyl-3-oxo-1,2,9,9a-tetrahydro-3H-fluoren-7-yl)oxy]acetate

Benzyloxycarbonylmethyl [(5,6-dichloro-9a-ethyl-3-oxo-1,2,9,9a-tetrahydro-3H-fluoren-7-yl)-oxy]acetate (1.0 g, 0.002 mole) (Step A) is dissolved in tetrahydrofuran (35 ml) and hydrogenated at ambient temperatures and atmospheric pressure employing 10% Pd/C (0.05 g.) as the catalyst in 2 hours. The catalyst is filtered through super-cel in an atmosphere of nitrogen. Then the filtrate is concentrated *in vacuo* at 50°C to give the product (0.74 g) which is purified by recrystallization from methanol (9 ml) to yield 0.50 g of product, m.p. 179—181°C.

By carrying out the reaction as described in Example 54, Step A, except that the benzyl bromoacetate is replaced by an equimolar quantity of:

Example 55

*Step A.* Benzyl 5-bromopentanoate

Example 56

*Step A.* Benzyl 2-bromopropanoate

Example 57

*Step A.* Benzyl 4-bromobutanoate

Example 58

*Step A.* Benzyl 2-bromo-2-methylpropanoate

There is obtained:

Example 55

*Step A.* 4-(Benzyloxycarbonyl)butyl [(5,6-dichloro-9a-ethyl-3-oxo-1,2,9,9a-tetrahydro-3H-fluoren-7-yl)oxy]acetate.

Example 56

*Step A.* 1-(Benzyloxycarbonyl)ethyl [(5,6-dichloro-9a-ethyl-3-oxo-1,2,9,9a-tetrahydro-3H-fluoren-7-yl)oxy]acetate.

Example 57

*Step A.* 3-(Benzyloxycarbonyl)propyl [(5,6-dichloro-9a-ethyl-3-oxo-1,2,9,9a-tetrahydro-3H-fluoren-7-yl)oxy]acetate.

Example 58

*Step A.* 1-Benzyloxycarbonyl-1-methylethyl [(5,6-dichloro-9a-ethyl-3-oxo-1,2,9,9a-tetrahydro-3H-fluoren-7-yl)oxy]acetate.

By carrying out the reaction as described in Example 54, Step B, except that the benzyloxycarbonylmethyl [(5,6-dichloro-9a-ethyl-3-oxo-1,2,9,9a-tetrahydro-3H-fluoren-7-yl)oxy]-acetate is replaced separately by the compounds produced in Example 55, Step A, Example 56, Step A, Example 57, Step A and in Example 58, Step A, there is produced:

Example 55

*Step B.* 4-Carboxybutyl [(5,6-dichloro-9a-ethyl-3-oxo-1,2,9,9a-tetrahydro-3H-fluoren-7-yl)oxy]-acetate.

Example 56

*Step B.* 1-Carboxyethyl [(5,6-dichloro-9a-ethyl-3-oxo-1,2,9,9a-tetrahydro-3H-fluoren-7-yl)oxy]-acetate.

Example 57

*Step B.* 3-Carboxypropyl [(5,6-dichloro-9a-ethyl-3-oxo-1,2,9,9a-tetrahydro-3H-fluoren-7-yl)oxy]-acetate.

Example 58

*Step B.* 1-Carboxy-1-methylethyl [(5,6-dichloro-9a-ethyl-3-oxo-1,2,9,9a-tetrahydro-3H-fluoren-7-yl)oxy]acetate.

By carrying out the reaction as described in Example 54, Step A, except that the [(5,6-dichloro-9a-ethyl-3-oxo-1,2,9,9a-tetrahydro-3H-fluoren-7-yl)oxy]acetic acid is replaced by an equimolar quantity of:

Example 59

*Step A.* [(9a-Allyl-5,6-dichloro-3-oxo-1,2,9,9a-tetrahydro-3H-fluoren-7-yl)oxy]acetic acid (Example 31, Step L).

Example 60

*Step A.* [(5,6-Dichloro-3-oxo-9a-propargyl-1,2,9,9a-tetrahydro-3H-fluoren-7-yl)oxy]acetic acid (Example 32, Step L).

Example 61

*Step A.* [(9a-Butyl-5,6-dichloro-3-oxo-1,2,9,9a-tetrahydro-3H-fluoren-7-yl)oxy]acetic acid (Example 6, Step E).

Example 62

*Step A.* [(9a-Benzyl-5,6-dichloro-3-oxo-1,2,9,9a-tetrahydro-3H-fluoren-7-yl)oxy]acetic acid (Example 9, Step D).

Example 63

*Step A.* (+) [(5,6-Dichloro-9a-ethyl-3-oxo-1,2,9,9a-tetrahydro-3H-fluoren-7-yl)oxy]acetic acid (Example 25).

Example 64

*Step A.* (—) [(5,6-Dichloro-9a-ethyl-3-oxo-1,2,9,9a-tetrahydro-3H-fluoren-7-yl)oxy]acetic acid (Example 25).

Example 65

*Step A.* (+) [(9a-Allyl-5,6-dichloro-3-oxo-1,2,9,9a-tetrahydro-3H-fluoren-7-yl)oxy]acetic acid (Example 27).

Example 66

*Step A.* (—) [(9a-Allyl-5,6-dichloro-3-oxo-1,2,9,9a-tetrahydro-3H-fluoren-7-yl)oxy]acetic acid (Example 27).

Example 67

*Step A.* (+) [(9a-Butyl-5,6-dichloro-3-oxo-1,2,9,9a-tetrahydro-3H-fluoren-7-yl)oxy]acetic acid (Example 26).

Example 68

*Step A.* (—) [(9a-Butyl-5,6-dichloro-3-oxo-1,2,9,9a-tetrahydro-3H-fluoren-7-yl)oxy]acetic acid (Example 26).

There is obtained:

Example 59

*Step A.* Benzyloxycarbonylmethyl [(9a-Allyl-5,6-dichloro-3-oxo-1,2,9,9a-tetrahydro-3H-fluoren-7-yl)-oxy]acetate.

Example 60

*Step A.* Benzyloxycarbonylmethyl [(5,6-dichloro-3-oxo-9a-propargyl-1,2,9,9a-tetrahydro-3H-fluoren-7-yl)oxy]acetate.

Example 61

*Step A.* Benzyloxycarbonylmethyl [(9a-butyl-5,6-dichloro-3-oxo-1,2,9,9a-tetrahydro-3H-fluoren-7-yl)-oxy]acetate.

Example 62

*Step A.* Benzyloxycarbonylmethyl [(9a-benzyl-5,6-dichloro-3-oxo-1,2,9,9a-tetrahydro-3H-fluoren-7-yl)oxy]acetate.

Example 63

*Step A.* Benzyloxycarbonylmethyl (+) [(5,6-dichloro-9a-ethyl-3-oxo-1,2,9,9a-tetrahydro-3H-fluoren-7-yl)oxy]acetate.

Example 64

*Step A.* Benzyloxycarbonylmethyl (—) [(5,6-dichloro-9a-ethyl-3-oxo-1,2,9,9a-tetrahydro-3H-fluoren-7-yl)oxy]acetate.

Example 65

*Step A.* Benzyloxycarbonylmethyl (+) [(9a-allyl-5,6-dichloro-3-oxo-1,2,9,9a-tetrahydro-3H-fluoren-7-yl)oxy]acetate.

Example 66

*Step A.* Benzyloxycarbonylmethyl (—) [(9a-allyl-5,6-dichloro-3-oxo-1,2,9,9a-tetrahydro-3H-fluoren-7-yl)oxy]acetate.

Example 67

*Step A.* Benzyloxycarbonylmethyl (+) [(9a-butyl-5,6-dichloro-3-oxo-1,2,9,9a-tetrahydro-3H-fluoren-7-yl)oxy]acetate.

Example 68

*Step A.* Benzyloxycarbonylmethyl (—) [(9a-butyl-5,6-dichloro-3-oxo-1,2,9,9a-tetrahydro-3H-fluoren-7-yl)oxy]acetate.

By carrying out the reaction as described in Example 54, Step B, except that the benzyloxycarbonylmethyl [(5,6-dichloro-9a-ethyl-3-oxo-1,2,9,9a-tetrahydro-3H-fluoren-7-yl)oxy]acetate is replaced by the products of Example 59, Step A, Example 60, Step A, Example 61, Step A, Example 62, Step A, Example 63, Step A, Example 64, Step A, Example 65, Step A, Example 66, Step A, Example 67, Step A and Example 68, Step A, respectively, there is obtained:

Example 59

*Step B.* Carboxymethyl [(9a-allyl-5,6-dichloro-3-oxo-1,2,9,9a-tetrahydro-3H-fluoren-7-yl)oxy]acetate.

Example 60

*Step B.* Carboxymethyl [(5,6-dichloro-3-oxo-9-propargyl-1,2,9,9a-tetrahydro-3H-fluoren-7-yl)oxy]acetate.

Example 61

*Step B.* Carboxymethyl [(9a-butyl-5,6-dichloro-3-oxo-1,2,9,9a-tetrahydro-3H-fluoren-7-yl)oxy]acetate.

Example 62

*Step B.* Carboxymethyl [(9a-benzyl-5,6-dichloro-3-oxo-1,2,9,9a-tetrahydro-3H-fluoren-7-yl)oxy]acetate.

Example 63

*Step B.* Carboxymethyl (+)[(5,6-dichloro-3-9a-ethyl-3-oxo-1,2,9,9a-tetrahydro-3H-fluoren-7-yl)oxy]acetate.

Example 64

*Step B.* Carboxymethyl (—)[(5,6-dichloro-9a-ethyl-3-oxo-1,2,9,9a-tetrahydro-3H-fluoren-7-yl)oxy]acetate.

Example 65

*Step B.* Carboxymethyl (+) [(9a-allyl-5,6-dichloro-3-oxo-1,2,9,9a-tetrahydro-3H-fluoren-7-yl)oxy]-acetate.

Example 66

*Step B.* Carboxymethyl (—)[(9a-allyl-5,6-dichloro-3-oxo-1,2,9,9a-tetrahydro-3H-fluoren-7-yl)oxy]acetate.

Example 67

*Step B.* Carboxymethyl (+)[(9a-butyl-5,6-dichloro-3-oxo-1,2,9,9a-tetrahydro-3H-fluoren-7-yl)oxy]acetate.

Example 68

*Step B.* Carboxymethyl (—)[(9a-butyl-5,6-dichloro-3-oxo-1,2,9,9a-tetrahydro-3H-fluoren-7-yl)oxy]acetate.

Example 69

2-(4-Morpholinyl)ethyl [(5,6-dichloro-9a-ethyl-3-oxo-1,2,9,9a-tetrahydro-3H-fluoren-7-yl)oxy]acetate.

*Step A.* 1-{[(5,6-dichloro-9a-ethyl-3-oxo-1,2,9,9a-tetrahydro-3H-fluoren-7-yl)oxy]acetyl}imidazole.

[(5,6-Dichloro-9a-ethyl-3-oxo-1,2,9,9a-tetrahydro-3H-fluoren-7-yl)oxy]acetic acid (Example 20, Step D) (0.71 g., 0.002 mole) is suspended in dry tetrahydrofuran (20 ml) and cooled to 0°C. A solution of 1,1′-carbonyldiimidazole (0.32 g, 0.002 mole) in tetrahydrofuran (5 ml) is added. The solution of 1-{[(5,6-dichloro-9a-ethyl-3-oxo-1,2,9,9a-tetrahydro-3H-fluoren-7-yl)oxy]acetyl}imidazole that is formed is used in the next step without isolation.

*Step B.* 2-(4-Morpholinyl)ethyl [(5,6-dichloro-9a-ethyl-3-oxo-1,2,9,9a-tetrahydro-3H-fluoren-7-yl)oxy]acetate.

To the solution of 1-{[(5,6-dichloro-9-ethyl-3-oxo-1,2,9,9a-tetrahydro-3H-fluoren-7-yl)oxy]-acetyl}imidazole (0.002 mole) prepared in Step A at 0°C is added with stirring 4-(2-hydroxyethyl)morpholine (0.26 g., 0.002 mole) and a catalytic amount of sodium hydride (10 mg). After stirring overnight at the ambient temperature, the colorless reaction mixture is concentrated *in vacuo* at 50°C.

The resultant yellow liquid is chromatographed using a silica-gel (60 gm) column and eluted with a mixture of methylene chloride and isopropyl alcohol (100/5 v.v). The product is triturated with ether to give analytically pure product, 0.58 g, m.p. 133—133.5°C.

By carrying out the reaction as described in Example 69, Step B, except that the 4-(2-hydroxyethyl)morpholine is replaced by an equimolar amount of:

Example 70: 2-(Dimethylamino)-ethanol
Example 71: 3-(Diethylamino)-propanol
Example 72: 1-(2-Hydroxyethyl)pyrrolidine
Example 73: 2-hydroxyisobutyric acid
Example 74: 1-methyl-3-hydroxypiperidine
Example 75: 1-methyl-4-hydroxypiperidine

there is obtained:

Example 70

2-(Dimethylamino)-ethyl [(5,6-dichloro-9a-ethyl-3-oxo-1,2,9,9a-tetrahydro-3H-fluoren-7-yl)oxy]acetate.

Example 71

3-(Diethylamino)-propyl [(5,6-dichloro-9a-ethyl-3-oxo-1,2,9,9a-tetrahydro-3H-fluoren-7-yl)oxy]acetate.

Example 72

2-(1-pyrrolidinyl)ethyl [(5,6-dichloro-9a-ethyl-3-oxo-1,2,9,9a-tetrahydro-3H-fluoren-7-yl)oxy]acetate.

Example 73

1-Carboxy-1-methylethyl [(5,6-dichloro-9a-ethyl-3-oxo-1,2,9,9a-tetrahydro-3H-fluoren-7-yl)oxy]acetate.

Example 74

1-Methyl-3-piperidyl [(5,6-dichloro-9a-ethyl-3-oxo-1,2,9,9a-tetrahydro-3H-fluoren-7-yl)oxy]acetate.

Example 75

1-Methyl-4-piperidyl [(5,6-dichloro-9a-ethyl-3-oxo-1,2,9,9a-tetrahydro-3H-fluoren-7-yl)oxy]acetate.

Example 76

(5-Hydroxymethyl-2-furyl)methyl [(5,6-dichloro-3-oxo-9a-ethyl-1,2,9,9a-tetrahydro-3H-fluoren-7-yl)oxy]acetate

[(5,6-dichloro-3-oxo-9a-ethyl-1,2,9,9a-tetrahydro-3H-fluoren-7-yl)oxy]acetic acid (Example 20, Step D) (1.43 g, 0.004 mole) is suspended in dry tetrahydrofuran (10 ml) and then triethylamine (0.401 g., 0.004 mole) is added. The resulting solution is cooled at 0°C followed by the addition of ethyl chloroformate (0.438 g, 0.004 mole) in tetrahydrofuran (5 ml) to form the mixed anhydride and triethylamine hydrochloride which precipitates. To this mixture is added 2,5-di(hydroxymethyl)furan (1.03 g, 0.008 mole) in tetrahydrofuran (5 ml). The reaction mixture is then allowed to rise to ambient temperature before refluxing for one hour. The triethylamine hydrochloride that separates (0.55 g) is filtered and the filtrate is concentrated *in vacuo* to give a brown solid (2.71 g) which is purified by column chromatography over silica gel (100 g) by elution with a mixture of methylene chloride-tetrahydrofuran (100/4 v.v) followed by a mixture of methylene-chloride-isopropanol (100/5 v.v) to give 0.55 g. of product which is recrystallized first from isopropanol and then from acetonitrile to give an impure material which melts at 135.5—137°C. This material is further purified by high pressure liquid chromatography using a Whatman Partisil M9 10/25 PAC column and methylene chloride-isopropanol alcohol (100/2 v.v) at a flow rate of 5 ml./min. The yield of analytically pure product is 280 mg., m.p. 142—143°C.

By carrying out the reaction as described in Example 76, except that the 2,5-di(hydroxymethyl)furan is replaced by an equimolar amount of:

Example 77: Dihydroxyacetone
Example 78: 2-Methoxyethanol
Example 79: Tetrahydrofurfuryl alcohol
Example 80: 1,1,1-Tris(hydroxymethyl)ethane

there is obtained:

Example 77

3-Hydroxy-2-oxopropyl [(5,6-dichloro-9a-ethyl-3-oxo-1,2,9,9a-tetrahydro-3H-fluoren-7-yl)oxy]acetate.

Example 78

2-Methoxyethyl [(5,6-dichloro-9a-ethyl-3-oxo-1,2,9,9a-tetrahydro-3H-fluoren-7-yl)oxy]acetate.

Example 79

Tetrahydrofurfuryl [(5,6-dichloro-9a-ethyl-3-oxo-1,2,9,9a-tetrahydro-3H-fluoren-7-yl)oxy]acetate.

Example 80

2,2-Bis(hydroxymethyl)propyl [(5,6-dichloro-9a-ethyl-3-oxo-1,2,9,9a-tetrahydro-3H-fluoren-7-yl)oxy]acetate.

Example 81

2-Oxopropyl [(5,6-dichloro-9a-ethyl-3-oxo-1,2,9,9a-tetrahydro-3H-fluoren-7-yl)oxy]acetate

Potassium carbonate (0.2 g) is suspended in a solution of [(5,6-dichloro-9a-ethyl-3-oxo-1,2,9,9a-tetrahydro-3H-fluoren-7-yl)oxy]acetic acid (Example 20, Step D) (0.4 g, 0.0011 mole) in dimethylformamide (3 ml). After warming the suspension on a steam bath for 15 minutes, chloroacetone (0.5 g, .005 mole) is added and the mixture heated for an additional 15 minutes. The reaction mixture is cooled, poured into water (50 ml) and the product extracted into ether (three 15 ml portions). The extract is dried over $Na_2SO_4$. After filtration, the extract is concentrated to give a yellow oil. Trituration with cold ether causes crystallization to occur. The crystalline product weighs 0.1 g and melts at 153—155°C.

Example 82

3-Hydroxypropyl [(5,6-dichloro-9a-ethyl-3-oxo-1,2,9,9a-tetrahydro-3H-fluoren-7-yl)oxy]acetate

By substitution of an equimolar amount of 3-bromo-1-propanol for the chloroacetone in Example 81, there is obtained a crude product which is column chromatographed on silica gel and eluted with a mixture of acetic acid, acetone and toluene (5/5/90 v.v.v). The product weighs 100 mg and melts at 114—117°C.

By carrying out the reaction as described in Example 81, except that the chloroacetone is replaced by an equimolar amount of:

Example 83: 2-Bromoethanol
Example 84: 2-Bromo-1,3-propanediol
Example 85: 2-Bromoethanesulfonamide
Example 86: 1-Bromo-2,3-propanediol

there is obtained:

Example 83

2-Hydroxyethyl [(5,6-dichloro-9a-ethyl-3-oxo-1,2,9,9a-tetrahydro-3H-fluoren-7-yl)oxy]acetate.

Example 84

1-(Hydroxymethyl)-2-hydroxyethyl [(5,6-dichloro-9a-ethyl-3-oxo-1,2,9,9a-tetrahydro-3H-fluoren-7-yl)oxy]acetate.

Example 85

2-Sulfamoylethyl [(5,6-dichloro-9a-ethyl-3-oxo-1,2,9,9a-tetrahydro-3H-fluoren-7-yl)oxy]acetate.

Example 86

2,3-Dihydroxypropyl [(5,6-dichloro-9a-ethyl-3-oxo-1,2,9,9a-tetrahydro-3H-fluoren-7-yl)oxy]acetate.

Example 87

3-Pyridylmethyl [(5,6-Dichloro-9a-ethyl-3-oxo-1,2,9,9a-tetrahydro-3H-fluoren-7-yl)oxy]acetate

A mixture of methyl [(5,6-dichloro-9a-ethyl-3-oxo-1,2,9,9a-tetrahydro-3H-fluoren-7-yl)oxy]-acetate (Example 20, Step C) (0.74 g, 0.002 mole), 3A molecular sieves (10 g) and 3-hydroxymethylpyridine (0.65 g, 0.006 mole) in methylene chloride (40 ml) is stirred at ambient temperature overnight.

The reaction mixture is then filtered through super-cel and washed with methylene chloride. The filtrate is concentrated *in vacuo* at 50°C. This residue is chromatographed over silica gel (30 g) by elution with a methylene chloride-isopropanol mixture (100/3 v.v.) to give 0.36 g of product which is recrystallized from isopropanol (7 ml) to yield 0.23 g of product containing isopropanol. This sample is dried at 83°C for 13 hours *in vacuo* to remove the solvent. The yield of product is 0.20 g., m.p. 120—121°C.

By carrying out the reaction as described in Example 87 except that the 3-hydroxymethylpyridine is replaced by an equimolar quantity of:

Example 88: 2-(hydroxymethyl)-pyridine
Example 89: 4-(hydroxymethyl)-pyridine
Example 90: 3-(hydroxymethyl)-pyridine-N-oxide

there is obtained:

Example 88

2-Pyridylmethyl [(5,6-dichloro-9a-ethyl-3-oxo-1,2,9,9a-tetrahydro-3H-fluoren-7-yl)oxy]acetate.

Example 89

4-Pyridylmethyl [(5,6-dichloro-9a-ethyl-3-oxo-1,2,9,9a-tetrahydro-3H-fluoren-7-yl)oxy]acetate.

Example 90

3-Pyridylmethyl [(5,6-dichloro-9a-ethyl-3-oxo-1,2,9,9a-tetrahydro-3H-fluoren-7-yl)oxy]acetate-N-oxide.

Example 91

[(5,6-dichloro-9a-ethyl-3-oxo-1,2,9,9a-tetrahydro-3H-fluoren-7-yl)oxy]acetic Acid Anhydride

A stirred suspension of 5,6-dichloro-9a-ethyl-3-oxo-1,2,9,9a-tetrahydro-3H-fluoren-7-yl)oxy]-acetoxy]acetic acid (14.2 gm., 0.04 mole) in methylene chloride (1.5 liters) is treated with a solution of N,N-dicyclohexylcarbodiimide (4.33 gm., 0.021 mole) in methylene chloride (100 ml.). After an hour, the solvent is removed by distillation and the residue treated with dry ether (150 ml.). The dicyclohexylurea is removed by filtration and the ether removed by evaporation at reduced pressure to give [(5,6-dichloro-9a-ethyl-3-oxo-1,2,9,9a-tetrahydro-3H-fluoren-7-yl)oxy]acetic acid anhydride.

Example 92

[(5,6-Dichloro-9a-ethyl-3-oxo-1,2,9,9a-tetrahydro-3H-fluoren-7-yl)oxy]acetamide

1-{[(5,6-Dichloro-9a-ethyl-3-oxo-1,2,9,9a-tetrahydro-3H-fluoren-7-yl)oxy]acetyl}imidazole (Example 69, Step A) (0.81 gm, 0.002 mole) in tetrahydrofuran (20 ml) is treated with 25% aqueous ammonium hydroxide (5 ml). After warming for an hour at 35°C, the solvent is removed *in vacuo* whereby the product remains which is washed with water and recrystallized from acetic acid.

By carrying out the reaction as described in Example 92, except that the 25% aqueous ammonium hydroxide is replaced by:

Example 93A: (t-Butoxycarbonyl)methylamine
Example 94A: 2-(t-Butyoxycarbonyl)ethylamine

there is obtained:

Example 93

*Step A.* N-(t-butoxycarbonylmethyl)-[(5,6-dichloro-9a-ethyl-3-oxo-1,2,9,9a-tetrahydro-3H-fluoren-7-yl)oxy]acetamide

Example 94

*Step A.* N-[2-(t-Butoxycarbonyl)ethyl]-[(5,6-dichloro-9a-ethyl-3-oxo-1,2,9,9a-tetrahydro-3H-fluoren-7-yl)oxy]acetamide.

Example 93

*Step B.* N-Carboxymethyl [(5,6-dichloro-9a-ethyl-3-oxo-1,2,9,9a-tetrahydro-3H-fluoren-7-yl)oxy]acetamide. N-(t-Butoxycarbonylmethyl)-[(5,6-dichloro-9a-ethyl-3-oxo-1,2,9,9a-tetrahydro-3H-fluoren-7-yl)oxy]acetamide (Example 93, Step A) (600 mg) is dissolved in toluene (100 ml), treated with p-toluenesulfonic acid (50 mg) and the mixture refluxed for 2 hours. The solvent is removed and the product dissolved in an aqueous sodium bicarbonate solution, filtered and acidified to Congo-red paper with dilute hydrochloric acid. The solid that separates is removed by filtration, washed with water and dried. The yield is 300 mg.

Example 94

*Step B.* N-(2-Carboxyethyl)-[(5,6-dichloro-9a-ethyl-3-oxo-1,2,9,9a-tetrahydro-3H-fluoren-7-yl)oxy]acetamide

By carrying out the reaction as described in Example 93, Step B, except that the N-(t-butoxycarbonylmethyl) - [(5,6 - dichloro - 9a - ethyl - 3 - oxo - 1,2,9,9a - tetrahydro - 3H - fluoren 7-yl)oxy]acetamide is replaced by an equimolar amount of N-[2-(t-butoxycarbonyl)ethyl]-[(5,6-dichloro-9a-ethyl-3-oxo-1,2,9,9a-tetrahydro-3H-fluoren-7-yl)oxy]acetamide. There is obtained N-(2-carboxyethyl)-[(5,6-dichloro-9a-ethyl-3-oxo-1,2,9,9a-tetrahydro-3H-fluoren-7-yl)oxy]acetamide.

Example 95

2,3-Dihydroxypropyl [(5,6-Dichloro-9a-ethyl-3-oxo-1,2,9,9a-tetrahydro-3H-fluoren-7-yl)oxy]acetate

*Step A.* (2,2-Dimethyl-1,3-dioxolan-4-yl)methyl [(5,6-dichloro-9a-ethyl-3-oxo-1,2,9,9a-tetrahydro-3H-fluoren-7-yl)oxy]acetate

A mixture of 2 gm, of [(5,6-dichloro-9a-ethyl-3-oxo-1,2,9,9a-tetrahydro-3H-fluoren-7-yl)oxy]acetic acid, 3 ml. of 2,2-dimethyl-4-hydroxymethyl-1,3-dioxolane and 0.5 g. of p-toluenesulfonic acid hydrate are heated on the steam bath for 3 hours. The dark reaction mixture then is chromatographed on 300 g of silica, and eluted with acetic acid-acetone-toluene (5:5:90). Fractions containing a single component (Rf~.4) are pooled and concentrated to a yellow oil. On standing overnight, a mixture of crystalline solid and yellow oil resulted. The mixture is filtered and the solid recrystallized from tetrahydrofuran-ether-petroleum ether (5:15:25). The yield of pure product is 0.3 g., m.p. 143—145°C.

*Step B.* 2,3-Dihydroxypropyl [5,6-dichloro-9a-ethyl-3-oxo-1,2,9,9a-tetrahydro-3H-fluoren-7-yl)oxy]-acetate

A mixture of 165 mg of (2,2-dimethyl-1,3-dioxolan-4-yl)methyl [(5,6-dichloro-9a-ethyl-3-oxo-1,2,9,9a-tetrahydro-3H-fluoren-7-yl)oxy]acetate from Example 95, Step A is dissolved in 100 ml. of

0.075 N hydrochloric acid and 20 ml. of acetone and heated, with stirring at 50—55°C for 2 hours. The mixture is cooled and neutralized with a solution of sodium bicarbonate. The solution is saturated with sodium chloride and extracted two times with 100 ml. portions of 20% tetrahydrofuran in ether. The extract is dried over $Na_2SO_4$. After filtration, the filtrate was concentrated to a yellow oil that solidified on standing. After recrystallization from tetrahydrofuran-ether-petroleum ether, there is obtained 110 mg. of product, m.p. 135—138°C.

Example 96

1-(Hydroxymethyl)-2-hydroxyethyl [(5,6-Dichloro-9a-ethyl-3-oxo-1,2,9,9a-tetrahydro-3H-fluoren-7-yl)oxy]acetate

*Step A.* (2-Phenyl-1,3-dioxan-5-yl) [(5,6-dichloro-9a-ethyl-3-oxo-1,2,9,9a-tetrahydro-3H-fluoren-7-yl)oxy]acetate

By substituting the 2,2-dimethyl-4-hydroxymethyl-1,3-dioxolane used in Example 95, Step A, with an equimolar quantity of 5-hydroxy-2-phenyl-1,3-dioxane and conducting the reaction as described in Example 95, Step A, there is obtained (2-phenyl-1,3-dioxan-5-yl) [(5,6-dichloro-9a-ethyl-3-oxo-1,2,9,9a-tetrahydro-3H-fluoren-7-yl)oxy]acetate.

*Step B.* 1-(Hydroxymethyl)-2-hydroxyethyl [(5,6-dichloro-9a-ethyl-3-oxo-1,2,9,9a-tetrahydro-3H-fluoren-7-yl)oxy]acetate

By substituting the (2,2-dimethyl-1,3-dioxolan-4-yl)methyl [(5,6-dichloro-9a-ethyl-3-oxo-1,2,9,9a-tetrahydro-3H-fluoren-7-yl)oxy]acetate used in Example 95, Step B by an equimolar amount of (1-phenyl-1,3-dioxan-5-yl [(5,6-dichloro-9a-ethyl-3-oxo-1,2,9,9a-tetrahydro-3H-fluoren-7-yl)oxy]-acetate and conducting the reaction as described in Example 95, Step B, there is obtained 1-(hydroxymethyl)-2-hydroxyethyl [(5,6-dichloro-9a-ethyl-3-oxo-1,2,9,9a-tetrahydro-3H-fluoren-7-yl)oxy]acetate. yl)oxy]acetate.

Example 97

[(5,6-Dichloro-9a-ethyl-3-oxo-1,2,9,9a-tetrahydro-3H-fluoren-7-yl)oxy]acetic acid hydrazide

By conducting a reaction as described in Example 92, except that an equimolar quantity of anhydrous hydrazine is used in place of the ammonium hydroxide, there is obtained [(5,6-dichloro-9a-ethyl-3-oxo-1,2,9,9a-tetrahydro-3H-fluoren-7-yl)oxy]acetic acid hydrazide.

Example 98

*Steps A and B.* The two enantiomers of [(5,6-dichloro-9a-cyclopropylmethyl-3-oxo-1,2,9,9a-tetrahydro-3H-fluoren-7-yl)oxy]acetic acid.

Carrying out the resolution as described in Example 25, except that the racemic [(5,6-dichloro-9a-ethyl-3-oxo-1,2,9,9a-tetrahydro-3H-fluoren-7-yl)oxy]acetic acid (Example 1, Step E) is substituted by an equimolar quantity of racemic [(5,6-dichloro-9a-cyclopropylmethyl-3-oxo-1,2,9,9a-tetrahydro-3H-fluoren-7-yl)oxy]acetic acid (Example 34, Step L), there is obtained the two enantiomers of [(5,6-dichloro-9a-cyclopropylmethyl-3-oxo-1,2,9,9a-tetrahydro-3H-fluoren-7-yl)oxy]acetic acid.

Example 99

*Steps A and B.* The two enantiomers of [(9a-benzyl-5,6-dichloro-3-oxo-1,2,9,9a-tetrahydro-3H-fluoren-7-yl)oxy]acetic acid.

Carrying out a reaction as described in Example 25, except that the racemic [(5,6-dichloro-9a-ethyl-3-oxo-1,2,9,9a-tetrahydro-3H-fluoren-7-yl)oxy]acetic acid (Example 1, Step E) is substituted by an equimolar quantity of racemic [(9a-benzyl-5,6-dichloro-3-oxo-1,2,9,9a-tetrahydro-3H-fluoren-7-yl)oxy]acetic acid (Example 9, Step E), there is obtained the two enantiomers of [(9a-benzyl-5,6-dichloro-3-oxo-1,2,9,9a-tetrahydro-3H-fluoren-7-yl)oxy]acetic acid.

Example 100

*Steps A and B.* The two enantiomers of [(5,6-dichloro-3-oxo-9a-propyl-1,2,9,9a-tetrahydro-3H-fluoren-7-yl)oxy]acetic acid.

Carrying out the resolution as described in Example 25, except that the racemic [(5,6-dichloro-9a-ethyl-3-oxo-1,2,9,9a-tetrahydro-3H-fluoren-7-yl)oxy]acetic acid is substituted by an equimolar quantity of [(5,6-dichloro-3-oxo-9a-propyl-1,2,9,9a-tetrahydro-3H-fluoren-7-yl)oxy]acetic acid (Example 4, Step E) there is obtained the two enantiomers of [(5,6-dichloro-3-oxo-9a-propyl-1,2,9,9a-tetrahydro-3H-fluoren-7-yl)oxy]acetic acid.

By carrying out a reaction as described in Example 54, Step A, except that the [(5,6-dichloro-9a-ethyl-3-oxo-1,2,9,9a-tetrahydro-3H-fluoren-7-yl)oxy]acetic acid is replaced by an equimolar quantity of:

Example 101

*Step A.* [(9a-Cyclopropyl-5,6-dichloro-3-oxo-1,2,9,9a-tetrahydro-3H-fluoren-7-yl)oxy]acetic acid (Example 33, Step L).

Example 102

*Step A.* (+)[(9a-Cyclopropylmethyl-5,6-dichloro-3-oxo-1,2,9,9a-tetrahydro-3H-fluoren-7-yl)oxy]acetic acid (Example 98).

Example 103

*Step A.* (—)[(9a-Cyclopropylmethyl-5,6-dichloro-3-oxo-1,2,9,9a-tetrahydro-3H-fluoren-7-yl)oxy]acetic acid (Example 98).

Example 104

*Step A.* (+)[(9a-Benzyl-5,6-dichloro-3-oxo-1,2,9,9a-tetrahydro-3H-fluoren-7-yl)oxy]acetic acid (Example 99).

Example 105

*Step A.* (—)[(9a-Benzyl-5,6-dichloro-3-oxo-1,2,9,9a-tetrahydro-3H-fluoren-7-yl)oxy]acetic acid (Example 99).

Example 106

*Step A.* (+)[5,6-Dichloro-3-oxo-9a-propargyl-1,2,9,9a-tetrahydro-3H-fluoren-7-yl)oxy]acetic acid (Example 28).

Example 107

*Step A.* (—)[(5,6-Dichloro-3-oxo-9a-propargyl-1,2,9,9a-tetrahydro-3H-fluoren-7-yl)oxy]acetic acid (Example 28).

Example 108

*Step A.* (+)[(5,6-Dichloro-3-oxo-9a-propyl-1,2,9,9a-tetrahydro-3H-fluoren-7-yl)oxy]acetic acid (Example 100).

Example 109

*Step A.* (—)[(5,6-Dichloro-3-oxo-9a-propyl-1,2,9,9a-tetrahydro-3H-fluoren-7-yl)oxy]acetic acid (Example 100).
there is obtained:

Example 101

*Step A.* Benzyloxycarbonylmethyl [(9a-cyclopropyl-5,6-dichloro-3-oxo-1,2,9,9a-tetrahydro-3H-fluoren-7-yl)oxy]acetate.

Example 102

*Step A.* Benzyloxycarbonylmethyl (+)[(9a-cyclopropylmethyl-5,6-dichloro-3-oxo-1,2,9,9a-tetrahydro-3H-fluoren-7-yl)oxy]acetate.

Example 103

*Step A.* Benzyloxycarbonylmethyl (—)[(9a-cyclopropylmethyl-5,6-dichloro-3-oxo-1,2,9,9a-tetrahydro-3H-fluoren-7-yl)oxy]acetate.

Example 104

*Step A.* Benzyloxycarbonylmethyl (+)[(9a-benzyl-5,6-dichloro-3-oxo-1,2,9,9a-tetrahydro-3H-fluoren-7-yl)oxy]acetate.

Example 105

Step A. Benzyloxycarbonylmethyl (—)[(9a-benzyl-5,6-dichloro-3-oxo-1,2,9,9a-tetrahydro-3H-fluoren-7-yl)oxy]acetate.

Example 106

*Step A.* Benzyloxycarbonylmethyl (+)[(5,6-dichloro-3-oxo-9a-propargyl-1,2,9,9a-tetrahydro-3H-fluoren-7-yl)oxy]acetate.

Example 107

*Step A.* Benzyloxycarbonylmethyl (—)[(5,6-dichloro-3-oxo-9a-propargyl-1,2,9,9a-tetrahydro-3H-fluoren-7-yl)oxy]acetate.

Example 108

*Step A.* Benzyloxycarbonylmethyl (+)[(5,6-dichloro-3-oxo-9a-propyl-1,2,9,9a-tetrahydro-3H-fluoren-7-yl)oxy]acetate.

Example 109

*Step A.* Benzyloxycarbonylmethyl (—)[(5,6-dichloro-3-oxo-9a-propyl-1,2,9,9a-tetrahydro-3H-fluoren-7-yl)oxy]acetate.

By carrying out a reaction as described in Example 54, Step B, except that the benzyloxycarbonylmethyl [(5,6-dichloro-9a-ethyl-3-oxo-1,2,9,9a-tetrahydro-3H-fluoren-7-yl)oxy]acetate is replaced by an equimolar quantity of the products of Example 101, Step A, Example 102, Step A, Example 103, Step A, Example 104, Step A, Example 105, Step A, Example 106, Step A, Example 107, Step A, Example 108, Step A, Example 109, Step A, respectively there is obtained:

Example 101

*Step B.* Carboxymethyl [(9a-cyclopropyl-5,6-dichloro-3-oxo-1,2,9,9a-tetrahydro-3H-fluoren-7-yl)oxy]acetate.

Example 102

*Step B.* Carboxymethyl (+)[(9a-cyclopropylmethyl-5,6-dichloro-3-oxo-1,2,9,9a-tetrahydro-3H-fluoren-7-yl)oxy]acetate.

Example 103

*Step B.* Carboxymethyl (—)[(9-cyclopropylmethyl-5,6-dichloro-3-oxo-1,2,9,9a-tetrahydro-3H-fluoren-7-yl)oxy]acetate.

Example 104

*Step B.* Carboxymethyl (+)[(9a-benzyl-5,6-dichloro-3-oxo-1,2,9,9a-tetrahydro-3H-fluoren-7-yl)oxy]acetate.

Example 105

*Step B.* Carboxymethyl (—)[(9a-benzyl-5,6-dichloro-3-oxo-1,2,9,9a-tetrahydro-3H-fluoren-7-yl)oxy]acetate.

Example 106

*Step B.* Carboxymethyl (+)[(5,6-dichloro-3-oxo-9a-propargyl-1,2,9,9a-tetrahydro-3H-fluoren-7-yl)oxy]acetate.

Example 107

*Step B.* Carboxymethyl (—)[(5,6-dichloro-3-oxo-9a-propargyl-1,2,9,9a-tetrahydro-3H-fluoren-7-yl)oxy]acetate.

Example 108

*Step B.* Carboxymethyl (+)[(5,6-dichloro-3-oxo-9a-propyl-1,2,9,9a-tetrahydro-3H-fluoren-7-yl)oxy]acetate.

Example 109

*Step B.* Carboxymethyl (—)[(5,6-dichloro-3-oxo-9a-propyl-1,2,9,9a-tetrahydro-3H-fluoren-7-yl)oxy]acetate.

Example 110

*Step A.* 3-(Benzyloxycarbonyl)propyl (+)[(5,6-dichloro-9a-ethyl-3-oxo-1,2,9,9a-tetrahydro-3H-fluoren-7-yl)oxy]acetate.

By carrying out the reaction as described in Example 57, Step A, except that the racemic [(5,6-dichloro-9a-ethyl-3-oxo-1,2,9,9a-tetrahydro-3H-fluoren-7-yl)oxy]acetic acid (Example 20, Step D) is replaced by an equimolar amount of (+)[(5,6-dichloro-9a-ethyl-3-oxo-1,2,9,9a-tetrahydro-3H-fluoren-7-yl)oxy]acetic acid, there is obtained 3-(benzyloxycarbonyl)propyl (+)[(5,6-dichloro-9a-ethyl-3-oxo-1,2,9,9a-tetrahydro-3H-fluoren-7-yl)oxy]acetate.

*Step B.* 3-Carboxypropyl (+)[(5,6-dichloro-3-9a-ethyl-1,2,9,9a-tetrahydro-3H-fluoren-7-yl)oxy]acetate.

By carrying out the reaction as described in Example 57, Step B, except that the racemic 3-(benzyloxycarbonyl)propyl [(5,6 - dichloro - 9a - ethyl - 3 - oxo - 1,2,9,9a - tetrahydro - 3H - fluoren - 7-yl)oxy]acetate is replaced by an equimolar amount of the 3-(benzyloxycarbonyl)propyl (+)[(5,6-dichloro-9a-ethyl-3-oxo-1,2,9,9a-tetrahydro-3H-fluoren-7-yl)oxy]acetate of Example 110, Step A, there is obtained:

3-Carboxypropyl (+)[(5,6-dichloro-9a-ethyl-3-oxo-1,2,9,9a-tetrahydro-3H-fluoren-7-yl)oxy]acetate.

Example 111

*Step A.* 3-(Benzyloxycarbonyl)propyl (—)[(5,6-dichloro-9a-ethyl-3-oxo-1,2,9,9a-tetrahydro-3H-fluoren-7-yl)oxy]acetate.

By carrying out the reaction as described in Example 57, Step A, except that the [(5,6-dichloro-9a-ethyl-3-oxo-1,2,9,9a-tetrahydro-3H-fluoren-7-yl)oxy]acetic acid is replaced by an equimolar amount of (—)[(5,6-dichloro-9a-ethyl-3-oxo-1,2,9,9a-tetrahydro-3H-fluoren-7-yl)oxy]acetic acid, there is obtained 3-(benzyloxycarbonyl)propyl (—)[(5,6-dichloro-9a-ethyl-3-oxo-1,2,9,9a-tetrahydro-3H-fluoren-7-yl)oxy]acetate.

*Step B.* 3-Carboxypropyl (—)[(5,6-dichloro-9a-ethyl-1,2,9,9a-tetrahydro-3H-fluoren-7-yl)oxy]acetate.

By carrying out the reaction as described in Example 57, Step B, except that the racemic 3-(benzyloxycarbonyl)propyl [(5,6 - dichloro - 9a - ethyl - 3 - oxo - 1,2,9,9a - tetrahydro - 3H - fluoren - 7-yl)oxy]acetate is replaced by an equimolar amount of the product of Example 111, Step A, there is obtained 3-carboxypropyl (—)[(5,6-dichloro-9a-ethyl-3-oxo-1,2,9,9a-tetrahydro-3H-fluoren-7-yl)oxy]-acetate.

Example 112

Dry-filled capsules containing 50 mg. of active ingredient per capsule

| | Per Capsule |
|---|---|
| Ethyl [(5,6-dichloro-9a-ethyl-3-oxo-1,2,9,9a-tetrahydro-3H-fluoren-7-yl)oxy]-acetate | 50 mg. |
| Lactose | 149 mg. |
| Magnesium Stearate | 1 mg. |
| Capsule (Size No. 1) | 200 mg. |

The ethyl [(5,6-dichloro-9a-ethyl-3-oxo-1,2,9,9a-tetrahydro-3H-fluoren-7-yl)oxy]acetate (Example 1, Step D) is reduced to a No. 60 powder and then lactose and magnesium stearate are passed through a No. 60 bolting cloth onto the powder and the combined ingredients admixed for 10 minutes and then filled into a No. 1 dry gelatin capsule.

Similar dry-filled capsules can be prepared by replacing the active ingredient of the above example by any of the other compounds of this invention.

Example 113

Parenteral Solution of the Sodium Salt of Carboxymethyl-[(5,6-dichloro-9a-ethyl-3-oxo-1,2,9,9a-tetrahydro-3H-fluoren-7-yl)oxy]acetate

Carboxymethyl [(5,6-dichloro-9a-ethyl-3-oxo-1,2,9,9a-tetrahydro-3H-fluoren-7-yl)oxy]acetate (Example 54, Step B) (100 mg.) is dissolved in 0.05N sodium bicarbonate solution (6 ml.) by warming and stirring. The solution is then diluted to 10 ml with water and sterilized by filtration. All the water used in the preparation is pyrogen-free.

Example 114

Parenteral Solution of the 1-Methylpiperazinium Salt of Carboxymethyl [(5,6-dichloro-9a-benzyl-3-oxo-1,2,9,9a-tetrahydro-3H-fluoren-7-yl)oxy]acetate

Carboxymethyl [(5,6-dichloro-9a-benzyl-3-oxo-1,2,9,9a-tetrahydro-3H-fluoren-7-yl)oxy]acetate (100 mg.) (Example 62, Step B) is dissolved by warming and stirring in a solution of 0.05N 1-methylpiperazine (6 ml). The solution is then diluted to 10 ml with water and sterilized by filtration. All the water used in the preparation is pyrogen-free.

Example 115

Dry-Filled Capsules Containing 50 mg. of Active Ingredient Per Capsule

| | Per Capsule |
|---|---|
| Carboxymethyl [(5,6-dichloro-9a-allyl-3-oxo-1,2,9,9a-tetrahydro-3H-fluoren-7-yl)oxy]acetate | 50 mg. |
| Lactose | 49 mg. |
| Magnesium Stearate | 1 mg. |
| Capsule (Size No. 1) | 100 mg. |

The carboxymethyl [(5,6-dichloro-9a-allyl-3-oxo-1,2,9,9a-tetrahydro-3H-fluoren-7-yl)oxy]acetate (Example 59, Step B) is reduced to a No. 60 powder and then lactose and magnesium stearate are passed through a No. 60 bolting cloth onto the powder and the combined ingredients admixed for 10 minutes and then filled into a No. 2 dry gelatin capsule.

Example 116

Parenteral Solution of the Sodium Salt of Carboxymethyl [(5,6-dichloro-9a-propargyl-3-oxo-1,2,9,9a-tetrahydro-3H-fluoren-7-yl)oxy]acetate

Carboxymethyl [(5,6 - Dichloro - 9a - propargyl - 3 - oxo - 1,2,9,9a - tetrahydro - 3H - fluoren - 7-yl)oxy]acetate (Example 60, Step B) (100 mg.) is dissolved by stirring and warming in a solution of 0.05N sodium bicarbonate (6 ml). The solution is then distilled to 10 ml and sterilized. All the water used in the preparation is pyrogen-free.

Example 117

Dry-Filled Capsules Containing 50 mg. of Active Ingredient Per Capsule

| | Per Capsule |
|---|---|
| Carboxymethyl(+)[(5,6-dichloro-9a-propyl-3-oxo-1,2,9,9a-tetrahydro-3H-fluoren-7-yl)-oxy]acetate | 50 mg. |
| Lactose | 49 mg. |
| Magnesium Stearate | 1 mg. |
| Capsule (Size No. 1) | 100 mg. |

The carboxymethyl (+)[(5,6 - dichloro - 9a - propyl - 3 oxo - 1,2,9,9a - tetrahydro - 3H - fluoren - 7-yl)oxy]acetate (Example 108, Step B) is reduced to a No. 60 powder and then lactose and magnesium stearate are passed through a No. 60 bolting cloth onto the powder and the combined ingredients admixed for 10 minutes and then filled into a No. 2 dry gelatin capsule.

Example 118

Parenteral Solution of the Sodium Salt of (+)carboxymethyl[(5,6-dichloro-9a-propyl-3-oxo-1,2,9,9a-tetrahydro-3H-fluoren-7-yl)oxy]acetate

Carboxymethyl (+)[(5,6-dichloro-9a-propyl-3-oxo-1,2,9,9a-tetrahydro-3H-fluoren-7-yl)oxy]acetate (Example 108, Step B) (100 mg) is dissolved by stirring and warming in a solution of 0.05N sodium bicarbonate (6 ml). The solution is then diluted to 10 ml. and sterilized by filtration. All the water used in the preparation is pyrogen-free.

Example 119

Parenteral Solution of the Sodium Salt of (—)Carboxymethyl[(5,6-dichloro-9a-propyl-3-oxo-1,2,9,9a-tetrahydro-3H-fluoren-7-yl)oxy]acetate

Carboxymethyl (—)[(5,6-dichloro-9a-propyl-3-oxo-1,2,9,9a-tetrahydro-3H-fluoren-7-yl)oxy]acetate (Example 109, Step B) (100 mg) is dissolved by stirring and warming in a solution of 0.05N sodium bicarbonate (6 ml). The solution is then diluted to 10 ml. and sterilized by filtration. All the water used in the preparation is pyrogen-free.

In formula Ib′

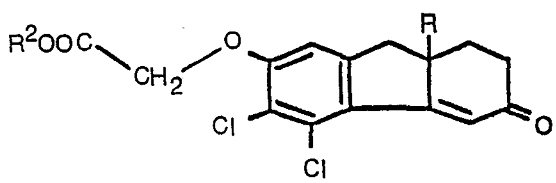

$R^2$ can also be hydrogen.

In addition to the preferred compounds at page 4 there also are preferred [(5,6-dichloro-9α-methyl-3-oxo-1,2,9,9α-tetrahydro-3H-fluoren-7-yl)oxy]acetic acid;

[(5,6-dichloro-3-oxo-9α-isopropyl-1,2,9,9α-tetrahydro-3H-fluoren-7-yl)oxy]acetic acid;

[(5,6-dichloro-3-oxo-9α-vinyl-1,2,9,9α-tetrahydro-3H-fluoren-7-yl)oxy]acetic acid;

[(5,6-dichloro-9α-cyclopropyl-3-oxo-1,2,9,9α-tetrahydro-3H-fluoren-7-yl)oxy]acetic acid, and

[(5,6-dichloro-9α-ethyl-3-oxo-1,2,9,9α-tetrahydro-3H-fluoren-7-yl)oxy]acetic acid hydrazide.

Especially preferred are the (+)R enantiomers of the racemic modification of each compound. The more preferred compounds are the (+)R enantiomers of the preferred compounds named specifically.

The parent acids of the esters set forth at page 4, e.g. [(5,6-dichloro-9α-ethyl-3-oxo-1,2,9,9α-tetrahydro-3H-fluoren-7-yl)oxy]acetic acid are also preferred, as well as the following esters set forth in Table A.

TABLE A

$R^1$ [(5,6-dichloro-3-oxo-9$\alpha$-R-1,2,9,9$\alpha$-tetrahydro-3H-fluoren-7-yl)oxy]acetate

| $R^1$ | R |
|---|---|
| 1-carboxy-1-methylethyl | ethyl |
| " | isopropyl |
| " | propyl |
| " | butyl |
| " | vinyl |
| " | allyl |
| " | propargyl |
| " | cyclopropyl |
| " | cyclopropylmethyl |
| " | benzyl |
| ethyl | ethyl |
| 3-pyridylmethyl | ethyl |
| 2-oxopropyl | ethyl |
| carboxymethyl | ethyl |
| 3-hydroxypropyl | ethyl |

Special methods are used to synthesize compounds of Formula II where R is vinyl (Formula II B) or cyclopropyl (Formula II C) as shown below.

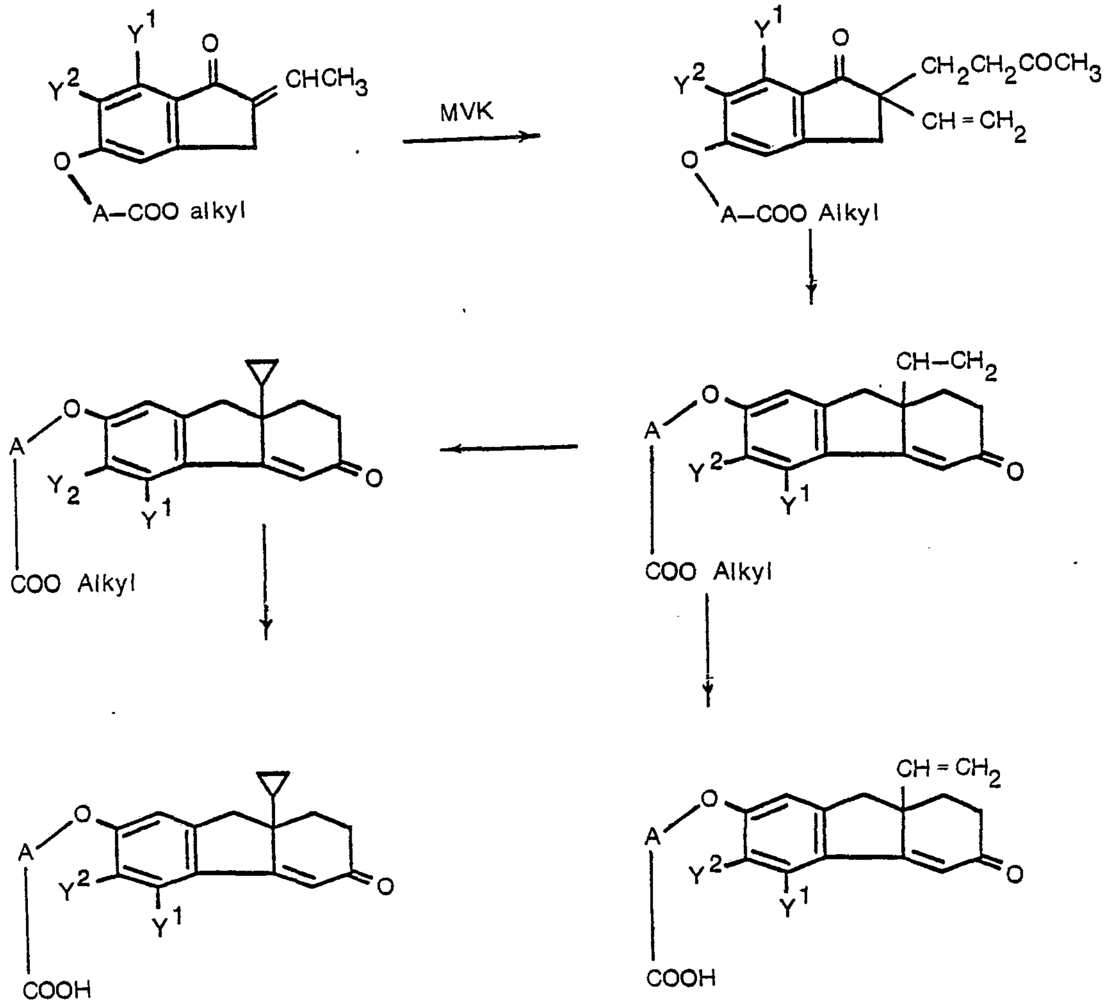


II C

II B

An alkyl [(2-ethylidene-1-oxo-1,2-dihydro-1H-inden-5-yl)oxy] alkonoate is treated with methyl vinyl ketone (MVK) in the presence of a base, such as, potassium tert.-butoxide in 1,2-dimethoxyethano at 0°C or Lithium dimethylamide in tetrahydrofuran at −70°. The reaction is conducted in an atmosphere of dry nitrogen.

The alkyl {[1-oxo-2-(3-oxobutyl)-2-vinyl-2,5-dihydro-1H-inden-5-yl]oxy}alkanoate produced is cyclized to an alkyl [(3-oxo-9$\alpha$-vinyl-1,2,9,9$\alpha$-tetrahydro-3H-fluoren-7-yl)oxy] alkanoate by heating the presence of a catalyst, such as pyrrolidine acetate in benzene or toluene. Heating with a base, such as, potassium tert.butoxide in tert-butyl alcohol also is effective. Finally, the compound of Formula II B is produced by saponification of the corresponding alkyl ester by heating in aqueous base, such as, 5% aqueous sodium hydroxide followed by neutralization with aqueous acid, such as, hydrochloric acid. The cyclization and saponification may be effected by heating the alkyl {[1-oxo-2-(3-oxobutyl)-2-vinyl-1,2-dihydro-2H-inden-5-yl]oxy}alkanoate with aqueous acid, such as aqueous acetic acid containing hydrochloric acid or by aqueous base, such as, sodium hydroxide, followed by acidification with aqueous acid, such as, hydrochloric acid.

The preparation of compounds of Formula II C is carried out by treating a [(3-oxo-9$\alpha$-vinyl-1,2,9,9$\alpha$-tetrahydro-3H-fluoren-7-yl)oxy]alkanoate with a base such as sodium hydride in dimethylformamide followed by the addition of a solution prepared from trimethylsulfoxonium iodide and sodium hydride in dimethylformamide. The [(9$\alpha$-cyclopropyl-3-oxo-1,2,9,9$\alpha$-tetrahydro-3H-fluoren-7-yl)oxy]alkanoate that is produced is saponified to a compound of Formula II C by heating with aqueous acid, such as, aqueous acetic acid containing hydrochloric acid or by aqueous base, such as, sodium hydroxide followed by acidification with aqueous acid, such as, hydrochloric acid.

Example 120

[(5,6-Dichloro-9$\alpha$-isopropyl-3-oxo-1,2,9,9$\alpha$-tetrahydro-3H-fluoren-7-yl)oxy]acetic acid

*Step A.* Methyl [(6,7-dichloro-2-isopropyl-1-oxo-2,3-dihydro-1H-inden-5-yl)oxy]acetate

[(6,7-Dichloro-2-isopropyl-1-oxo-2,3-dihydro-1H-inden-5-yl)oxy]acetic acid 33.4 gm. 0.1 mole) is dissolved in dimethylformamide (300 ml.) and potassium carbonate (20.7 gm., 0.15 mole) is added. The mixture is stirred and heated at 60°C. for 10 minutes in a vessel protected from atmospheric moisture. Methyl iodide (12.6 ml., 28.4 gm., 0.2 mole) is added and heating and stirring continued for 3 hours. The mixture is cooled, added, with stirring to water (3 liters) and the solid that separates removed by filtration and dried. Recrystallization from methanol gives methyl [(6,7-dichloro-2-isopropyl-1-oxo-2,3-dihydro-1H-inden-5-yl)oxy]acetate.

*Step B.* Methyl {[6,7-dichloro-2-isopropyl-1-oxo-2-(3-oxobutyl-2,3-dihydro-1H-inden-5-yl]oxy}-acetate

Methyl [(6,7-dichloro-2-isopropyl-1-oxo-2,3-dihydro-1H-inden-5-yl)oxy]acetate (16.5 gm., 0.05 mole) is suspended in dry tetrahydrofuran (100 ml.) containing triton-B (1 ml.). The suspension is stirred at ambient temperature and methyl vinyl ketone (5.02 gm., 0.072 mole) is added. The solution which becomes warm initially, is stirred at ambient temperature for 4 hours. Then, the stirring solution is treated with methyl vinyl ketone (0.3 ml.) and triton-B (0.6 ml.) every 4 hours for the next twenty hours.

The reaction mixture is evaporated *in vacuo* at 50°C. and the residual material dissolved in either (100 ml.). The ether solution is washed with water (two 20 ml. portions), dried over anhydrous magnesium sulfate and filtered. The filtrate is evaporated *in vacuo* and the residue triturated with ether (10 ml.), filtered and dried. The product is methyl {[6,7-dichloro-2-isopropyl-1-oxo-2-(3-oxobutyl)-2,3-dihydro-1H-inden-5-yl]oxy}acetate.

*Step C.* Methyl [5,6-dichloro-9$\alpha$-isopropyl-3-oxo-1,2,9,9$\alpha$-tetrahydro-3H-fluoren-7-yl)oxy]acetate

A mixture of methyl [(6,7-dichloro-2-isopropyl-1-oxo-2-(3-oxobutyl)-2,3-dihydro-1H-inden-5-yl)oxy]acetate (9.3 gm., 0.024 mole) potassium tert.-butoxide (2.70 gm., 0.024 mole) in tert.-butyl alcohol (100 ml.) is heated at 85°C. for three hours. The solution is concentrated *in vacuo* at 50°C. to obtain the crude product.

The product is subjected to column chromatography on silica gel (300 gm.) using a mixture of dichloromethane and tetrahydrofuran (100/4 v./v.) as the eluent. The product is pure methyl [(5,6-dichloro-9$\alpha$-isopropyl-3-oxo-1,2,9,9$\alpha$-tetrahydro-3H-fluoren-7-yl)oxy]acetate.

*Step D.* [(5,6-Dichloro-9$\alpha$-isopropyl-3-oxo-1,2,9,9$\alpha$-tetrahydro-3H-fluoren-7-yl)oxy]acetic acid.

Methyl [(5,6-dichloro-9$\alpha$-isopropyl-3-oxo-1,2,9,9$\alpha$-tetrahydro-3H-fluoren-7-yl)oxy]acetate (4.2 gm., 0.011 mole) is added to a solution composed of 20% aqueous sodium hydroxide solution (4.32 ml., 0.022 mole) and methanol (45 ml.). The mixture is stirred and heated at reflux for two hours and then concentrated *in vacuo* at 50°C. The residue is dissolved in water (50 ml.) and made acid to Congo red paper with 6 normal hydrochloric acid. The solid that separates is removed by filtration, washed with water, then with a little methanol and finally with a little ether. After drying, the product is recrystallized from acetic acid to give pure [(5,6-dichloro-9$\alpha$-isopropyl-3-oxo-1,2,9,9$\alpha$-tetrahydro-3H-fluoren-7-yl)oxy]acetic acid. Using a procedure similar to that described in Example 120, except that in Step A there is substituted for the [(6,7-dichloro-2-isopropyl-1-oxo-2,3-dihydro-1H-inden-5-yl)oxy]acetic acid, an equimolar amount of:

Example 121

*Step A.* [(6,7-Dichloro-2-cyclopentyl-1-oxo-2,3-dihydro-1H-inden-5-yl)oxy]acetic acid.

Example 122

*Step A.* [(6,7-Dichloro-1-oxo-2-phenyl-2,3-dihydro-1H-inden-5-yl)oxy]acetic acid.

Example 123

*Step A.* [(2-Benzyl-6,7-dichloro-1-oxo-2,3-dihydro-1H-inden-5-yl)oxy]acetic acid.

In Examples 121, Step A through 123, Step A, the reaction is carried out as described in Example 120, Step A, there is obtained:

Example 121

*Step A.* Methyl [(6,7-dichloro-2-cyclopentyl-1-oxo-2,3-dihydro-1H-inden-5-yl)oxy]acetate.

Example 122

*Step A.* Methyl [(6,7-dichloro-1-oxo-2-phenyl-2,3-dihydro-1H-inden-5-yl)oxy]acetate.

Example 123

*Step A.* Methyl [(2-benzyl-6,7-dichloro-1-oxo-2,3-dihydro-1H-inden-5-yl)oxy]acetate.

In Example 120, Step B, the starting material is the product from Step A. Using the products from Step A of Example 121, 122 and 123 as starting materials but conducting the reaction as in Example 120, Step B, there is obtained:

Example 121

*Step B.* Methyl [(6,7-dichloro-2-cyclopentyl-1-oxo-2-(3-oxobutyl)-2,3-dihydro-1H-inden-5-yl)acetate.

Example 122

*Step B.* Methyl [(6,7-dichloro-1-oxo-2-(3-oxobutyl)-2-phenyl-2,3-dihydro-1H-inden-5-yl)oxy]acetate.

Example 123

*Step B.* Methyl [(2-benzyl-6,7-dichloro-1-oxo-2-(3-oxobutyl)-2,3-dihydro-1H-inden-5-yl)oxy]acetate.

Using the products from Step B of Example 121, 122, 123 as starting material in place for the Example 120, Step B, and conducting the reaction as in Example 120, Step C there is obtained:

Example 121

*Step C.* Methyl [(5,6-dichloro-9a-cyclopentyl-3-oxo-1,2,9,9a-tetrahydro-3H-fluoren-7-yl)-oxy]acetate.

Example 122

*Step C.* Methyl [(5,6-dichloro-3-oxo-9a-phenyl-1,2,9,9a-tetrahydro-3H-fluoren-7-yl)oxy]acetate.

Example 123

*Step C.* Methyl [(9a-benzyl-5,6-dichloro-3-oxo-1,2,9,9a-tetrahydro-3H-fluoren-7-yl)oxy]acetate.

Using the products from Step C of Example 121, 122 and 123 as starting materials in place of the product from Example 120, Step C and conducting the reaction as in Example 120, Step D, there is obtained:

Example 121

*Step D.* [(5,6-Dichloro-9a-cyclopentyl-3-oxo-1,2,9,9a-tetrahydro-3H-fluoren-7-yl)oxy]acetate.

Example 122

*Step D.* [(5,6-Dichloro-3-oxo-9a-phenyl-1,2,9,9a-tetrahydro-3H-fluoren-7-yl)oxy]acetate.

Example 123

*Step D.* [(9a-Benzyl-5,6-dichloro-3-oxo-1,2,9,9a-tetrahydro-3H-fluoren-7-yl)oxy]acetate.

**Claims**

1. A compound of formula I

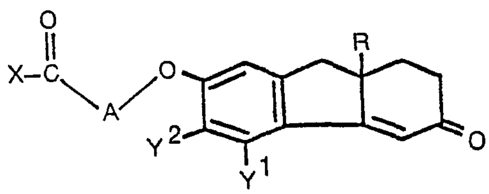

I

wherein

R is H, lower alkyl, branched or unbranched; aryl, aryl $C_1$ to $C_4$ alkyl where the alkyl is branched or unbranched; $C_2$ to $C_4$ alkynyl, branched or unbranched; $C_3$ to $C_6$ cycloalkyl and $C_3$ to $C_6$ cycloalkyl-$C_1$ to $C_4$ alkyl, branched or unbranched; $C_2$ to $C_4$ alkenyl, branched or unbranched;

X is hydroxy, a group forming an acid anhydride, amide, hydrazide, or guanidide or a group having the formula —O—$R^1$, wherein

$R^1$ is lower alkyl, lower alkenyl, lower alkynyl, lower cycloalkyl, heteroaryl, aryl, carboxy-substituted cycloalkyl, substituted lower alkyl, where the substituent is carboxy, lower alkoxycarbonyl, oxo, hydroxy, lower alkoxy, halo, lower acyloxy, lower dialkylamino, sulfamoyl, pyridyl, furyl, tetrahydrofuryl, 1-methylpiperidyl, morpholinyl, pyrrolidinyl, 1-methylpiperazinyl, thienyl or aryl.

$Y^1$ and $Y^2$ are independently Cl and $CH_3$;

A is the group

$$-CH_2-CH_2- \text{ or } -\overset{R^2}{\underset{R^3}{C}}-$$

wherein

$R^2$ is H, methyl or ethyl,

$R^3$ is H, F or methyl or

$R^2$ and $R^3$ may be joined together to form the ring

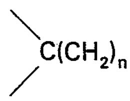

where

n is the integer 2, 3 or 4

and pharmaceutically acceptable salts of the compounds of formula I.

2. A compound of formula I wherein

X is the group of formula —$OR^1$, wherein $R^1$ is H, an alkyl group having 1—4 carbon atoms, an alkenyl group having 2—4 carbon atoms, an alkynyl group having 3 or 4 carbon atoms, cycloalkyl having 4 or 5 carbon atoms, carboxy-cycloalkyl, imidazolyl, pyridyl, thiazolyl, pyrazinyl, furyl, phenyl, carboxyphenyl, hydroxymethylphenyl, carboxy-substituted lower cycloalkyl or substituted lower alkyl, where the substituent is carboxy, lower alkoxycarbonyl, oxo, hydroxy, lower alkoxy, halo, lower acyloxy, lower dialkylamino, sulfamoyl, pyridyl, furyl, tetrahydrofuryl, 1-methylpiperidyl, morpholinyl, pyrrolidinyl, 1-methylpiperazinyl, thienyl or aryl.

3. A compound according to claim 1 or 2, wherein X is the group of formula —O—$R^1$ wherein $R^1$ is lower alkyl or carboxyloweralkyl.

4. A compound according to claim 1 or 2, where $Y^1$ and $Y^2$ are Cl.

5. A compound according to claim 1 or 2, wherein A is the group

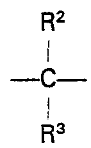

wherein $R^2$ and $R^3$ are as defined in claim 1.

6. A compound according to claim 1 or 2, wherein R is phenyl.

7. A compound according to claim 1 which is the [(5,6-dichloro-3-oxo-9α-propyl-1,2,9,9α-tetrahydro-3H-fluoren-7-yl)oxy]acetic acid.

8. A composition in unit dosage form for the treatment of grey matter edema comprising a compound of the formula I

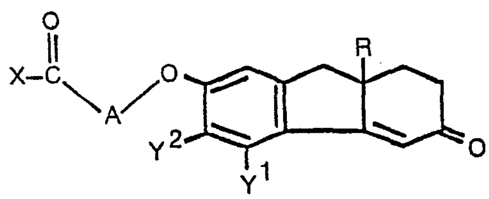

I

wherein

R, X, $Y^1$, $Y^2$ and A have the same meaning as in formula I of claim 1

or a pharmaceutically acceptable salt thereof.

9. A process for making a compound of formula Ib

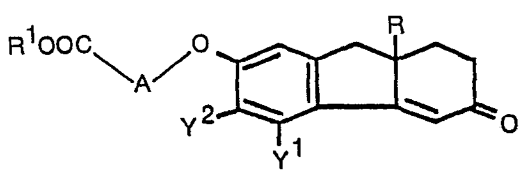

Ib

wherein

R, $R^1$, $Y^1$, $Y^2$ and A have the same meaning as in formula I of claim 1

which comprises reacting a compound of formula II

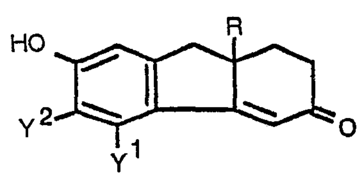

II

wherein
R, $Y^1$ and $Y^2$ are as in formula Ib
with an ester of formula III

$$X—A—COOR^1$$

wherein
X is a halo and
A and $R^1$ are as in formula Ib,
preferably in the presence of a base.

10. A process for making a compound of formula Ib

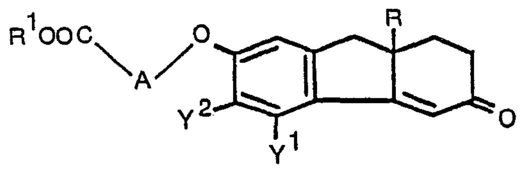

Ib

wherein
R, $R^1$, $Y^1$, $Y^2$ and A have the same meaning as in formula I of claim 1, which comprises the cyclization of a compound of formula IV

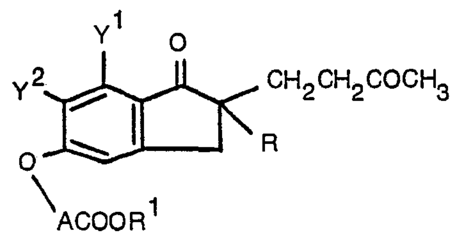

IV

by heating it in a solvent with a catalyst such as pyrrolidine acetate.

11. A process for making a compound of formula Ib

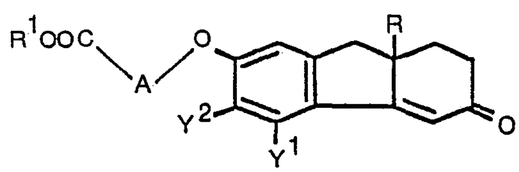

Ib

wherein
R, $R^1$, $Y^1$, $Y^2$ and A have the same meaning as in formula I of claim 1 which comprises a transesterification of a compound of formula Ic

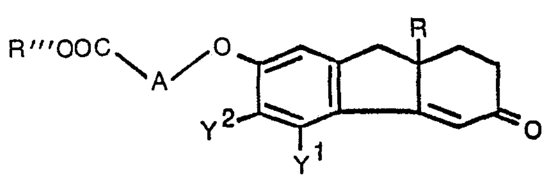

Ic

wherein
R''' has the same meaning as $R^1$ in formula I, but cannot be identical with $R^1$ with an alcohol of formula $R^1$-OH by heating the mixture in a solvent in the presence of an ion exchange resin and a catalytic amount of $R^1$-OM where M represents an alkali metal ion.

**Revendications**

1. Un composé de formule (I)

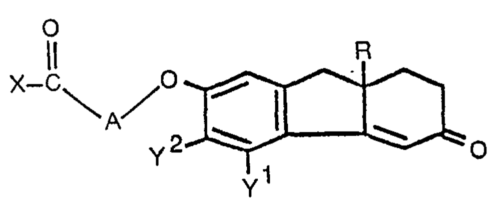

formule I

où

R est H, un alkyle inférieur, droit ou ramifié; un aryle, un aryle $C_1$ à $C_4$ alkyle où l'alkyle est droit ou ramifié; un $C_2$ à $C_4$ alkynyle, droit ou ramifié; un $C_3$ à $C_6$ cycloalkyle et un $C_3$ à $C_6$ cycloalkyl $C_1$ à $C_4$ alkyle, droits ou ramifiés; un $C_2$ à $C_4$ alcényle droit ou ramifié;

X est un hydroxy, un groupe formant un anhydride d'acide, un amide, un hydrazide, ou un guanidide ou un groupe ayant la formula $-O-R^1$, où

$R^1$ est un alkyle inférieur un alcényle inférieur, un alcynyle inférieur, un cycloalkyle inférieur, un hétéroaryle, un aryle, un cycloalkyle-carboxy substitué, un alkyle inférieur substitué, où le substituant est un carboxy, un alkoxycarbonyle inférieur, un oxo, un hydroxy, un alkoxy inférieur, un halo, un acyloxy inférieur, un dialkylamino inférieur, un sulfamoyle, un pyridyle, un furyle, un tétrahydrofuryle, un 1-méthylpipéridyle, un morpholinyle, un pyrrolidinyle, un 1-méthylpipérazinyle, un thiényle ou un aryle.

$Y^1$ et $Y^2$ sont, d'une façon indépendante, Cl et $CH_3$;

A est le groupe

$-CH_2-CH_2-$ ou 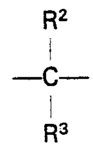

où

$R^2$ est H, un méthyle ou un éthyle,

$R^3$ est H, F ou un méthyle ou

$R^2$ et $R^3$ peuvent former ensemble le cycle

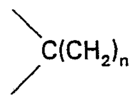

où

n est le nombre entier 2, 3 ou 4

et des sels acceptables pharmaceutiquement des composés de Formule (I).

2. Un composé de formule (I) où

X est le groupe de formule $-OR^1$, où $R^1$ est H, un groupe alkyle ayant 1—4 atomes de carbone, un groupe alcényle ayant 2—4 atomes de carbone, un groupe alcynyle ayant 3 ou 4 atomes de carbone, un cycloalkyle ayant 4 ou 5 atomes de carbone, un carboxycycloalkyle, un imidazolyle, un pyridyle, un thiazolyle, un pyrazinyle, un furyle, un phényle, un carboxyphényle, un hydroxyméthyl-phényle, un cycloalkyle inférieur-carboxy substitué ou un alkyle inférieur substitué, où le substituant est un carboxy, un alkoxycarbonyle inférieur, un oxo, un hydroxy, un alkoxy inférieur, un halogéno, un acyloxy inférieur, un dialkylamino inférieur, un sulfamoyle, un pyridyle, un furyle, un tétrahydro-furyle, un 1-méthylpipéridyle, un morpholinyle, un pyrrolidinyle, un 1-méthylpipérazinyle, un thiényle ou un aryle.

3. Un composé selon les revendications 1 ou 2, dans lequel X est le groupe de formule $-O-R^1$ où $R^1$ est un alkyle inférieur ou un carboxyalkyle inférieur.

4. Un composé selon les revendications 1 ou 2, dans lequel $Y^1$ et $Y^2$ sont Cl.

5. Un composé selon les revendications 1 ou 2, dans lequel A est le groupe

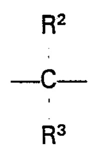

où $R^2$ et $R^3$ sont comme définis dans la revendication 1.

6. Un composé selon les revendications 1 ou 2, dans lequel R est un phényle.

7. Un composé selon la revendication 1 qui est l'acide [(5,6-dichloro-3-oxo-9$\alpha$-propyl-1,2,9,9a-tétrahydro-3H-fluoren-7-yl)oxy]acétique.

8. Une composition sous forme de dose unitaire pour le traitement d'un oedème de la matière grise comprenant un composé de formule (I)

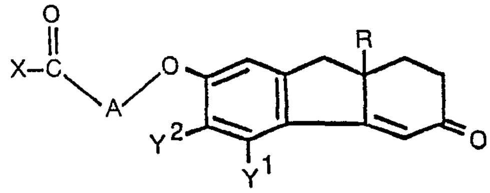

Formule I

où

R, X, $Y^1$, $Y^2$ et A ont la même signification que dans la formule (I) de la revendication 1 ou un sel acceptable pharmaceutiquement à partir de celle-ci.

9. Un procédé de préparation d'un composé de formule (Ib)

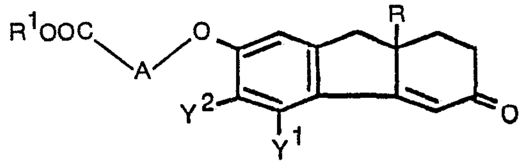

formule Ib

où

R, $R^1$, $Y^1$, $Y^2$ et A ont la même signification que dans la formule (I) de la revendication 1 qui comprend la réaction d'un composé de formule (II)

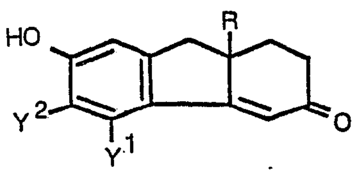

formule II

où

R, $Y^1$ et $Y^2$ sont comme dans la formule (Ib)
avec un ester de formule (III)

$$X—A—COOR^1 \qquad (III)$$

où

X est un halogène et
A et $R^1$ sont comme dans la formule (Ib)
de préférence en présence d'un base.

10. Un procédé de préparation d'un composé de formule (Ib)

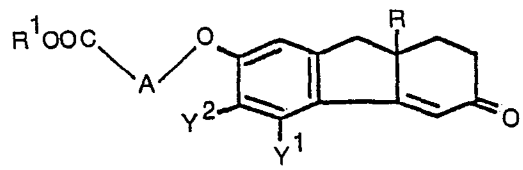

formule Ib

où

R, $R^1$, $Y^1$, $Y^2$ et A ont la même signification que dans la formule I de la revendication 1, qui comprend la cyclisation d'un composé de formule (IV)

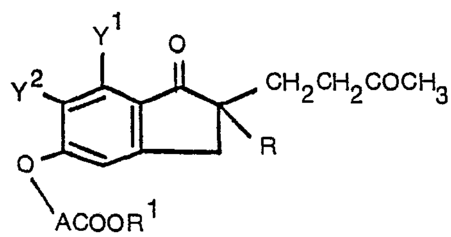

formule IV

en le chauffant dans un solvant avec un catalyseur comme l'acétate de pyrrolidine.

11. Un procédé de préparation d'un composé de formule (Ib)

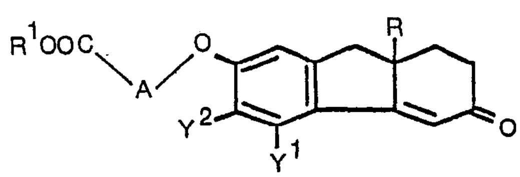

formule Ib

où

R, $R^1$, $Y^1$, $Y^2$ et A ont la même signification que dans la formule (I) de la revendication 1 qui comprend une transestérification d'un composé de formule Ic

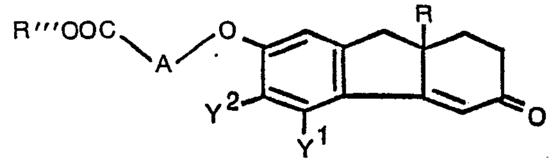

formule Ic

où

R''' a la même signification que $R^1$ dans la formule (I), mais ne peut pas être identique à $R^1$ avec un alcool de formule $R^1$-OH par chauffage du mélange dans un solvant en présence d'une résine échangeuse d'ion et d'une quantité catalytique de $R^1$-OM où M représente un ion de métal alcalin.

## Patentansprüche

1. Verbindung der Formel I

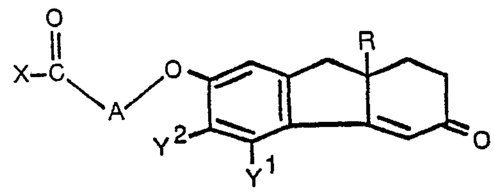

I

worin

R H, niedrig-Alkyl, verzweigt oder unverzweigt; Aryl, Aryl-$C_1$ bis $C_4$-alkyl, worin das Alkyl verzweigt oder unverzweigt ist; $C_2$ bis $C_4$-Alkinyl, verzweigt oder unverzweigt; $C_3$ bis $C_6$-Cycloalkyl und $C_3$ bis $C_6$-Cycloalkyl-$C_1$ bis $C_4$-alkyl, verzweigt oder unverzweigt; $C_2$ bis $C_4$-Alkenyl, verzweigt oder unverzweigt; ist;

X Hydroxy, eine Gruppe die ein Säureanhydrid, Amid, Hydrazid oder Guanidid bildet oder eine Gruppe mit der Formel —O—$R^1$ ist, worin

$R^1$ niedrig-Alkyl, niedrig-Alkenyl, niedrig-Alkinyl, niedrig-Cycloalkyl, Heteroaryl, Aryl, Carboxy-substituiertes Cycloalkyl, substituiertes niedrig-Alkyl, worin der Substituent Carboxy, niedrig-Alkoxycarbonyl, Oxo, Hydroxy, niedrig-Alkoxy, Halogen, niedrig-Acyloxy, niedrig-Dialkylamino, Sulfamoyl, Pyridyl, Furyl, Tetrahydrofuryl, 1-Methylpiperidyl, Morpholinyl, Pyrrolidinyl, 1-Methylpiperazinyl, Thienyl oder Aryl ist, bedeutet;

$Y^1$ und $Y^2$ unabhängig Cl und $CH_3$ sind,

A die Gruppe

$$-CH_2CH_2- \text{ oder } -\overset{R^2}{\underset{R^3}{C}}- \text{ ist}$$

worin

$R^2$ H, Methyl oder Ethyl ist,

$R^3$ H, F oder Methyl ist oder

$R^2$ und $R^3$ miteinander verbunden sein können, unter Bildung des Ringes

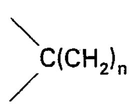

worin n die ganze Zahl 2, 3 oder 4 ist

und pharmazeutisch brauchbare Salze der Verbindungen der Formel I.

2. Verbindung der Formel I worin

X die Gruppe der Formel —$OR^1$ ist, worin $R^1$ H, eine Alkylgruppe mit 1—4 Kohlenstoffatomen, eine Alkenylgruppe mit 2—4 Kohlenstoffatomen, eine Alkinylgruppe mit 3 oder 4 Kohlenstoffatomen, Cycloalkyl mit 4 oder 5 Kohlenstoffatomen, Carboxycycloalkyl, Imidazolyl, Pyridyl, Thiazolyl, Pyrazinyl, Furyl, Phenyl, Carboxyphenyl, Hydroxymethylphenyl, Carboxy-substituiertes niedrig-Cycloalkyl oder substituiertes neidrig-Alkyl ist, worin der Substituent Carboxy, niedrig-Alkoxycarbonyl, Oxo, Hydroxy, niedrig-Alkoxy, Halogen, niedrig-Acyloxy, niedrig-Dialkylamino, Sulfamoyl, Pyridyl, Furyl, Tetrahydrofuryl, 1-Methylpiperidyl, Morpholinyl, Pyrrolidinyl, 1-Methylpiperazinyl, Thienyl oder Aryl ist.

3. Verbindung nach Anspruch 1 oder 2, worin X die Gruppe der Formel —O—$R^1$ ist, worin $R^1$ niedrig-Alkyl oder Carboxy-niedrig-Alkyl ist.

4. Verbindung nach Anspruch 1 oder 2, worin $Y^1$ und $Y^2$ Cl sind.

5. Verbindung nach Anspruch 1 oder 2, worin A die Gruppe

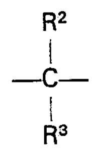 ist

worin $R^2$ und $R^3$, wie in Anspruch 1, definiert sind.

6. Verbindung nach Anspruch 1 oder 2, worin R Phenyl ist.

7. Verbindung nach Anspruch 1, nämlich [(5,6-Dichlor-3-oxo-9$\alpha$-propyl-1,2,9,9$\alpha$-tetra-hydro-3H-fluoren-7-yl)-oxy]-essigsäure.

8. Zusammensetzung in Dosiseinheitsform zur Behandlung von Ödemen der grauen Substanz, enthaltend eine Verbindung der Formel I

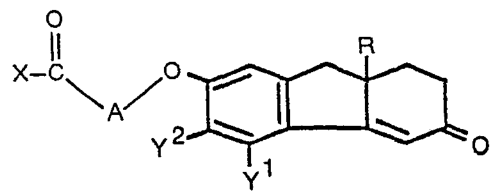

I

worin

R, X, $Y^1$, $Y^2$ und A die gleiche Bedeutung wie in der Formel I von Anspruch 1 aufweisen oder ein pharmazeutisch brauchbares Salz davon.

9. Verfahren zur Herstellung einer Verbindung der Formel Ib

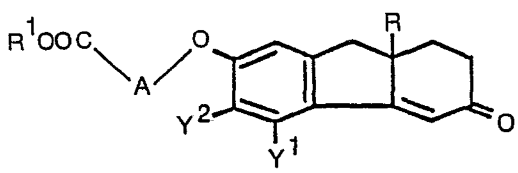

Ib

worin

R, $R^1$, $Y^1$, $Y^2$ und A die gleiche Bedeutung wie in der Formel I von Anspruch 1 aufweisen, durch Reaktion einer Verbindung der Formel II

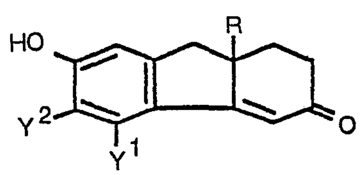

II

worin

R, $Y^1$ und $Y^2$ wie in der Formel Ib definiert sind mit einem Ester der Formel III

$$X—A—COOR^1$$

worin

X ein Halogen ist und

A und $R^1$ wie in der Formel Ib sind, vorzugsweise in Anwesenheit einer Base.

10. Verfahren zur Herstellung einer Verbindung der Formel Ib

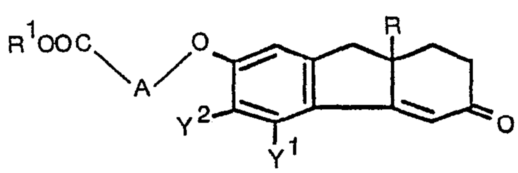

Ib

worin

R, $R^1$, $Y^1$, $Y^2$ und A die gleiche Bedeutung wie in der Formel I von Anspruch 1 aufweisen, durch Cyclisieren einer Verbindung der Formel IV

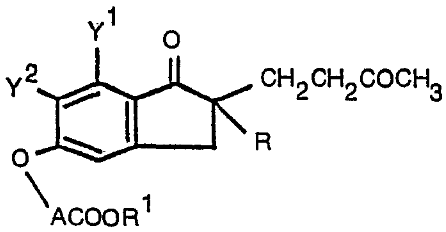

IV

durch Erwärmen in einem Lösungsmittel mit einem Katalysator wie Pyrrolidinacetat.

11. Verfahren zur Herstellung einer Verbindung der Formel Ib

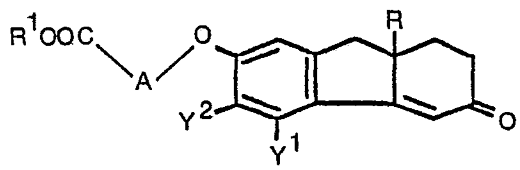

Ib

worin

R, $R^1$, $Y^1$, $Y^2$ und A die gleiche Bedeutung wie in der Formel I von Anspruch 1 aufweisen, durch Umesterung einer Verbindung der Formel Ic

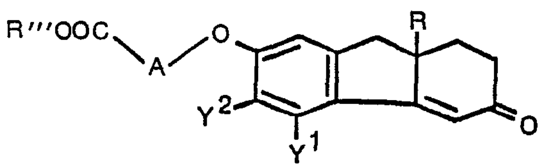

Ic

worin

R''' die gleiche Bedeutung wie $R^1$ in der Formel I hat, jedoch nicht identisch mit $R^1$ sein kann, mit einem Alkohol der Formel $R^1$-OH, durch Erwärmen des Gemischs in einem Lösungsmittel in Anwesenheit eines Ionenaustauscherharzes und einer katalytischen Menge von $R^1$-OM, worin M ein Alkalimetallion darstellt.